# EUROPEAN PATENT SPECIFICATION

(11) **EP 2 678 038 B1**
(45) Date of publication and mention of the grant of the patent: **08.05.2019**
(21) Application number: 12707225.4
(22) Date of filing: 10.02.2012
(51) Int. Cl.: A61K 47/60, A61K 39/39, A61K 39/00, A61K 47/54, A61K 47/64, A61K 47/55

(54) **VACCINE COMPOSITION COMPRISING COMPLEXED IMMUNOSTIMULATORY NUCLEIC ACIDS AND ANTIGENS PACKAGED WITH DISULFIDE-LINKED POLYETHYLENEGLYCOL/PEPTIDE CONJUGATES**
IMPFSTOFFZUSAMMENSETZUNG MIT KOMPLEXIERTEN IMMUNSTIMULIERENDEN NUKLEINSÄUREN UND ANTIGENE MIT DISULFIDVERNETZTEN POLYETHYLENGLYCOL-/PEPTID-KONJUGATEN
COMPOSITION DE VACCIN COMPRENANT DES ACIDES NUCLÉIQUES IMMUNOSTIMULATEURS COMPLEXÉS ET ANTIGÈNES EMBALLÉS AVEC DES CONJUGUÉS DE POLYÉTHYLÈNEGLYCOL/PEPTIDE À LIAISON DISULFURE

(30) Priority: 21.02.2011 WO PCT/EP2011/000827
(43) Date of publication of application: 01.01.2014
(73) Proprietor: CureVac AG, 72076 Tübingen (DE)
(72) Inventor: BAUMHOF, Patrick, 72144 Dusslingen (DE); KALLEN, Karl-Josef, 50226 Königsdorf (DE); FOTIN-MLECZEK, Mariola, 71065 Sindelfingen (DE)
(74) Representative: Graf von Stosch, Andreas
(86) International application number: PCT/EP2012/000614
(87) International publication number: WO 2012/113513

(56) References cited:
- WO-A1-2009/030254
- WO-A1-2009/144230
- WO-A1-2011/026641
- BOLHASSANI AZAM ET AL: "Improvement of different vaccine delivery systems for cancer therapy", MOLECULAR CANCER, BIOMED CENTRAL, LONDON, GB, vol. 10, no. 1, 7 January 2011 (2011-01-07), page 3, XP021088453, ISSN: 1476-4598, DOI: 10.1186/1476-4598-10-3
- SAKAE M ET AL: "Highly efficient in vivo gene transfection by plasmid/PEI complexes coated by anionic PEG derivatives bearing carboxyl groups and RGD peptide", BIOMEDICINE AND PHARMACOTHERAPY, ELSEVIER, FR, vol. 62, no. 7, 1 September 2008 (2008-09-01), pages 448-453, XP023520697, ISSN: 0753-3322, DOI: 10.1016/J.BIOPHA.2007.12.009 [retrieved on 2008-01-14]
- FUJITA T ET AL: "Calcium enhanced delivery of tetraarginine-PEG-lipid-coated DNA/protamine complexes", INTERNATIONAL JOURNAL OF PHARMACEUTICS, ELSEVIER BV, NL, vol. 368, no. 1-2, 23 February 2009 (2009-02-23), pages 186-192, XP025929000, ISSN: 0378-5173, DOI: 10.1016/J.IJPHARM.2008.09.060 [retrieved on 2008-10-19]
- GARINOT ET AL: "PEGylated PLGA-based nanoparticles targeting M cells for oral vaccination", JOURNAL OF CONTROLLED RELEASE, ELSEVIER, AMSTERDAM, NL, vol. 120, no. 3, 17 July 2007 (2007-07-17) , pages 195-204, XP022156267, ISSN: 0168-3659, DOI: 10.1016/J.JCONREL.2007.04.021
- FOTIN-MLECZEK MARIOLA ET AL: "Messenger RNA-based vaccines with dual activity induce balanced TLR-7 dependent adaptive immune responses and provide antitumor activity.", JOURNAL OF IMMUNOTHERAPY (HAGERSTOWN, MD. : 1997) JAN 2011 LNKD- PUBMED:21150709, vol. 34, no. 1, January 2011 (2011-01), pages 1-15, XP008144564, ISSN: 1537-4513
- Katrin Knop ET AL: "Poly(ethylene glycol) in Drug Delivery: Pros and Cons as Well as Potential Alternatives", Angewandte Chemie International Edition, vol. 49, no. 36, 20 July 2010 (2010-07-20) , pages 6288-6308, XP55251511, DE ISSN: 1433-7851, DOI: 10.1002/anie.200902672

## Description

The present invention is directed to an inventive vaccine composition comprising a) an adjuvant component comprising or consisting of at least one immunostimulatory nucleic acid sequence, complexed with a cationic or polycationic peptide or a protein as a complexing agent; b) a nucleic acid sequence encoding an antigen, wherein the nucleic acid is an RNA; and c) a polymeric carrier molecule as defined herein for combined packaging the adjuvant component and the RNA encoding the antigen. The present invention is also directed to the first medical use of such an inventive vaccine composition and to the second medical use of such an inventive vaccine composition or components thereof for the treatment of diseases, such as infectious or cancer or tumour diseases as defined herein. The present invention furthermore discloses kits comprising such a vaccine composition.

Induction and/or enhancement of immune responses of the innate and/or the adaptive immune system plays an important role in the treatment and prevention of numerous diseases. For such a purpose, the immune system is typically modulated by, e.g., via administration of an immunostimulatory agent, an adjuvant and/or a vaccine. However, the immune system of vertebrates such as humans is very complex and finely regulated. It consists of many types of proteins, cells, organs, and tissues, which interact in an elaborate and dynamic network. The immune system typically protects these organisms from infections with layered defences of increasing specificity. One layer of defence comprises physical or chemical barriers and allows an *a priori* elimination of at least some pathogens and antigens. A further layer of defence includes the innate and the adaptive immune system.

The innate immune system as part of the immune system is the dominant system of host defense in most organisms and comprises barriers such as humoral and chemical barriers including, e.g., inflammation, the complement system and cellular barriers. The innate immune system is typically based on a small number of receptors, called pattern recognition receptors. They recognize conserved molecular patterns that distinguish foreign organisms, like viruses, bacteria, fungi and parasites from cells of their hosts. Such pathogen-associated molecular patterns include viral nucleic acid sequences, components of bacterial and fungal walls, flagellar proteins, and more.

As part of the more complex immune response of vertebrates, the immune system adapts over time to recognize particular pathogens or antigens more efficiently (adaptive immune system). This adaptation process creates immunological memories and allows even more effective protection during future encounters with these pathogens. This process of adaptive or acquired immunity forms the basis for vaccination strategies. In contrast to the innate immune system as described above, the adaptive immune system is antigen-specific and requires the recognition of specific "self" or "non-self" antigens during a process called antigen presentation. Furthermore, unlike cells of the innate immune system, which recognize and respond to pathogens in a generic way, the adaptive immune system confers long-lasting or protective immunity to the host and thus allows a more tailored response to specific pathogens, pathogen-infected cells or antigens. The ability to mount these tailored responses is maintained in the body by so called "memory cells". Should an antigen or a pathogen enter/infect the body more than once, these specific memory cells are used to quickly eliminate it. The adaptive immune system thus allows for a stronger immune response as well as an immunological memory, wherein different immune responses are possible in favour of specific diseases. E.g., in case of infections, each pathogen is "remembered" by a signature antigen, whereas in case of cancer diseases tumour antigens or self-antigens may be recognized and neutralized by the adaptive immune system.

Both basic mechanisms of the immune system, i.e. the innate immune system as well as the adaptive immune system, may thus form targets for curative treatments and prevention of numerous diseases. Appropriate methods, which are presently known in the art, either utilize adjuvants to elicit an innate immune response or utilize antigens, pathogens or immunogens in order to evoke an adaptive immune response, or, in some rare cases, both.

An adaptive immune response may be elicited by administering to the cells or the host organism a specific foreign antigen as described above. Such an antigen may be either in the form of the peptide or protein antigens or the antigen may be encoded by a nucleic acid sequence, e.g. a cDNA or a messenger RNA. In order to elicit an efficient adaptive immune response, an additional unspecific stimulation of the innate immune system is advantageous, e.g. when providing an unspecific stimulus parallel to the antigen specific signal. The parallel unspecific stimulus turns the immune system into an activated state, which improves an adaptive immune response. Compounds capable of providing such an unspecific immune response are typically termed "adjuvants". A number of compounds and composition s have been proposed as adjuvants in the prior art, for example Freund's adjuvant, metal oxides, e.g. alum (aluminium hydroxide), inorganic chelates or salts thereof, various paraffin-like oils, synthetic resins, alginates, mucoids, polysaccharide compounds, caseinates, as well as compounds isolated from blood and/or blood clots, such as, for example, fibrin derivatives, etc. These adjuvants typically may be used in combination with other compounds, such as e.g. proteins, peptides, DNA or RNA molecules or other therapeutically active compounds, dependent on the result to be achieved.

However, free messenger RNA (mRNA) molecules, cDNAs or nucleic acid sequences in general, which may encode a specific antigen or any other therapeutically active protein, suitable for a specific therapy, typically do not show a significant or even no immunostimulatory property. Nevertheless, such immunostimulatory properties may be conferred to the mRNA molecule, the cDNA or the nucleic acid sequence, when complexed with a peptide or protein, such as protamin or a nucleic acid sequence binding protein. In this context, the mRNA molecule or the nucleic acid sequence may be formulated such, that a complex is formed between the mRNA molecule or the nucleic acid sequence and the peptide or protein, wherein different complexes may be formed between the mRNA molecule or the nucleic acid sequence and the peptide or protein. Particularly strong (adjuvant) complexes can occur, when the nucleic acid sequence, which is usually negatively charged at neutral pH, is bound by a cationic or polycationic peptide or protein.

When using mRNA or coding nucleic acid sequences in vaccination methods, translation of the mRNA or coding nucleic acid sequence *in vivo* remains the most important and essential factor for inducing an adaptive immune response or for expressing the encoded protein in general, e.g. in case of a therapeutically active protein or peptide. Accordingly, the complexed mRNA or coding nucleic acid sequence will have to be released from the complex with the (cationic) peptide or protein subsequent to transfection of the complex into the cells to allow efficient translation of the mRNA. Unfortunately, this does not occur in most cases. More typically, complexing the mRNA molecule or the nucleic acid sequence with a cationic or polycationic compound may even prevent the nucleic acid sequence from translation or at least significantly reduce the translation rate *in vivo* due to the strong binding of the polycationic compound to the mRNA molecule, cDNA or nucleic acid sequence in general. Accordingly, it is difficult to obtain a good immunostimulatory property of the composition with regard to the innate immune system taking these compounds and to ensure in parallel an efficient translation of the mRNA molecule, cDNA or nucleic acid sequence in general when using such a formulation.

One possibility to circumvent the above problem may be the administration of an adjuvant and mRNA in separated formulations. This, however, renders administration much more complicated. It is also preferred that the adjuvant and the antigen-encoding mRNA enter the same cell to achieve an optimal immune response. Furthermore, an adjuvant beneficially supports the induction of an adaptive immune response, if it induces an innate immune response in the same cell, in which the antigen is expressed by the encoding mRNA.

Another possibility to circumvent the above problem may be the exclusive administration of naked mRNA, cDNA or nucleic acid sequence. Such an approach, though advantageous for the purpose of efficient translation of the mRNA, cDNA or nucleic acid sequence *in vivo,* dispenses the advantageous activation of the innate immune system elicited by an adjuvant as described above. Furthermore, the naked mRNA, cDNA or nucleic acid sequence is typically degraded *in vivo* within an extremely short time limit, which does not allow providing efficient adaptive immune responses, when the antigen is encoded by the naked mRNA, cDNA or nucleic acid sequence.

A further approach may be the administration of an antigen in its protein or peptide form and additionally administering an adjuvant. However, this approach most likely leads to an extremely short exposition of the antigen to the immune system and thus to a poor (adaptive) immune response. It may even lead to a degradation of the antigen prior to recognition by the immune system and thus to a complete silencing of the desired immune response. Furthermore, similar problems may occur with regard to such an administration as already outlined before, since it cannot be ensured, that the adjuvant and the antigen enter the same cell to achieve an optimal immune response.

Thus, none of these approaches is in fact convincing and leads to an efficient innate and/or adaptive immune response. Moreover, if the antigen is administered in form of an mRNA, cDNA or nucleic acid sequence, the vaccination approach typically fails to provide a good translation of the administered mRNA, cDNA or nucleic acid sequence.

One obstacle in this context appears the necessity to both prevent degradation of an mRNA, cDNA or nucleic acid sequence to be administered *in vivo* while thereby allowing for a good transfection rate and subsequently an efficient translation. Additionally, it is necessary to enhance the immune response most efficiently and preferably to ensure that the adjuvant and the antigen enter the same cell to achieve an optimal immune response. In this context, many approaches have been carried out to enhance transfection rate and subsequently translation of a nucleic acid sequence to be administered. Such approaches, however, still do not lead to a good result *in vivo.*

WO 2009/144230 A1 relates to protamine/RNA nanoparticles in combination with an adjuvant for immune stimulation. The disclosed complex is, however, provided in a simple aqueous solution of salt ions that is not capable of promoting efficient packaging and/or import of the complexed RNA into the target cells.
As known to a skilled person, the full therapeutic potential of peptide-, protein-, and nucleic acid sequence-based drugs is frequently compromised by their limited ability to cross the plasma membrane of mammalian cells, resulting in poor cellular access and inadequate therapeutic efficacy. Today this hurdle represents a major challenge for the biomedical development and commercial success of many biopharmaceuticals (see e.g. Foerg and Merkle, Journal of Pharmaceutical Sciences, published online at www.interscience.wiley.com, 2008, 97(1): 144-62). This hurdle also represents a major challenge, when developing new vaccines based on the transfection of an mRNA, cDNA or nucleic acid sequence encoding an antigen or a therapeutically active protein.

To achieve successful transfer of e.g. nucleic acid sequences into an individual's cells, a number of different hurdles have to be passed, which likewise amount for the additional administration of further components. As an example, the transport of nucleic acid sequences typically occurs *via* association of the nucleic acid sequence with the cell membrane and subsequent uptake by the endosomes. In the endosomes, the introduced nucleic acid sequences are separated from the cytosol. As expression occurs in the cytosol, these nucleic acid sequences have to depart the cytosol. If the nucleic acid sequences do not manage departing the cytosol, either the endosome fuses with the lysosome leading to a degradation of its content, or the endosome fuses with the cell membrane leading to a return of its content into the extracellular medium. For efficient transfer of nucleic acid sequences, the endosomal escape thus appears to be one of the most important steps additional to the efficiency of transfection itself. Until now, there are different approaches addressing these issues. However, no approach was at least successful in all aspects. Furthermore, when administering a vaccine, comprising an antigen and additionally further compounds such as an adjuvant, it is necessary to ensure that the antigen and the further compounds reach the same cell to provide an efficient immune response.

One approach to achieve such objects may be the use of cationic polymers for formulating and transfecting such compounds. Cationic polymers turned out to be efficient in transfection of nucleic acid sequences, as they can tightly complex and condense a negatively charged nucleic acid sequence. Thus, a number of cationic polymers have been explored as carriers for *in vitro* and *in vivo* gene delivery. These include e.g. polyethylenimine (PEI), polyamidoamine and polypropylamine dendrimers, polyallylamine, cationic dextran, chitosan, cationic proteins and cationic peptides. However, although most cationic polymers share the function of condensing DNA into small particles and facilitating cellular uptake via endocytosis through charge-charge interaction with anionic sites on cell surfaces, their transfection activity and toxicity differ dramatically. Interestingly, cationic polymers exhibit better transfection efficiency with rising molecular weight due to stronger complexation of the negatively charged nucleic acid sequence cargo. However, a rising molecular weight also leads to a rising toxicity of the cationic polymer. PEI is perhaps the most active and most studied polymer for gene delivery, but its main drawback as a transfection reagent relates to its non-biodegradable nature and toxicity. Furthermore, even though polyplexes formed by high molecular weight polymers exhibit improved stability under physiological conditions, data have indicated that such polymers can hinder nucleic acid sequence unpacking. For example, poly (L-lysine) (PLL) of 19 and 36 residues was shown to dissociate from DNA more rapidly than PLL of 180 residues resulting in significantly enhanced short-term gene expression. A minimum length of six to eight cationic amino acids is required to compact DNA into structures active in receptor-mediated gene delivery. However, polyplexes formed with short polycations are unstable under physiological conditions and typically aggregate rapidly in physiological salt solutions. To overcome this negative impact, Read *et al*. (see Read, M. L., K. H. Bremner, et al. (2003). "Vectors based on reducible polycations facilitate intracellular release of nucleic acid sequences." J Gene Med 5(3): 232-45; and Read, M. L., S. Singh, et al. (2005). "A versatile reducible polycation-based system for efficient delivery of a broad range of nucleic acid sequences." Nucleic acid sequences Res 33(9): e86) developed a new type of synthetic vector based on a linear reducible polycation (RPC) prepared by oxidative polycondensation of the peptide Cys-Lys₁₀-Cys that can be cleaved by the intracellular environment to facilitate release of nucleic acid sequences. They could show that polyplexes formed by RPC are destabilised by reducing conditions enabling efficient release of DNA and mRNA. Cleavage of the RPC also reduced toxicity of the polycation to levels comparable with low molecular weight peptides. The disadvantage of this approach of Read *et al*. (2003, supra) was that the endosomolytic agent chloroquine or the cationic lipid DOTAP was additionally necessary to enhance transfection efficiency to adequate levels. As a consequence Read *et al*. (2005, supra) included histidine residues in the RPCs which have a known endosomal buffering capacity. They could show that histidine-rich RPCs can be cleaved by the intracellular reducing environment enabling efficient cytoplasmic delivery of a broad range of nucleic acid sequences, including plasmid DNA, mRNA and siRNA molecules without the requirement for the endosomolytic agent chloroquine.

Unfortunately; Read *et al*. (2005, supra) did not assess whether histidine-rich RPCs can be directly used for *in vivo* applications. In their study, transfections were performed in the absence of serum to avoid masking the ability of histidine residues to enhance gene transfer that may have arisen from binding of serum proteins to polyplexes restricting cellular uptake. Preliminary experiments indicate that the transfection properties of histidine-rich RPC polyplexes can be affected by the presence of serum proteins with a 50% decrease in GFP-positive cells observed in 10% FCS (fetal calf serum). For *in vivo* application they propose modifications with the hydrophilic polymer poly-[N-(2hydroxypropyl)methacrylamide]. Unfortunately, Read *et a*/*.* (2005, supra) did not prevent aggregation of polyplexes and binding of polycationic proteins to serum proteins. Furthermore, due to the large excess of polymer, which is characterized by the high N/P ratio, strong cationic complexes are formed when complexing the nucleic acid sequence, which are only of limited use *in vivo* due to their strong tendency of salt induced agglomeration and interactions with serum contents (opsonization). Additionally, these complexes may excite an acute immune response, when used for purposes of gene therapy. Read *et al*. (2003, supra) did also not provide *in vivo* data for the RPC based complexes shown in the publication. It has also turned out that these strong cationic RPC based complexes are completely inactive subsequent to local administration into the dermis. Furthermore Read *et al*. (2005, supra) used stringent oxidation conditions (30% DMSO) to induce the generation of high molecular polymers with as long as possible chain lengths ("step-growth polymerization") to ensure complete complexation of the nucleic acid sequence cargo.

In an approach similar to Read *et al*. (2005, supra) McKenzie *et al*. (McKenzie, D. L., K. Y. Kwok, et al. (2000), J Biol Chem 275(14): 9970-7., McKenzie, D. L., E. Smiley, et al. (2000), Bioconjug Chem 11(6): 901-9, and US 6,770,740 B1) developed self-crosslinking peptides as gene delivery agents by inserting multiple cysteines into short synthetic peptides for the purpose of decreasing toxicity as observed with high-molecular polycations. For complexation of DNA they mixed the self-crosslinking peptides with DNA to induce interpeptide disulfide bonds concurrently to complexation of the DNA cargo. For *in vivo* gene delivery approaches they propose the derivatization of the self-crosslinking peptides with a stealthing (e.g. polyethylene glycol) or targeting agent operatively attached to the peptide at a site distal from each terminus. In a further approach the same authors developed for the purpose of masking DNA peptide condensates and thereby reducing interaction with blood components, the derivatization of the non crosslinking cationic peptide CWK₁₈ with polyethylene glycol by reducible or non-reducible linkages (Kwok, K. Y., D. L. McKenzie, et al. (1999). "Formulation of highly soluble poly(ethylene glycol)-peptide DNA condensates." J Pharm Sci 88(10): 996-1003).
Summarizing the above, the present prior art utilizing cationic polymers suffers from various disadvantages. One particular disadvantage of the self-crosslinking peptides as described by Read *et al*. (2003, supra) or McKenzie *et al.* (2000 I and II, supra and US 6,770,740 B1) concerns the high positive charge on the surface of the particles formed. Due to the high positive charge the particles exhibit a high instability towards agglomeration when subjecting these particles *in vivo* to raised salt concentrations. Such salt concentrations, however, typically occur *in vivo* in cells or extracellular media. Furthermore, high positively charged complexes show a strong tendency of opsonization. This leads to an enhanced uptake by macrophages and furthermore to a fast inactivation of the complex due to degradation. Additionally, in biological systems positively charged complexes can easily be bound or immobilized by negatively charged components of the extracellular matrix or the serum. Also, the nucleic acid sequences in the complex may be released too early, leading to reduced efficiency of the transfer and half life of the complexes *in vivo.* Furthermore, a reversible derivatization of carriers with a stealthing agent being advantageous for *in vivo* gene delivery, such as polyethylene glycol (PEG), was only possible for peptide monomers but not for self-crosslinking peptides or rather for a polymeric carrier with a defined polymer chain length. In particular, such a reversible derivatization was not possible at the terminal ends of the crosslinked cationic peptide carrier. Additionally, in the prior art only high-molecular polymers with long polymer chains or with an undefined polymer chain length consisting of self-crosslinking peptides were described, which unfortunately compact their cargo to such an extent that cargo release in the cell is limited. The extremely undefined polymer chain length is further problematic regarding approvement of a medicament based on RPC. One precondition for an approvement of a medicament is that every preparation of the medicament has always the same composition , the same structure and the same properties. This cannot be ensured for complexes based on RPC's from the prior art. Furthermore the RPC-based polymers or complexes provided in the prior art are difficult to characterize due to their undefined structure or polymer chain length. But characterization of the resulting complex or of the polymeric carrier is absolutely necessary for the approvement of a medicament.

Accordingly, there is still the need in the art for providing an efficient method for vaccination, which allows eliciting an adaptive immune response, wherein the administration is not severely impaired by early degradation of the antigen or by an inefficient translation of the mRNA due to inefficient release of the mRNA in the cell. Most importantly, there is an intensive need in the art to allow for provision of additional components, such as e.g. an adjuvant compound, and to provide same to the same cell together with the antigen before the antigen or the further component is metabolically cleaved.

This object is solved by the subject matter of the present invention, preferably by the subject matter of the attached claims. Particularly, the present invention solves the above object according to a first embodiment by a vaccine composition , comprising or consisting of:
a) an adjuvant component comprising or consisting of at least one immunostimulatory nucleic acid sequence, complexed with a cationic or polycationic peptide or a protein as a complexing agent;
b) a nucleic acid sequence encoding an antigen, wherein the nucleic acid is an RNA ; and
c) a polymeric carrier molecule as defined herein for combined packaging the adjuvant component and the RNA encoding the antigen.

The inventive vaccine composition is preferably capable to elicit an innate and an adaptive immune response due to both the adjuvant component and the antigen contained in the inventive vaccine composition, wherein the administration of the inventive vaccine composition is preferably not impaired by early degradation of the antigen or by inefficient translation of a nucleic acid sequence encoding such an antigen. The inventive vaccine composition ; due to the use of one carrier molecule for both the adjuvant component and the antigen, furthermore ensures that the adjuvant component and the antigen are transported to the same cell before they can be metabolically cleaved or cleared from the tissue or cells. Furthermore, the carrier molecule of the inventive vaccine composition as such can be cleared from the tissue subsequent to the release of its cargo before it can accumulate and reach toxic levels. The carrier also prevents agglomeration of the adjuvant component comprising or consisting of at least one immunostimulatory nucleic acid sequence, complexed with a complexing agent. The carrier furthermore efficiently prevents degradation of the antigen by RNAses, when provided as a nucleic acid sequence. Finally, the inventive vaccine composition allows for incorporation of endosomolytic peptides into the vaccine, preferably into the carrier of the inventive vaccine composition and/or into the complex of the adjuvant component with a complexing agent, *via* simplified administration of such an endosomolytic peptide to the specific component during preparation of said component.

The inventive vaccine composition comprises an adjuvant component comprising or consisting of at least one immunostimulatory nucleic acid sequence, complexed with a complexing agent as defined herein. In this context, the at least one immunostimulatory nucleic acid sequence may be selected from any nucleic acid sequence, known to a skilled person to be immunostimulatory, including, without being limited thereto, immunostimulatory CpG nucleic acid sequences, immunostimulatory RNA (is)RNA, ribosomal RNA (rRNA), transfer RNA (tRNA), messenger RNA (mRNA), viral RNA (vRNA), etc. Such an immunostimulatory nucleic acid may comprise a length of about 1000 to 5000, of 500 to 5000, of 5 to 5000, or of 5 to 1000, 5 to 500, 5 to 250, of 5 to 100, of 5 to 50 or of 5 to 30 nucleotides but also a length of about 10 to 1000, 10 to 500, 20 to 500, 30 to 500, 40 to 500 or 50 to 500 nucleotides.

According to one aspect, the at least one immunostimulatory nucleic acid sequence of the adjuvant component as defined herein may be selected from an immunostimulatory CpG nucleic acid sequence, in particular CpG-RNA or CpG-DNA, which preferably induces an innate immune response. A CpG-RNA or CpG-DNA used according to the invention can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid sequence used according to the invention is preferably in the form of CpG-RNA or CpG-DNA, more preferably in the form of single-stranded CpG-RNA (ss CpG-RNA) or CpG-DNA. Also preferably, such CpG nucleic acid sequences have a length as described above. Preferably the CpG motifs are unmethylated.

Likewise, according to a further aspect, the at least one immunostimulatory nucleic acid sequence may be selected from an immunostimulatory RNA (isRNA), which preferably elicits an innate immune response. Such an immunostimulatory RNA may be any (double-stranded or single-stranded) RNA, e.g. a coding RNA, as defined herein. Preferably, the immunostimulatory RNA may be a single-stranded, a double-stranded or a partially double-stranded RNA, more preferably a single-stranded RNA, and/or a circular or linear RNA, more preferably a linear RNA. More preferably, the immunostimulatory RNA may be a (linear) single-stranded RNA. Even more preferably, the immunostimulatory RNA may be a (long) (linear) single-stranded) non-coding RNA. In this context it is particular preferred that the isRNA carries a triphosphate at its 5'-end which is the case for *in vitro* transcribed RNA. An immunostimulatory RNA may also occur as a short RNA oligonucleotide as defined herein. An immunostimulatory RNA as used herein may furthermore be selected from any class of RNA molecules, found in nature or being prepared synthetically, and which can induce an innate immune response and may support an adaptive immune response induced by an antigen. In this context, an immune response may occur in various ways. A substantial factor for a suitable (adaptive) immune response is the stimulation of different T-cell sub-populations. T-lymphocytes are typically divided into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of the effector proteins (cytokines) produced by them. Thus, Th1 cells assist the cellular immune response by activation of macrophages and cytotoxic T-cells. Th2 cells, on the other hand, promote the humoral immune response by stimulation of B-cells for conversion into plasma cells and by formation of antibodies (e.g. against antigens). The Th1/Th2 ratio is therefore of great importance in the induction and maintenance of an adaptive immune response. In connection with the present invention, the Th1/Th2 ratio of the (adaptive) immune response is preferably shifted in the direction towards the cellular response (Th1 response) and a cellular immune response is thereby induced. According to one example, the innate immune system, which may support an adaptive immune response, may be activated by ligands of Toll-like receptors (TLRs). TLRs are a family of highly conserved pattern recognition receptor (PRR) polypeptides that recognize pathogen-associated molecular patterns (PAMPs) and play a critical role in innate immunity in mammals. Currently at least thirteen family members, designated TLR1 - TLR13 (Toll-like receptors: TLR1, TLR2, TLR3, TLR4, TLR5, TLR6, TLR7, TLR8, TLR9, TLR10, TLR11, TLR12 or TLR13), have been identified. Furthermore, a number of specific TLR ligands have been identified. It was e.g. found that unmethylated bacterial DNA and synthetic analogs thereof (CpG DNA) are ligands for TLR9 (Hemmi H et al. (2000) Nature 408:740-5; Bauer S et al. (2001) Proc NatlAcadSci USA 98, 9237-42). Furthermore, it has been reported that ligands for certain TLRs include certain nucleic acid sequences and that certain types of RNA are immunostimulatory in a sequence-independent or sequence-dependent manner, wherein these various immunostimulatory RNAs may e.g. stimulate TLR3, TLR7, or TLR8, or intracellular receptors such as RIG-I, MDA-5, etc. E.g. Lipford *et al*. determined certain G,U-containing oligoribonucleotides as immunostimulatory by acting via TLR7 and TLR8 (see WO 03/086280). The immunostimulatory G,U-containing oligoribonucleotides described by Lipford *et al*. were believed to be derivable from RNA sources including ribosomal RNA, transfer RNA, messenger RNA, and viral RNA.

The immunostimulatory RNA (isRNA) used as the at least one immunostimulatory nucleic acid sequence of the adjuvant component as defined herein may thus comprise any RNA sequence known to be immunostimulatory, including, without being limited thereto, RNA sequences representing and/or encoding ligands of TLRs, preferably selected from human family members TLR1 - TLR10 or murine family members TLR1 - TLR13, more preferably selected from (human) family members TLR1 - TLR10, even more preferably from TLR7 and TLR8, ligands for intracellular receptors for RNA (such as RIG-I or MDA-5, etc) (see e.g. Meylan, E., Tschopp, J. (2006), Mol. Cell 22, 561-569), or any other immunostimulatory RNA sequence. Furthermore, (classes of) immunostimulatory RNA molecules, used as the at least one immunostimulatory nucleic acid sequence of the adjuvant component as defined herein may include any other RNA capable of eliciting an immune response. Without being limited thereto, such an immunostimulatory RNA may include ribosomal RNA (rRNA), transfer RNA (tRNA), messenger RNA (mRNA), and viral RNA (vRNA). Such an immunostimulatory RNA may comprise a length of 1000 to 5000, of 500 to 5000, of 5 to 5000, or of 5 to 1000, 5 to 500, 5 to 250, of 5 to 100, of 5 to 50 or of 5 to 30 nucleotides or a length as defined above.

According to a particularly preferred aspect, such immunostimulatory nucleic acid sequences, particularly isRNA, used as the at least one immunostimulatory nucleic acid sequence of the adjuvant component as defined herein, may preferably consist of or comprise a nucleic acid sequence of formula (I) or (II):

GₗXₘGₙ (formula (I))

wherein:
- G: is guanosine, uracil or an analogue of guanosine or uracil;
- X: is guanosine, uracil, adenosine, thymidine, cytosine or an analogue of the above-mentioned nucleotides;
- l: is an integer from 1 to 40,
wherein
when l = 1 G is guanosine or an analogue thereof,
when l > 1 at least 50% of the nucleotides are guanosine or an analogue thereof;
- m: is an integer and is at least 3;
wherein
when m = 3 X is uracil or an analogue thereof,
when m > 3 at least 3 successive uracils or analogues of uracil occur;
- n: is an integer from 1 to 40,
wherein
when n = 1 G is guanosine or an analogue thereof,
when n > 1 at least 50% of the nucleotides are guanosine or an analogue thereof.

CₗXₘCₙ (formula (II))

wherein:
- C: is cytosine, uracil or an analogue of cytosine or uracil;
- X: is guanosine, uracil, adenosine, thymidine, cytosine or an analogue of the above-mentioned nucleotides;
- l: is an integer from 1 to 40,
wherein
when l = 1 C is cytosine or an analogue thereof,
when l > 1 at least 50% of the nucleotides are cytosine or an analogue thereof;
- m: is an integer and is at least 3;
wherein
when m = 3 X is uracil or an analogue thereof,
when m > 3 at least 3 successive uracils or analogues of uracil occur;
- n: is an integer from 1 to 40,
wherein
when n = 1 C is cytosine or an analogue thereof,
when n > 1 at least 50% of the nucleotides are cytosine or an analogue thereof.

The nucleic acid sequences of formula (I) or (II), which may be used as the at least one immunostimulatory nucleic acid sequence of the adjuvant component as defined herein, may be relatively short nucleic acid sequences with a typical length of approximately from 5 to 100 (but may also be longer than 100 nucleotides for specific aspects, e.g. up to 200 nucleotides), from 5 to 90 or from 5 to 80 nucleotides, preferably a length of approximately from 5 to 70, more preferably a length of approximately from 8 to 60 and, more preferably a length of approximately from 15 to 60 nucleotides, more preferably from 20 to 60, most preferably from 30 to 60 nucleotides. If the at least one immunostimulatory nucleic acid sequence of the adjuvant component as defined herein has a maximum length of e.g. 100 nucleotides, m will typically be <=98. The number of nucleotides G in the nucleic acid sequence of formula (I) is determined by l or n. l and n, independently of one another, are each an integer from 1 to 40, wherein when l or n = 1 G is guanosine or an analogue thereof, and when l or n > 1 at least 50% of the nucleotides are guanosine or an analogue thereof. For example, without implying any limitation, when l or n = 4 Gₗ or Gₙ can be, for example, a GUGU, GGUU, UGUG, UUGG, GUUG, GGGU, GGUG, GUGG, UGGG or GGGG, etc.; when l or n = 5 Gₗ or Gₙ can be, for example, a GGGUU, GGUGU, GUGGU, UGGGU, UGGUG, UGUGG, UUGGG, GUGUG, GGGGU, GGGUG, GGUGG, GUGGG, UGGGG, or GGGGG, etc.; etc. A nucleotide adjacent to Xₘ in the nucleic acid sequence of formula (I) according to the invention is preferably not a uracil. Similarly, the number of nucleotides C in the nucleic acid sequence of formula (II) according to the invention is determined by l or n. l and n, independently of one another, are each an integer from 1 to 40, wherein when l or n = 1 C is cytosine or an analogue thereof, and when l or n > 1 at least 50% of the nucleotides are cytosine or an analogue thereof. For example, without implying any limitation, when l or n = 4, Cₗ or Cₙ can be, for example, a CUCU, CCUU, UCUC, UUCC, CUUC, CCCU, CCUC, CUCC, UCCC or CCCC, etc.; when l or n = 5 Cₗ or Cₙ can be, for example, a CCCUU, CCUCU, CUCCU, UCCCU, UCCUC, UCUCC, UUCCC, CUCUC, CCCCU, CCCUC, CCUCC, CUCCC, UCCCC, or CCCCC, etc.; etc. A nucleotide adjacent to Xₘ in the nucleic acid sequence of formula (I) according to the invention is preferably not a uracil. Preferably, for formula (I), when l or n > 1, at least 60%, 70%, 80%, 90% or even 100% of the nucleotides are guanosine or an analogue thereof, as defined above. The remaining nucleotides to 100% (when guanosine constitutes less than 100% of the nucleotides) in the flanking sequences G₁ and/or Gₙ are uracil or an analogue thereof, as defined hereinbefore. Also preferably, l and n, independently of one another, are each an integer from 2 to 30, more preferably an integer from 2 to 20 and yet more preferably an integer from 2 to 15. The lower limit of l or n can be varied if necessary and is at least 1, preferably at least 2, more preferably at least 3, 4, 5, 6, 7, 8, 9 or 10. This definition applies correspondingly to formula (II).

According to a particularly preferred aspect, a nucleic acid sequence according to any of formulas (I) or (II) above, which may be used as the at least one immunostimulatory nucleic acid sequence of the adjuvant component as defined herein, may be selected from a sequence preferably consisting of or comprising any of the following sequences:
- GGUUUUUUUUUUUUUUUGGG (SEQ ID NO: 1);
- GGGGGUUUUUUUUUUGGGGG (SEQ ID NO: 2);
- GGGGGUUUUUUUUUUUUUUUUUUUUUUUUUUUUUUGGGGG (SEQ ID NO: 3);
- GUGUGUGUGUGUUUUUUUUUUUUUUUUGUGUGUGUGUGU (SEQ ID NO: 4);
- GGUUGGUUGGUUUUUUUUUUUUUUUUUGGUUGGUUGGUU (SEQ ID NO: 5);
- GGGGGGGGGUUUGGGGGGGG (SEQ ID NO: 6);
- GGGGGGGGUUUUGGGGGGGG (SEQ ID NO: 7);
- GGGGGGGUUUUUUGGGGGGG (SEQ ID NO: 8);
- GGGGGGGUUUUUUUGGGGGG (SEQ ID NO: 9);
- GGGGGGUUUUUUUUGGGGGG (SEQ ID NO: 10);
- GGGGGGUUUUUUUUUGGGGG (SEQ ID NO: 11);
- GGGGGGUUUUUUUUUUGGGG (SEQ ID NO: 12);
- GGGGGUUUUUUUUUUUGGGG (SEQ ID NO: 13);
- GGGGGUUUUUUUUUUUUGGG (SEQ ID NO: 14);
- GGGGUUUUUUUUUUUUUGGG (SEQ ID NO: 15);
- GGGGUUUUUUUUUUUUUUGG (SEQ ID NO: 16);
- GGUUUUUUUUUUUUUUUUGG (SEQ ID NO: 17);
- GUUUUUUUUUUUUUUUUUUG (SEQ ID NO: 18);
- GGGGGGGGGGUUUGGGGGGGGG (SEQ ID NO: 19);
- GGGGGGGGGUUUUGGGGGGGGG (SEQ ID NO: 20);
- GGGGGGGGUUUUUUGGGGGGGG (SEQ ID NO: 21);
- GGGGGGGGUUUUUUUGGGGGGG (SEQ ID NO: 22);
- GGGGGGGUUUUUUUUGGGGGGG (SEQ ID NO: 23);
- GGGGGGGUUUUUUUUUGGGGGG (SEQ ID NO: 24);
- GGGGGGGUUUUUUUUUUGGGGG (SEQ ID NO: 25);
- GGGGGGUUUUUUUUUUUGGGGG (SEQ ID NO: 26);
- GGGGGGUUUUUUUUUUUUGGGG (SEQ ID NO: 27);
- GGGGGUUUUUUUUUUUUUGGGG (SEQ ID NO: 28);
- GGGGGUUUUUUUUUUUUUUGGG (SEQ ID NO: 29);
- GGGUUUUUUUUUUUUUUUUGGG (SEQ ID NO: 30);
- GGUUUUUUUUUUUUUUUUUUGG (SEQ ID NO: 31);
- GGGGGGGGGGGUUUGGGGGGGGGG (SEQ ID NO: 32);
- GGGGGGGGGGUUUUGGGGGGGGGG (SEQ ID NO: 33);
- GGGGGGGGGUUUUUUGGGGGGGGG (SEQ ID NO: 34);
- GGGGGGGGGUUUUUUUGGGGGGGG (SEQ ID NO: 35);
- GGGGGGGGUUUUUUUUGGGGGGGG (SEQ ID NO: 36);
- GGGGGGGGUUUUUUUUUGGGGGGG (SEQ ID NO: 37);
- GGGGGGGGUUUUUUUUUUGGGGGG (SEQ ID NO: 38);
- GGGGGGGUUUUUUUUUUUGGGGGG (SEQ ID NO: 39);
- GGGGGGGUUUUUUUUUUUUGGGGG (SEQ ID NO: 40);
- GGGGGGUUUUUUUUUUUUUGGGGG (SEQ ID NO: 41);
- GGGGGGUUUUUUUUUUUUUUGGGG (SEQ ID NO: 42);
- GGGGUUUUUUUUUUUUUUUUGGGG (SEQ ID NO: 43);
- GGGUUUUUUUUUUUUUUUUUUGGG (SEQ ID NO: 44);
- GUUUUUUUUUUUUUUUUUUUUUUUUUUUUUUG (SEQ ID NO: 45);
- GGUUUUUUUUUUUUUUUUUUUUUUUUUUUUUUGG (SEQ ID NO: 46);
- GGGGGUUUUUUUUUUUUUUUUUUUUUUUUUUUUUUGGG (SEQ ID NO: 47);
- GGGGUUUUUUUUUUUUUUUUUUUUUUUUUUUUUUGGG (SEQ ID NO: 48);
- GGGGGUUUUUUUUUUUUUUUUUUUUUUUUUUUUUUGGGG (SEQ ID NO: 49);
- GGGGGGUUUUUUUUUUUUUUUUUUUUUUUUUUUUUUGGGGG (SEQ ID NO: 50);
- GGGGGGGUUUUUUUUUUUUUUUUUUUUUUUUUUUUUUGGGGGG (SEQ ID NO: 51);
- GGGGGGGGUUUUUUUUUUUUUUUUUUUUUUUUUUUUUUGGGGGGG (SEQ ID NO: 52);
- GGGGGGGGGUUUUUUUUUUUUUUUUUUUUUUUUUUUUUUGGGGGGGG (SEQ ID NO: 53);
- GGUUUGG (SEQ ID NO: 54);
- GGUUUUGG (SEQ ID NO: 55);
- GGUUUUUGG (SEQ ID NO: 56);
- GGUUUUUUGG (SEQ ID NO: 57);
- GGUUUUUUUGG (SEQ ID NO: 58);
- GGUUUUUUUUGG (SEQ ID NO: 59);
- GGUUUUUUUUUGG (SEQ ID NO: 60);
- GGUUUUUUUUUUGG (SEQ ID NO: 61);
- GGUUUUUUUUUUUGG (SEQ ID NO: 62);
- GGUUUUUUUUUUUUGG (SEQ ID NO: 63);
- GGUUUUUUUUUUUUUGG (SEQ ID NO: 64);
- GGUUUUUUUUUUUUUUGG (SEQ ID NO: 65);
- GGUUUUUUUUUUUUUUUGG (SEQ ID NO: 66);
- GGGUUUGGG (SEQ ID NO: 67);
- GGGUUUUGGG (SEQ ID NO: 68);
- GGGUUUUUGGG (SEQ ID NO: 69);
- GGGUUUUUUGGG (SEQ ID NO: 70);
- GGGUUUUUUUGGG (SEQ ID NO: 71);
- GGGUUUUUUUUGGG (SEQ ID NO: 72);
- GGGUUUUUUUUUGGG (SEQ ID NO: 73);
- GGGUUUUUUUUUUGGG (SEQ ID NO: 74);
- GGGUUUUUUUUUUUGGG (SEQ ID NO: 75);
- GGGUUUUUUUUUUUUGGG (SEQ ID NO: 76);
- GGGUUUUUUUUUUUUUGGG (SEQ ID NO: 77);
- 
GGGUUUUUUUUUUUUUGGGUUUUUUUUUUUUUGGGUUUUUUUUUUUUUU
UUUUUUGGG
(SEQ ID NO: 78);
- GGGUUUUUUUUUUUUUUUGGGGGGUUUUUUUUUUUUUUUGGG (SEQ ID NO: 79);
- 
GGGUUGGGUUGGGUUGGGUUGGGUUGGGUUGGGUUGGGUUU
GGG (SEQ ID NO: 80);
- GGUUUUUUUUUUUUUUUGGG (short GU-rich, SEQ ID NO: 81)
   or
- 
CCCUUUUUUUUUUUUUCCCUUUUUUUUUUUUUUUUCCCUUUUUUUUUUUUU
UUUUCCC
(SEQ ID NO: 82)
- CCCUUUCCCUUUCCCUUUCCCUUUCCCUUUCCCUUUCCCUUUCCCUUUCCC (SEQ ID NO: 83)
- CCCUUUUUUUUUUUUUUUCCCCCCUUUUUUUUUUUUUUUCCC (SEQ ID NO: 84)
or from a sequence having at least 60%, 70%, 80%, 90%, or even 95% sequence identity with any of these sequences

According to a further particularly preferred aspect, an immunostimulatory nucleic acid sequence, particularly an isRNA, used as the at least one immunostimulatory nucleic acid sequence of the adjuvant component as defined herein, preferably consists of or comprises a nucleic acid sequence of formula (III) or (IV):

(NᵤGₗXₘGₙNᵥ)ₐ, (formula (III))

wherein:
- G: is guanosine (guanine), uridine (uracil) or an analogue of guanosine (guanine) or uridine (uracil), preferably guanosine (guanine) or an analogue thereof;
- X: is guanosine (guanine), uridine (uracil), adenosine (adenine), thymidine (thymine), cytidine (cytosine), or an analogue of these nucleotides (nucleosides), preferably uridine (uracil) or an analogue thereof;
- N: is a nucleic acid sequence having a length of about 4 to 50, preferably of about 4 to 40, more preferably of about 4 to 30 or 4 to 20 nucleotides (nucleosides), each N independently being selected from guanosine (guanine), uridine (uracil), adenosine (adenine), thymidine (thymine), cytidine (cytosine) or an analogue of these nucleotides (nucleosides);
- a: is an integer from 1 to 20, preferably from 1 to 15, most preferably from 1 to 10;
- l: is an integer from 1 to 40,
wherein when l = 1, G is guanosine (guanine) or an analogue thereof,
when l > 1, at least 50% of these nucleotides (nucleosides) are guanosine (guanine) or an analogue
thereof;
- m: is an integer and is at least 3;
wherein when m = 3, X is uridine (uracil) or an analogue thereof, and
when m > 3, at least 3 successive uridines (uracils) or analogues of uridine (uracil) occur;
- n: is an integer from 1 to 40,
wherein when n = 1, G is guanosine (guanine) or an analogue thereof,
when n > 1, at least 50% of these nucleotides (nucleosides) are guanosine (guanine) or an analogue
thereof;
- u, v: may be independently from each other an integer from 0 to 50,
preferably wherein when u = 0, v ≥ 1, or
when v = 0, u ≥ 1;
wherein the immunostimulatory nucleic acid sequence of formula (III) has a length of at least 50 nucleotides, preferably of at least 100 nucleotides, more preferably of at least 150 nucleotides, even more preferably of at least 200 nucleotides and most preferably of at least 250 nucleotides.

(NᵤCₗXₘCₙNᵥ)ₐ (formula (IV))

wherein:
- C: is cytidine (cytosine), uridine (uracil) or an analogue of cytidine (cytosine) or uridine (uracil), preferably cytidine (cytosine) or an analogue thereof;
- X: is guanosine (guanine), uridine (uracil), adenosine (adenine), thymidine (thymine), cytidine (cytosine) or an analogue of the above-mentioned nucleotides (nucleosides), preferably uridine (uracil) or an analogue thereof;
- N: is each a nucleic acid sequence having independent from each other a length of about 4 to 50, preferably of about 4 to 40, more preferably of about 4 to 30 or 4 to 20 nucleotides (nucleosides), each N independently being selected from guanosine (guanine), uridine (uracil), adenosine (adenine), thymidine (thymine), cytidine (cytosine) or an analogue of these nucleotides (nucleosides);
- a: is an integer from 1 to 20, preferably from 1 to 15, most preferably from 1 to 10;
- l: is an integer from 1 to 40,
wherein when l = 1, C is cytidine (cytosine) or an analogue thereof,
when l > 1, at least 50% of these nucleotides (nucleosides) are cytidine (cytosine) or an analogue
thereof;
- m: is an integer and is at least 3;
wherein when m = 3, X is uridine (uracil) or an analogue thereof,
when m > 3, at least 3 successive uridines (uracils) or analogues of uridine (uracil) occur;
- n: is an integer from 1 to 40,
wherein when n = 1, C is cytidine (cytosine) or an analogue thereof,
when n > 1, at least 50% of these nucleotides (nucleosides) are cytidine (cytosine) or an analogue
thereof.
- u, v: may be independently from each other an integer from 0 to 50,
preferably wherein when u = 0, v ≥ 1, or
when v = 0, u ≥ 1;
wherein the immunostimulatory nucleic acid sequence of formula (IV) according to the invention has a length of at least 50 nucleotides, preferably of at least 100 nucleotides, more preferably of at least 150 nucleotides, even more preferably of at least 200 nucleotides and most preferably of at least 250 nucleotides.

For formula (IV), any of the definitions given above for elements N (i.e. Nᵤ and Nᵥ) and X (Xₘ), particularly the core structure as defined above, as well as for integers a, l, m, n, u and v, similarly apply to elements of formula (IV) correspondingly, wherein in formula (IV) the core structure is defined by CₗXₘCₙ. The definition of bordering elements Nᵤ and Nᵥ is identical to the definitions given above for Nᵤ and Nᵥ.

According to a very particularly preferred aspect, the nucleic acid sequence according to formula (III), used as the at least one immunostimulatory nucleic acid sequence of the adjuvant component as defined herein, may be selected from e.g. any of the following sequences:

UAGCGAAGCUCUUGGACCUAGGUUUUUUUUUUUUUUUGGGUGCGUUCCUAGAA
GUACACG (SEQ ID NO: 85)

UAGCGAAGCUCUUGGACCUAGGUUUUUUUUUUUUUUGGGUGCGUUCCUAGAA
GUACACGAUCGCUUCGAGAACCUGGAUCCAAAAAAAAAAAAAACCCACGCAAGGA
UCUUCAUGUGC (SEQ ID NO: 86)

GGGAGAAAGCUCAAGCUUGGAGCAAUGCCCGCACAUUGAGGAAACCGAGUUGCAU
AUCUCAGAGUAUUGGCCCCGUGUAGGUUAUUCUUGACAGACAGUGGAGCUUAU
UCACUCCCAGGAUCCGAGUCGCAUACUACGGUACUGGUGACAGACCUAGGUCGUC
AGUUGACCAGUCCGCCACUAGACGUGAGUCCGUCAAAGCAGUUAGAUGUUACACU
CUAUUAGAUC (SEQ ID NO: 87)

GGGAGAAAGCUCAAGCUUGGAGCAAUGCCCGCACAUUGAGGAAACCGAGUUGCAU
AUCUCAGAGUAUUGGCCCCGUGUAGGUUAUUCUUGACAGACAGUGGAGCUUAU
UCACUCCCAGGAUCCGAGUCGCAUACUACGGUACUGGUGACAGACCUAGGUCGUC
AGUUGACCAGUCCGCCACUAGACGUGAGUCCGUCAAAGCAGUUAGAUGUUACACU
CUAUUAGAUCUCGGAUUACAGCUGGAAGGAGCAGGAGUAGUGUUCUUGCUCUAA
GUACCGAGUGUGCCCAAUACCCGAUCAGCUUAUUAACGAACGGCUCCUCCUCUUA
GACUGCAGCGUAAGUGCGGAAUCUGGGGAUCAAAUUACUGACUGCCUGGAUUAC
CCUCGGACAUAUAACCUUGUAGCACGCUGUUGCUGUAUAGGUGACCAACGCCCAC
UCGAGUAGACCAGCUCUCUUAGUCCGGACAAUGAUAGGAGGCGCGGUCAAUCUAC
UUCUGGCUAGUUAAGAAUAGGCUGCACCGACCUCUAUAAGUAGCGUGUCCUCUA
G (SEQ ID NO: 88)

GGGAGAAAGCUCAAGCUUGGAGCAAUGCCCGCACAUUGAGGAAACCGAGUUGCAU
AUCUCAGAGUAUUGGCCCCGUGUAGGUUAUUCUUGACAGACAGUGGAGCUUAU
UCACUCCCAGGAUCCGAGUCGCAUACUACGGUACUGGUGACAGACCUAGGUCGUC
AGUUGACCAGUCCGCCACUAGACGUGAGUCCGUCAAAGCAGUUAGAUGUUACACU
CUAUUAGAUCUCGGAUUACAGCUGGAAGGAGCAGGAGUAGUGUUCUUGCUCUAA
GUACCGAGUGUGCCCAAUACCCGAUCAGCUUAUUAACGAACGGCUCCUCCUCUUA
GACUGCAGCGUAAGUGCGGAAUCUGGGGAUCAAAUUACUGACUGCCUGGAUUAC
CCUCGGACAUAUAACCUUGUAGCACGCUGUUGCUGUAUAGGUGACCAACGCCCAC
UCGAGUAGACCAGCUCUCUUAGUCCGGACAAUGAUAGGAGGCGCGGUCAAUCUAC

UUCUGGCUAGUUAAGAAUAGGCUGCACCGACCUCUAUAAGUAGCGUGUCCUCUA
GAGCUACGCAGGUUCGCAAUAAAAGCGUUGAUUAGUGUGCAUAGAACAGACCUCU
UAUUCGGUGAAACGCCAGAAUGCUAAAUUCCAAUAACUCUUCCCAAAACGCGUAC
GGCCGAAGACGCGCUUAUCUUGUGUACGUUCUCGCACAUGGAAGAAUCAGCG
GGCAUGGUGGUAGGGCAAUAGGGGAGCUGGGUAGCAGCGAAAAGGGCCCCUGC
GCACGUAGCUUCGCUGUUCGUCUGAAACAACCCGGCAUCCGUUGUAGCGAUCCCG
UUAUCAGUGUUAUUCUUGUGCGCACUAAGAUUCAUGGUGUAGUCGACAAUAACA
GCGUCUUGGCAGAUUCUGGUCACGUGCCCUAUGCCCGGGCUUGUGCCUCUCAGG
UGCACAGCGAUACUUAAAGCCUUCAAGGUACUCGACGUGGGUACCGAUUCGUGAC
ACUUCCUAAGAUUAUUCCACUGUGUUAGCCCCGCACCGCCGACCUAAACUGGUCC
AAUGUAUACGCAUUCGCUGAGCGGAUCGAUAAUAAAGCUUGAAUU (SEQ ID NO:
89)

GGGAGAAAGCUCAAGCUUAUCCAAGUAGGCUGGUCACCUGUACAACGUAGCCGGU
AUUUUUUUUUUUUUUUUUUUUUUGACCGUCUCAAGGUCCAAGUUAGUCUGCCU
AUAAAGGUGCGGAUCCACAGCUGAUGAAAGACUUGUGCGGUACGGUUAAUCUCC
CCUUUUUUUUUUUUUUUUUUUUUUAGUAAAUGCGUCUACUGAAUCCAGCGAUGA
UGCUGGCCCAGAUC (SEQ ID NO: 90)

GGGAGAAAGCUCAAGCUUAUCCAAGUAGGCUGGUCACCUGUACAACGUAGCCGGU
AUUUUUUUUUUUUUUUUUUUUUGACCGUCUCAAGGUCCAAGUUAGUCUGCCU
AUAAAGGUGCGGAUCCACAGCUGAUGAAAGACUUGUGCGGUACGGUUAAUCUCC
CCUUUUUUUUUUUUUUUUUUUUUAGUAAAUGCGUCUACUGAAUCCAGCGAUGA
UGCUGGCCCAGAUCUUCGACCACAAGUGCAUAUAGUAGUCAUCGAGGGUCGCCU
UUUUUUUUUUUUUUUUUUUUUUGGCCCAGUUCUGAGACUUCGCUAGAGACUAC
AGUUACAGCUGCAGUAGUAACCACUGCGGCUAUUGCAGGAAAUCCCGUUCAGGU
UUUUUUUUUUUUUUUUUUUUUUCCGCUCACUAUGAUUAAGAACCAGGUGGAGUGU
CACUGCUCUCGAGGUCUCACGAGAGCGCUCGAUACAGUCCUUGGAAGAAUCUUU
UUUUUUUUUUUUUUUUUUUUUGUGCGACGAUCACAGAGAACUUCUAUUCAUGCAG
GUCUGCUCUA (R 722 SEQ ID NO: 91)

GGGAGAAAGCUCAAGCUUAUCCAAGUAGGCUGGUCACCUGUACAACGUAGCCGGU
AUUUUUUUUUUUUUUUUUUUUUGACCGUCUCAAGGUCCAAGUUAGUCUGCCU
AUAAAGGUGCGGAUCCACAGCUGAUGAAAGACUUGUGCGGUACGGUUAAUCUCC
CCUUUUUUUUUUUUUUUUUUUUUAGUAAAUGCGUCUACUGAAUCCAGCGAUGA
UGCUGGCCCAGAUCUUCGACCACAAGUGCAUAUAGUAGUCAUCGAGGGUCGCCU
UUUUUUUUUUUUUUUUUUUUUUGGCCCAGUUCUGAGACUUCGCUAGAGACUAC
AGUUACAGCUGCAGUAGUAACCACUGCGGCUAUUGCAGGAAAUCCCGUUCAGGU
UUUUUUUUUUUUUUUUUUUUUCCGCUCACUAUGAUUAAGAACCAGGUGGAGUGU
CACUGCUCUCGAGGUCUCACGAGAGCGCUCGAUACAGUCCUUGGAAGAAUCUUU
UUUUUUUUUUUUUUUUUUUUUGUGCGACGAUCACAGAGAACUUCUAUUCAUGCAG
GUCUGCUCUAGAACGAACUGACCUGACGCCUGAACUUAUGAGCGUGCGUAUUUU
UUUUUUUUUUUUUUUUUUUUCCUCCCAACAAAUGUCGAUCAAUAGCUGGGCUGU
UGGAGACGCGUCAGCAAAUGCCGUGGCUCCAUAGGACGUGUAGACUUCUAUUUU
UUUUUUUUUUUUUUUUUUUUCCCGGGACCACAAAUAAUAUUCUUGCUUGGUUGGGC
GCAAGGGCCCCGUAUCAGGUCAUAAACGGGUACAUGUUGCACAGGCUCCUUUUU
UUUUUUUUUUUUUUUUUUUUCGCUGAGUUAUUCCGGUCUCAAAGACGGCAGACG
UCAGUCGACAACACGGUCUAAAGCAGUGCUACAAUCUGCCGUGUUCGUGUUUUU
UUUUUUUUUUUUUUUUUGUGAACCUACACGGCGUGCACUGUAGUUCGCAAUUCAU
AGGGUACCGGCUCAGAGUUAUGCCUUGGUUGAAAACUGCCCAGCAUACUUUUUU

UUUUUUUUUUUUUUCAUAUUCCCAUGCUAAGCAAGGGAUGCCGCGAGUCAUGU
UAAGCUUGAAUU (SEQ ID NO: 92)

According to another very particularly preferred aspect, the nucleic acid sequence according to formula (IV), used as the at least one immunostimulatory nucleic acid sequence of the adjuvant component as defined herein, may be selected from e.g. any of the following sequences: 
UAGCGAAGCUCUUGGACCUACCUUUUUUUUUUUUUUCCCUGCGUUCCUAGAAGU
ACACG (SEQ ID NO: 93)
 or

UAGCGAAGCUCUUGGACCUACCUUUUUUUUUUUUUUCCCUGCGUUCCUA
GAAGUACACGAUCGCUUCGAGAACCUGGAUGGAAAAAAAAAAAAAAGGGACGCAA
GGAUCUUCAUGUGC (SEQ ID NO: 94)

According to a last particularly preferred aspect, an immunostimulatory nucleic acid sequence, particularly an isRNA, used as the at least one immunostimulatory nucleic acid sequence, may be a nucleic acid encoding an antigen as defined below, preferably an RNA or mRNA encoding an antigen as defined below.

The inventive vaccine composition comprises an adjuvant component comprising or consisting of at least one immunostimulatory nucleic acid sequence, complexed with a complexing agent as defined herein. In general, such a complexing agent is preferably any compound, which may be capable of complexing and preferably thereby condensing the immunostimulatory nucleic acid sequence contained in the adjuvant component. Such a complexing agent may be selected from e.g. peptides, proteins, polymers or any further compound capable of complexing and condensing a nucleic acid sequence.
In the context of the present invention, a "complexed" nucleic acid sequence is usually to be understood as a nucleic acid sequence as defined herein, preferably the at least one immunostimulatory nucleic acid sequence of the adjuvant component, which interacts with one or more complexing agent(s) as defined herein by forming a non-covalent complex between the (immunostimulatory) nucleic acid sequence and the complexing agent. Herein, "non-covalent" means that a reversible association of the (immunostimulatory) nucleic acid sequence and the complexing agent is formed by non-covalent interactions of these molecules, wherein the molecules are associated together by any type of interaction of electrons, other than a covalent bond, particularly electrostatic interactions, preferably electrostatic interactions due to charged residues contained in the molecule, but also via hydrogen bonds, salt bridges and van der Waals interactions, which provide attractive forces between molecules. Association of a nucleic acid sequence, e.g. the at least one immunostimulatory nucleic acid sequence of the adjuvant component, and at least one complexing agent may be in equilibrium with dissociation of that complex, however, when using cationic or polycationic compounds as defined herein for complexing, usually no dissociation of the formed complex occurs and thus the equilibrium is preferably fully shifted towards the complex.

In the inventive vaccine composition, the complexing agent is a cationic or polycationic compound, more specifically a cationic or polycationic peptide or protein. The so called "adjuvant component" of the inventive vaccine composition is then preferably prepared by complexing the at least one immunostimulatory nucleic acid sequence of the adjuvant component with said cationic or polycationic compound, preferably in a specific ratio, to form a stable complex. In this context, it is preferably important, that no free cationic or polycationic compound or only a negligible small amount of free cationic or polycationic compound remains in the adjuvant component after complexing the at least one immunostimulatory nucleic acid sequence of the adjuvant component.

The term "cationic or polycationic compound" generally refers to a positively charged molecule, which is preferably positively charged (cation) at a pH value of about 1 to 9, more preferably of a pH value of or below 9, of or below 8, of or below 7, most preferably at physiological pH values, e.g. about 7.3 to 7.4. Accordingly, a cationic or polycationic compound, e.g. cationic peptide, protein or polymer as described herein, is preferably positively charged under physiological conditions, particularly under physiological salt conditions of the cell *in vivo.* The definition "cationic" may also refer to "polycationic" components and *vice versa.*

The ratio of the at least one immunostimulatory nucleic acid sequence and the cationic or polycationic compound in the adjuvant component is typically selected from a range which allows for entirely complexing the at least one immunostimulatory nucleic acid sequence in such a way that no free cationic or polycationic compound or only a negligible small amount thereof remains in the mixture. Preferably the ratio of the at least one immunostimulatory nucleic acid sequence of the adjuvant component to the cationic or polycationic compound is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w). For the inventive vaccine composition, the ratio of the at least one immunostimulatory nucleic acid sequence of the adjuvant component to the cationic or polycationic compound in the adjuvant component, which is present as a protein or peptide, may also be calculated on the basis of the nitrogen/phosphate ratio (N/P-ratio) of the entire adjuvant component, which resembles the ratio of nucleic acid sequences to peptides used and present in the complex. In the context of the present invention, an N/P-ratio is preferably in the range of about 0.01-4, preferably in a range of about 0.01-3, 0.01-2 or even 0.1-2, and more preferably in a range of about 0.1-1.5, 0.1-1.0, 0.3-2, 0.3-1.0 or 0.4-2 regarding the ratio of immunostimulatory nucleic acid sequence : cationic or polycationic peptide in the complex. Likewise preferably, this ratio is in the range of about 0.4-1.5. Such an N/P ratio preferably ensures a net negative or at least a neutral charge of the entire adjuvant component, which allows a further effective packaging of the adjuvant component, preferably in complex with the antigen as defined herein, with the carrier molecule as defined herein. Even more preferably, the adjuvant component, preferably in complex with the antigen as defined herein, comprises a negative net charge to allow more efficient binding of the adjuvant component, preferably in complex with the antigen as defined herein, and the carrier molecule as defined according to the inventive vaccine composition. This avoids the risk of repelling charges and thus the risk of an early degradation of the inventive vaccine composition prior to targeting a cell or tissue as a whole. Accordingly, the N/P ratios of immunostimulatory nucleic acid sequence : cationic or polycationic peptide in the adjuvant component may be most preferably in the range of about 0.01-2 or 0.1-1.5 regarding the ratio of nucleic acids: cationic or polycationic peptide in the complex, and likewise most preferably in the range of about 0.1-1.

In the context of the present disclosure, a cationic or polycationic compound is in general preferably selected from any cationic or polycationic compound suitable for complexing and thereby stabilizing (and condensing) a nucleic acid sequence, particularly the at least one immunostimulatory nucleic acid sequence of the adjuvant component, e.g. by associating the at least one immunostimulatory nucleic acid sequence of the adjuvant component with the cationic or polycationic compound. Such a cationic or polycationic compound *per se* does not need to exhibit any adjuvant properties, since an adjuvant property, particularly the capability of inducing an innate immune response, is preferably created upon complexing the at least one immunostimulatory nucleic acid sequence of the adjuvant component with the cationic or polycationic compound. The inventive vaccine composition comprises a cationic or polycationic peptide or a protein as complexing agent. Particularly preferred, cationic or polycationic peptides or proteins may be selected from protamine, nucleoline, spermine or spermidine, poly-L-lysine (PLL), basic polypeptides, poly-arginine, cell penetrating peptides (CPPs), chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia*)*,* pAntp, plsl, etc., antimicrobial-derived CPPs e.g. Buforin-2, Bac715-24, SynB, SynB(1), pVEC, hCT-derived peptides, SAP, MAP, KALA, PpTG20, Proline-rich peptides, L-oligomers, Arginine-rich peptides, Calcitonin-peptides, FGF, Lactoferrin, poly-L-Lysine, poly-Arginine, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, Calcitonin peptide(s), etc.

Further cationic or polycationic compounds, which can (according to non-claimed embodiments further disclosed herein) be used for complexing the at least one immunostimulatory nucleic acid sequence of the adjuvant component above, may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers , such as PMOXA-PDMS copolymers, etc., Blockpolymers consisting of a combination of one or more cationic blocks (e.g. selected og a cationic polymer as mentioned above) and of one or more hydrophilic- or hydrophobic blocks (e.g polyethyleneglycole); etc. Association or complexing the at least one immunostimulatory nucleic acid sequence of the adjuvant component with cationic or polycationic compounds preferably provides adjuvant properties to the at least one nucleic acid sequence and confers a stabilizing effect to the at least one immunostimulatory nucleic acid sequence of the adjuvant component by complexation. The procedure for stabilizing the at least one immunostimulatory nucleic acid is in general as described in EP-A-1083232.

Further preferred cationic or polycationic compounds, which can be used for complexing the at least one immunostimulatory nucleic acid sequence of the adjuvant component above, may include oligopeptides having the following sum formula (V):

{(Arg)ₗ;(Lys)ₘ;(His)ₙ;(Orn)ₒ;(Xaa)ₓ};

wherein l + m + n +o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10%, more preferably at least 20%, at least 30%, at least 40%, at least 50% (e.g. at least 50, 51, 52, 53, 54, 55, 56, 57, 58, or 59%) at least 60% (e.g. at least 60, 61, 62, 63, 64, 65, 66, 67, 68, or 69%), at least 70% (e.g. at least 70, 71, 72, 73, 74, 75, 76, 77, 78, or 79%), at least 80% (at least 80, 81, 82, 83, 84, 85, 86, 87, 88, or 89%) at least 90% (e.g. at least 90, 91, 92, 93, 94, 95, 96, 97, 98, or 99%), or even 100% of all amino acids of the oligopeptide of formula (V); and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide of formula (V), or at least 80%, at least 70%, at least 60%, at least 50% (e.g. at least 50, 51, 52, 53, 54, 55, 56, 57, 58, or 59%), at least 40 % (e.g. at least 40, 41, 42, 43, 44, 45, 46, 47, 48, or 49%), at least 30 % (e.g. at least 30, 31, 32, 33, 33, 35, 36, 37, 38, or 39%), at least 20 % (e.g. at least 20, 21, 22, 23, 22, 25, 26, 27, 28, or 29%), at least 10 % (e.g. at least 10, 11, 12, 13, 11, 15, 16, 17, 18, or 19%), or at least 9, 8, 7, 6, 5, 4, 3, 2, 1 or even 0%. Any of amino acids Arg, Lys, His, Orn and Xaa may be positioned at any place of the oligopeptide of formula (V). In this context cationic peptides or proteins (preferably of formula (V)) in the range of 7-30 amino acids are particular preferred.

According to a particular preferred aspect, oligopeptides of formula (V) as shown above, may, without being restricted thereto, comprise at least one of the following subgroup of formulae:
Arg₇, Arg₈, Arg₉, Arg₁₀, Arg₁₁, Arg₁₂, Arg₁₃, Arg₁₄, Arg₁₅, Arg₁₆, Arg₁₇, Arg₁₈, Arg₁₉, Arg₂₀, Arg₂₁, Arg₂₂, Arg₂₃, Arg₂₄, Arg₂₅, Arg₂₆, Arg₂₇, Arg₂₈, Arg₂₉, Arg₃₀, (SEQ ID NOs: 95-118);
Lys₇, Lys₈, Lys₉, Lys₁₀, Lys₁₁, Lys₁₂, Lys₁₃, Lys₁₄, Lys₁₅, Lys₁₆, Lys₁₇, Lys₁₈, Lys₁₉, Lys₂₀, Lys₂₁, Lys₂₂, Lys₂₃, Lys₂₄, Lys₂₅, Lys₂₆, Lys₂₇, Lys₂₈, Lys₂₉, Lys₃₀, (SEQ ID NOs: 119-142);
His₇, His₈, His₉, His₁₀, His₁₁, His₁₂, His₁₃, His₁₄, His₁₅, His₁₆, His₁₇, His₁₈, His₁₉, His₂₀, His₂₁, His₂₂, His₂₃, His₂₄, His₂₅, His₂₆, His₂₇, His₂₈, His₂₉, His₃₀, (SEQ ID NOs: 143-166);
Orn₇, Orn₈, Orn₉, Orn₁₀, Orn₁₁, Orn₁₂, Orn₁₃, Orn₁₄, Orn₁₅, Orn₁₆, Orn₁₇, Orn₁₈, Orn₁₉, Orn₂₀, Orn₂₁, Orn₂₂, Orn₂₃, Orn₂₄, Orn₂₅, Orn₂₆, Orn₂₇, Orn₂₈, Orn₂₉, Orn₃₀, (SEQ ID NOs: 167-190);

According to a further particularly preferred aspect, oligopeptides of formula (V) as shown above may be preferably selected from, without being restricted thereto, at least one of the following subgroup of formulae. The following formulae (as with formula (V)) do not specify any amino acid order, but are intended to reflect sum formulae by exclusively specifying the (number of) amino acids as components of the respective peptide. Accordingly, as an example, formula Arg₍₇₋₂₉₎Lys₁ is intended to mean that peptides falling under this formula contain 7 to 19 Arg residues and 1 Lys residue of whatsoever order. If the peptides contain 7 Arg residues and 1 Lys residue, all variants having 7 Arg residues and 1 Lys residue are encompassed. The Lys residue may therefore be positioned anywhere in the e.g. 8 amino acid long sequence composed of 7 Arg and 1 Lys residues. The subgroup preferably comprises:
Arg₍₄₋₂₉₎Lys₁, Arg₍₄₋₂₉₎His₁, Arg₍₄₋₂₉Orn₁, Lys₍₄₋₂₉₎His₁, Lys₍₄₋₂₉₎Orn₁, His₍₄₋₂₉₎Orn₁, Arg₍₃₋₂₈₎Lys₂, Arg₍₃₋₂₈₎His₂, Arg₍₃₋₂₈₎Orn₂, Lys₍₃₋₂₈₎His₂, Lys₍₃₋₂₈₎Orn₂, His₍₃₋₂₈₎Orn₂, Arg₍₂₋₂₇₎Lys₃, Arg₍₂₋₂₇₎His₃, Arg₍₂₋₂₇₎Orn₃, Lys₍₂₋₂₇₎His₃, Lys₍₂₋₂₇₎Orn₃, His₍₂₋₂₇₎Orn₃, Arg₍₁₋₂₆₎Lys₄, Arg₍₁₋₂₆₎His₄, Arg₍₁₋₂₆₎Orn₄, Lys₍₁₋₂₆₎His₄, Lys₍₁₋₂₆₎Orn₄, His₍₁₋₂₆₎Orn₄,
Arg₍₃₋₂₈₎Lys₁His₁, Arg₍₃₋₂₈₎Lys₁Orn₁, Arg₍₃₋₂₈₎His₁Orn₁, Arg₁Lys₍₃₋₂₈₎His₁, Arg₁Lys₍₃₋₂₈₎Orn₁, Lys₍₃₋₂₈₎His₁Orn₁, Arg₁Lys₁His₍₃₋₂₈₎, Arg₁His₍₃₋₂₈₎Orn₁, Lys₁His₍₃₋₂₈₎Orn₁;
Arg₍₂₋₂₇₎Lys₂His₁, Arg(₂₋₂₇₎Lys₁His₂, Arg₍₂₋₂₇₎Lys₂Orn₁, Arg₍₂₋₂₇₎Lys₁Orn₂, Arg₍₂₋₂₇₎His₂Orn₁, Arg₍₂₋₂₇₎His₁Orn₂, Arg₂Lys₍₂₋₂₇₎His₁, Arg₁Lys₍₂₋₂₇₎His₂, Arg₂Lys₍₂₋₂₇₎Orn₁, Arg₁Lys₍₂₋₂₇₎Orn₂, Lys₍₂₋₂₇₎His₂Orn₁, Lys₍₂₋₂₇₎His₁Orn₂, Arg₂Lys₁His₍₂₋₂₇₎, Arg₁Lys₂His₍₂₋₂₇₎, Arg₂His₍₂₋₂₇₎Orn₁, Arg₁His₍₂₋₂₇₎Orn₂, Lys₂His₍₂₋₂₇₎Orn₁, Lys₁His₍₂₋₂₇₎Orn₂;
Arg₍₁₋₂₆₎Lys₃His₁, Arg₍₁₋₂₆₎Lys₂His₂, Arg₍₁₋₂₆₎Lys₁His₃, Arg₍₁₋₂₆₎Lys₃Orn₁, Arg₍₁₋₂₆₎Lys₂Orn₂, Arg₍₁₋₂₆₎Lys₁Orn₃, Arg₍₁₋₂₆₎His₃Orn₁, Arg₍₁₋₂₆₎His₂Orn₂, Arg₍₁₋₂₆₎His₁Orn₃, Arg₃Lys₍₁₋₂₆₎His₁, Arg₂Lys₍₁₋₂₆₎His₂, Arg₁Lys₍₁₋₂₆₎His₃, Arg₃Lys₍₁₋₂₆₎Orn₁, Arg₂Lys₍₁₋₂₆₎Orn₂, Arg₁Lys₍₁₋₂₆₎Orn₃, Lys₍₁₋₂₆₎His₃Orn₁, Lys₍₁₋₂₆₎His₂Orn₂, Lys₍₁₋₂₆₎His₁Orn₃, Arg₃Lys₁His₍₁₋₂₆₎, Arg₂Lys₂His₍₁₋₂₆₎, Arg₁Lys₃His₍₁₋₂₆₎, Arg₃His₍₁₋₂₆₎Orn₁, Arg₂His₍₁₋₂₆₎Orn₂, Arg₁His₍₁₋₂₆₎Orn₃, Lys₃His₍₁₋₂₆₎Orn₁, Lys₂His₍₁₋₂₆₎Orn₂, Lys₁His₍₁₋₂₆₎Orn₃;
Arg₍₂₋₂₇₎Lys₁His₁Orn₁, Arg₁Lys₍₂₋₂₇₎His₁Orn₁, Arg₁Lys₁His₍₂₋₂₇₎Orn₁, Arg₁Lys₁His₁Orn₍₂₋₂₇₎;
Arg₍₁₋₂₆₎Lys₂His₁Orn₁, Arg₍₁₋₂₆₎Lys₁His₂Orn₁, Arg₍₁₋₂₆₎Lys₁His₁Orn₂, Arg₂Lys₍₁₋₂₆₎His₁Orn₁, Arg₁Lys₍₁₋₂₆₎His₂Orn₁, Arg₁Lys₍₁₋₂₆₎His₁Orn₂, Arg₂Lys₁His₍₁₋₂₆₎Orn₁, Arg₁Lys₂His₍₁₋₂₆₎Orn₁, Arg₁Lys₁His₍₁₋₂₆₎Orn₂, Arg₂Lys₁His₁Orn₍₁₋₂₆₎, Arg₁Lys₂His₁Orn₍₁₋₂₆₎, Arg₁Lys₁His₂Orn₍₁₋₂₆₎;
Arg₍₇₋₁₄₎Lys₁, Arg₍₇₋₁₄₎His₁, Arg₍₇₋₁₄₎Orn₁, Lys₍₇₋₁₄₎His₁, Lys₍₇₋₁₄₎Orn₁, His₍₇₋₁₄₎Orn₁,;
   Arg₍₆₋₁₃₎Lys₂, Arg₍₆₋₁₃₎His₂, Arg₍₆₋₁₃₎Orn₂, Lys₍₆₋₁₃₎His₂, Lys₍₆₋₁₃₎Orn₂, His₍₆₋₃₎Orn₂,;
   Arg₍₅₋₁₂₎Lys₃, Arg₍₅₋₁₂₎His₃, Arg₍₅₋₁₂₎Orn₃, Lys₍₅₋₁₂₎His₃, Lys₍₅₋₁₂₎Orn₃, His₍₅₋₁₂₎Orn₃,;
   Arg₍₄₋₁₎Lys₄, Arg₍₄₋₁₁₎His₄, Arg₍₄₋₁₁₎Orn₄, Lys₍₄₋₁₁₎His₄, Lys₍₄₋₁₎Orn₄, His₍₄₋₁₁₎Orn₄,;
   Arg₍₃₋₁₀₎Lys₅, Arg₍₃₋₁₀₎His₅, Arg₍₃₋₁₀₎Orn₅, Lys₍₃₋₁₀₎His₅, Lys₍₃₋₁₀₎Orn₅, His₍₃₋₁₀₎Orn₅,;
   Arg₍₂₋₉₎Lys₆, Arg₍₂₋₉₎His₆, Arg₍₂₋₉₎Orn₆, Lys₍₂₋₉₎His₆, Lys₍₂₋₉₎Orn₆, His₍₂₋₉₎Orn₆,;
   Arg₍₁₋₈₎Lys₇, Arg₍₁₋₈₎His₇, Arg₍₁₋₈₎Orn₇, Lys₍₁₋₈₎His₇, Lys₍₁₋₈₎Orn₇, His₍₁₋₈₎Orn₇,;
or
Arg₍₆₋₁₃₎Lys₁His₁, Arg₍₆₋₁₃₎Lys₁Orn₁, Arg₍₆₋₁₃₎His₁Orn₁, Arg₁Lys₍₆₋₁₃₎His₁, Arg₁Lys₍₆₋₁₃₎Orn₁, Lys₍₆₋₁₃₎His₁Orn₁, Arg₁Lys₁His₍₆₋₁₃₎, Arg₁His₍₆₋₁₃₎Orn₁, Lys₁His₍₆₋₁₃₎Orn₁;
Arg₍₅₋₁₂₎Lys₂His₁, Arg₍₅₋₁₂₎Lys₁His₂, Arg₍₅₋₁₂₎Lys₂Orn₁, Arg₍₅₋₁₂₎Lys₁Orn₂, Arg₍₅₋₁₂₎His₂Orn₁, Arg₍₅₋₁₂₎His₁Orn₂, Arg₂Lys₍₅₋₁₂₎His₁, Arg₁Lys₍₅₋₁₂₎His₂, Arg₂Lys₍₅₋₁₂₎Orn₁, Arg₁Lys₍₅₋₁₂₎Orn₂, Lys₍₅₋₁₂₎His₂Orn₁, Lys₍₅₋₁₂₎His₁Orn₂, Arg₂Lys₁His₍₅₋₁₂₎, Arg₁Lys₂His₍₅₋₁₂₎, Arg₂His₍₅₋₁₂₎Orn₁, Arg₁His₍₅₋₁₂₎Orn₂, Lys₂His₍₅₋₁₂₎Orn₁, Lys₁His₍₅₋₁₂₎Orn₂;
Arg₍₄₋₁₁₎Lys₃His₁, Arg₍₄₋₁₁₎Lys₂His₂, Arg₍₄₋₁₁₎Lys₁His₃, Arg₍₄₋₁₁₎Lys₃Orn₁, Arg₍₄₋₁₁₎Lys₂Orn₂, Arg₍₄₋₁₁₎Lys₁Orn₃, Arg₍₄₋₁₁₎His₃Orn₁, Arg₍₄₋₁₁₎His₂Orn₂, Arg₍₄₋₁₁₎His₁Orn₃, Arg₃Lys₍₄₋₁₁₎His₁, Arg₂Lys₍₄₋₁₁₎His₂, Arg₁Lys₍₄₋₁₁₎His₃, Arg₃Lys₍₄₋₁₁₎Orn₁, Arg₂Lys₍₄₋₁₁₎Orn₂, Arg₁Lys₍₄₋₁₁₎Orn₃, Lys₍₄₋₁₁₎His₃Orn₁, Lys₍₄₋₁₁₎His₂Orn₂, Lys₍₄₋₁₁₎His₁Orn₃, Arg₃Lys₁His₍₄₋₁₁₎, Arg₂Lys₂His₍₄₋₁₁₎, Arg₁Lys₃His₍₄₋₁₁₎, Arg₃His₍₄₋₁₁₎Orn₁, Arg₂His₍₄₋₁₁₎Orn₂, Arg₁His₍₄₋₁₁₎Orn₃, Lys₃His₍₄₋₁₁₎Orn₁, Lys₂His₍₄₋₁₎Orn₂, Lys₁His₍₄₋₁₁₎Orn₃;
Arg₍₃₋₁₀₎Lys₄His₁, Arg₍₃₋₁₀₎Lys₃His₂, Arg₍₃₋₁₀₎Lys₂His₃, Arg₍₃₋₁₀₎Lys₁His₄, Arg₍₃₋₁₀₎Lys₄Orn₁, Arg₍₃₋₁₀₎Lys₃Orn₂, Arg₍₃₋₁₀₎Lys₂Orn₃, Arg₍₃₋₁₀₎Lys₁Orn₄, Arg₍₃₋₁₀₎His₄Orn₁, Arg₍₃₋₁₀₎His₃Orn₂, Arg₍₃₋₁₀₎His₂Orn₃, Arg₍₃₋₁₀₎His₁Orn₄, Arg₄Lys₍₃₋₁₀₎His₁, Arg₃Lys₍₃₋₁₀₎His₂, Arg₂Lys₍₃₋₁₀₎His₃, Arg₁Lys₍₃₋₁₀₎His₄, Arg₄Lys₍₃₋₁₀₎Orn₁, Arg₃Lys₍₃₋₁₀₎Orn₂, Arg₂Lys₍₃₋₁₀₎Orn₃, Arg₁Lys₍₃₋₁₀₎Orn₄, Lys₍₃₋₁₀₎His₄Orn₁, Lys₍₃₋₁₀₎His₃Orn₂, Lys₍₃₋₁₀₎His₂Orn₃, Lys₍₃₋₁₀₎His₁Orn₄, Arg₄Lys₁His₍₃₋₁₀₎, Arg₃Lys₂His₍₃₋₁₀₎, Arg₂Lys₃His₍₃₋₁₀₎, Arg₁Lys₄His₍₃₋₁₀₎, Arg₄His₍₃₋₁₀₎Orn₁, Arg₃His₍₃₋₁₀₎Orn₂, Arg₂His₍₃₋₁₀₎Orn₃, Arg₁His₍₃₋₁₀₎Orn₄, Lys₄His₍₃₋₁₀₎Orn₁, Lys₃His₍₃₋₁₀₎Orn₂, Lys₂His₍₃₋₁₀₎Orn₃, Lys₁His₍₃₋₁₀₎Orn₄;
Arg₍₂₋₉₎Lys₅His₁, Arg₍₂₋₉₎Lys₄His₂, Arg₍₂₋₉₎Lys₃His₃, Arg₍₂₋₉₎Lys₂His₄, Arg₍₂₋₉₎Lys₁His₅, Arg₍₂₋₉₎Lys₅Orn₁, Arg₍₂₋₉₎Lys₄Orn₂, Arg₍₂₋₉₎Lys₃Orn₃, Arg₍₂₋₉₎Lys₂Orn₄, Arg₍₂₋₉₎Lys₁Orn₅, Arg₍₂₋₉₎His₅Orn₁, Arg₍₂₋₉₎His₄Orn₂, Arg₍₂₋₉₎His₃Orn₃, Arg₍₂₋₉₎His₂Orn₄, Arg₍₂₋₉₎His₁Orn₃, Arg₅Lys₍₂₋₉₎His₁, Arg₄Lys₍₂₋₉₎His₂, Arg₃Lys₍₂₋₉₎His₃, Arg₂Lys₍₂₋₉₎His₄, Arg₁Lys₍₂₋₉₎His₅, Arg₅Lys₍₂₋₉₎Orn₁, Arg₄Lys₍₂₋₉₎Orn₂, Arg₃Lys₍₂₋₉₎Orn₃, Arg₂Lys₍₂₋₉₎Orn₄, Arg₁Lys₍₂₋₉₎Orn₅, Lys₍₂₋₉₎His₅Orn₁, Lys₍₂₋₉₎His₄Orn₂, Lys₍₂₋₉₎His₃Orn₃, Lys₍₂₋₉₎His₂Orn₄, Lys₍₂₋₉₎His₁Orn₅, Arg₅Lys₁His₍₂₋₉₎, Arg₄Lys₂His₍₂₋₉₎, Arg₃Lys₃His₍₂₋₉₎, Arg₂Lys₄His₍₂₋₉₎, Arg₁Lys₅His₍₂₋₉₎, Arg₅His₍₂₋₉₎Orn₁, Arg₄His₍₂₋₉₎Orn₂, Arg₃His₍₂₋₉₎Orn₃, Arg₂His₍₂₋₉₎Orn₄, Arg₁His₍₂₋₉₎Orn₅, Lys₅His₍₂₋₉₎Orn₁, Lys₄His₍₂₋₉₎Orn₂, Lys₃His₍₂₋₉₎Orn₃, Lys₂His₍₂₋₉₎Orn₄, Lys₁His₍₂₋₉₎Orn₅;
Arg₍₁₋₈₎Lys₆His₁, Arg₍₁₋₈₎Lys₅His₂, Arg₍₁₋₈₎Lys₄His₃, Arg₍₁₋₈₎Lys₃His₄, Arg₍₁₋₈₎Lys₂His₅, Arg₍₁₋₈₎Lys₁His₆, Arg₍₁₋₈₎Lys₆Orn₁, Arg₍₁₋₈₎Lys₅Orn₂, Arg₍₁₋₈₎Lys₄Orn₃, Arg₍₁₋₈₎Lys₃Orn₄, Arg₍₁₋₈₎Lys₂Orn₅, Arg₍₁₋₈₎Lys₁Orn₆, Arg₍₁₋₈₎His₆Orn₁, Arg₍₁₋₈₎His₅Orn₂, Arg₍₁₋₈₎His₄Orn₃, Arg₍₁₋₈₎His₃Orn₄, Arg₍₁₋₈₎His₂Orn₅, Arg₍₁₋₈₎His₁Orn₆, Arg₆Lys₍₁₋₈₎His₁, Arg₅Lys₍₁₋₈₎His₂, Arg₄Lys₍₁₋₈₎His₃, Arg₃Lys₍₁₋₈₎His₄, Arg₂Lys₍₁₋₈₎His₅, Arg₁Lys₍₁₋₈₎His₆, Arg₆Lys₍₁₋₈₎Orn₁, Arg₅Lys₍₁₋₈₎Orn₂, Arg₄Lys₍₁₋₈₎Orn₃, Arg₃Lys₍₁₋₈₎Orn₄, Arg₂Lys₍₁₋₈₎Orn₅, Arg₁Lys₍₁₋₈₎Orn₆, Lys₍₁₋₈₎His₆Orn₁, Lys₍₁₋₈₎His₅Orn₂, Lys₍₁₋₈₎His₄Orn₃, Lys₍₁₋₈₎His₃Orn₄, Lys₍₁₋₈₎His₂Orn₅, Lys₍₁₋₈₎His₁Orn₆, Arg₆Lys₁His₍₁₋₈₎, Arg₅Lys₂His₍₁₋₈₎, Arg₄Lys₃His₍₁₋₈₎, Arg₃Lys₄His₍₁₋₈₎, Arg₂Lys₅His₍₁₋₈₎, Arg₁Lys₆His₍₁₋₈₎, Arg₆His₍₁₋₈₎Orn₁, Arg₅His₍₁₋₈₎Orn₂, Arg₄His₍₁₋₈₎Orn₃, Arg₃His₍₁₋₈₎Orn₄, Arg₂His₍₁₋₈₎Orn₅, Arg₁His₍₁₋₈₎Orn₆, Lys₆His₍₁₋₈₎Orn₁, Lys₅His₍₁₋₈₎Orn₂, Lys₄His₍₁₋₈₎Orn₃, Lys₃His₍₁₋₈₎Orn₄, Lys₂His₍₁₋₈₎Orn₅, Lys₁His₍₁₋₈₎Orn₆;
Arg₍₅₋₁₂₎Lys₁His₁Orn₁, Arg₁Lys₍₅₋₁₂₎His₁Orn₁, Arg₁Lys₁His₍₅₋₁₂₎Orn₁, Arg₁Lys₁His₁Orn₍₅₋₁₂₎;
Arg₍₄₋₁₁₎Lys₂His₁Orn₁, Arg₍₄₋₁₁₎Lys₁His₂Orn₁, Arg₍₄₋₁₁₎Lys₁His₁Orn₂, Arg₂Lys₍₄₋₁₁₎His₁Orn₁, Arg₁Lys₍₄₋₁₁₎His₂Orn₁, Arg₁Lys₍₄₋₁₁₎His₁Orn₂, Arg₂Lys₁His₍₄₋₁₁₎Orn₁, Arg₁Lys₂His₍₄₋₁₁₎Orn₁, Arg₁Lys₁His₍₄₋₁₁₎Orn₂, Arg₂Lys₁His₁Orn₍₄₋₁₁₎, Arg₁Lys₂His₁Orn₍₄₋₁₁₎, Arg₁Lys₁His₂Orn₍₄₋₁₁₎;
Arg₍₃₋₁₀₎Lys₃His₁Orn₁, Arg₍₃₋₁₀₎Lys₂His₂Orn₁, Arg₍₃₋₁₀₎Lys₂His₁Orn₂, Arg₍₃₋₁₀₎Lys₁His₂Orn₂, Arg₍₃₋₁₀₎Lys₁His₁Orn₃, Arg₃Lys₍₃₋₁₀₎His₁Orn₁, Arg₂Lys₍₃₋₁₀₎His₂Orn₁, Arg₂Lys₍₃₋₁₀₎His₁Orn₂, Arg₁Lys₍₃₋₁₀₎His₂Orn₂, Arg₁Lys₍₃₋₁₀₎His₁Orn₃, Arg₃Lys₁His₍₃₋₁₀₎Orn₁, Arg₂Lys₂His₍₃₋₁₀₎Orn₁, Arg₂Lys₁His₍₃₋₁₀₎Orn₂, Arg₁Lys₂His₍₃₋₁₀₎Orn₂, Arg₁Lys₁His₍₃₋₁₀₎Orn₃, Arg₃Lys₁His₁Orn₍₃₋₁₀₎, Arg₂Lys₂His₁Orn₍₃₋₁₀₎, Arg₂Lys₁His₂Orn₍₃₋₁₀₎, Arg₁Lys₂His₂Orn₍₃₋₁₀₎, Arg₁Lys₁His₃Orn₍₃₋₁₀₎;
Arg₍₂₋₉₎Lys₄His₁Orn₁, Arg₍₂₋₉₎Lys₁His₄Orn₁, Arg₍₂₋₉₎Lys₁His₁Orn₄, Arg₍₂₋₉₎Lys₃His₂Orn₁, Arg₍₂₋₉₎Lys₃His₁Orn₂, Arg₍₂₋₉₎Lys₂His₃Orn₁, Arg₍₂₋₉₎Lys₂His₁Orn₃, Arg₍₂₋₉₎Lys₁His₂Orn₃, Arg₍₂₋₉₎Lys₁His₃Orn₂, Arg₍₂₋₉₎Lys₂His₂Orn₂, Arg₄Lys₍₂₋₉₎His₁Orn₁, Arg₁Lys₍₂₋₉₎His₄Orn₁, Arg₁Lys₍₂₋₉₎His₁Orn₄, Arg₃Lys₍₂₋₉₎His₂Orn₁, Arg₃Lys₍₂₋₉₎His₁Orn₂, Arg₂Lys₍₂₋₉₎His₃Orn₁, Arg₂Lys₍₂₋₉₎His₁Orn₃, Arg₁Lys₍₂₋₉₎His₂Orn₃, Arg₁Lys₍₂₋₉₎His₃Orn₂, Arg₂Lys₍₂₋₉₎His₂Orn₂, Arg₄Lys₁His₍₂₋₉₎Orn₁, Arg₁Lys₄His₍₂₋₉₎Orn₁, Arg₁Lys₁His₍₂₋₉₎Orn₄, Arg₃Lys₂His₍₂₋₉₎Orn₁, Arg₃Lys₁His₍₂₋₉₎Orn₂, Arg₂Lys₃His₍₂₋₉₎Orn₁, Arg₂Lys₁His₍₂₋₉₎Orn₃, Arg₁Lys₂His₍₂₋₉₎Orn₃, Arg₁Lys₃His₍₂₋₉₎Orn₂, Arg₂Lys₂His₍₂₋₉₎Orn₂, Arg₄Lys₁His₁Orn₍₂₋₉₎, Arg₁Lys₄His₁Orn₍₂₋₉₎, Arg₁Lys₁His₄Orn₍₂₋₉₎, Arg₃Lys₂His₁Orn₍₂₋₉₎, Arg₃Lys₁His₂Orn₍₂₋₉₎, Arg₂Lys₃His₁Orn₍₂₋₉₎, Arg₂Lys₁His₃Orn₍₂₋₉₎, Arg₁Lys₂His₃Orn₍₂₋₉₎, Arg₁Lys₃His₂Orn₍₂₋₉₎, Arg₂Lys₂His₂Orn₍₂₋₉₎;
Arg₍₁₋₈₎Lys₅His₁Orn₁, Arg₍₁₋₈₎Lys₁His₅Orn₁, Arg₍₁₋₈₎Lys₁His₁Orn₅, Arg₍₁₋₈₎Lys₄His₂Orn₁, Arg₍₁₋₈₎Lys₂His₄Orn₁, Arg₍₁₋₈₎Lys₂His₁Orn₄, Arg₍₁₋₈₎Lys₁His₂Orn₄, Arg₍₁₋₈₎Lys₁His₄Orn₂, Arg₍₁₋₈₎Lys₄His₁Orn₂, Arg₍₁₋₈₎Lys₃His₃Orn₁, Arg₍₁₋₈₎Lys₃His₁Orn₃, Arg₍₁₋₈₎Lys₁His₃Orn₃, Arg₅Lys₍₁₋₈₎His₁Orn₁, Arg₁Lys₍₁₋₈₎His₅Orn₁, Arg₁Lys₍₁₋₈₎His₁Orn₅, Arg₄Lys₍₁₋₈₎His₂Orn₁, Arg₂Lys₍₁₋₈₎His₄Orn₁, Arg₂Lys₍₁₋₈₎His₁Orn₄, Arg₁Lys₍₁₋₈₎His₂Orn₄, Arg₁Lys₍₁₋₈₎His₄Orn₂, Arg₄Lys₍₁₋₈₎His₁Orn₂, Arg₃Lys₍₁₋₈₎His₃Orn₁, Arg₃Lys₍₁₋₈₎His₁Orn₃, Arg₁Lys₍₁₋₈₎His₃Orn₃, Arg₅Lys₁His₍₁₋₈₎Orn₁, Arg₁Lys₅His₍₁₋₈₎Orn₁, Arg₁Lys₁His₍₁₋₈₎Orn₅, Arg₄Lys₂His₍₁₋₈₎Orn₁, Arg₂Lys₄His₍₁₋₈₎Orn₁, Arg₂Lys₁His₍₁₋₈₎Orn₄, Arg₁Lys₂His₍₁₋₈₎Orn₄, Arg₁Lys₄His₍₁₋₈₎Orn₂, Arg₄Lys₁His₍₁₋₈₎Orn₂, Arg₃Lys₃His₍₁₋₈₎Orn₁, Arg₃Lys₁His₍₁₋₈₎Orn₃, Arg₁Lys₃His₍₁₋₈₎Orn₃, Arg₅Lys₁His₁Orn₍₁₋₈₎, Arg₁Lys₅His₁Orn₍₁₋₈₎, Arg₁Lys₁His₅Orn₍₁₋₈₎, Arg₄Lys₂His₁Orn₍₁₋₈₎, Arg₂Lys₄His₁Orn₍₁₋₈₎, Arg₂Lys₁His₄Orn₍₁₋₈₎, Arg₁Lys₂His₄Orn₍₁₋₈₎, Arg₁Lys₄His₂Orn₍₁₋₈₎, Arg₄Lys₁His₂Orn₍₁₋₈₎, Arg₃Lys₃His₁Orn₍₁₋₈₎, Arg₃Lys₁His₃Orn₍₁₋₈₎, Arg₁Lys₃His₃Orn₍₁₋₈₎;

According to another preferred aspect, the oligopeptide of formula (V) as shown above is selected from the subgroup comprising a sequence according to:

| | |
|---|---|
| His₃Arg₉ | His-His-His-Arg-Arg-Arg-Arg-Arg-Arg-Arg-Arg-Arg (SEQ ID NO: 191) |
| Arg₉His₃: | Arg-Arg-Arg-Arg-Arg-Arg-Arg-Arg-Arg-His-His-His (SEQ ID NO: 192) |
| His₃Arg₄His₃: | His-His-His-Arg-Arg-Arg-Arg-His-His-His (SEQ ID NO: 193) |
| His₃Arg₉His₃: | His-His-His-Arg-Arg-Arg-Arg-Arg-Arg-Arg-Arg-Arg-His-His-His (SEQ ID NO: 194) |
| His₆Arg₄His₆: | His-His-His-His-His-His-Arg-Arg-Arg-Arg-His-His-His-His-His- His (SEQ ID NO: 195) |
| His₆Arg₉His₆: | His-His-His-His-His-His-Arg-Arg-Arg-Arg-Arg-Arg-Arg-Arg- His-His-His-His-His-His (SEQ ID NO: 196) |
| TyrSer₂Arg₉Ser₂Tyr: | Tyr-Ser-Ser-Arg-Arg-Arg-Arg-Arg-Arg-Arg-Arg-Arg-Ser-Ser-Tyr (SEQ ID NO: 197) |
| His₃Arg₉SerSerTyr: | His-His-His-Arg-Arg-Arg-Arg-Arg-Arg-Arg-Arg-Arg-Ser-Ser-Tyr (SEQ ID NO: 198) |
| (ArgLysHis)₄: | Arg-Lys-His-Arg-Lys-His-Arg-Lys-His-Arg-Lys-His (SEQ ID NO: 199) |
| Tyr(ArgLysHis)₂Arg: | Tyr-Arg-Lys-His-Arg-Lys-His-Arg (SEQ ID NO: 200) |

Further preferred cationic or polycationic compounds, which can be used for complexing the at least one immunostimulatory nucleic acid sequence of the adjuvant component as defined above may comprise disulfide-crosslinked cationic compounds, i.e. cationic compounds, which have been formed by crosslinking at least two (same or different) disulfide-crosslinkable cationic components. In this context, disulfide-crosslinkable cationic components may generally be selected from any cationic or polycationic peptide, protein or polymer suitable for this purpose, preferably as defined above, wherein each such cationic or polycationic protein, peptide or polymer suitable as a disulfide-crosslinkable cationic component preferably contains at least one -SH moiety. Most preferably each such cationic or polycationic protein, peptide or polymer suitable as a disulfide-crosslinkable cationic component contains at least one cysteine residue or any further chemical group exhibiting an -SH moiety, capable to form a disulfide linkage upon condensation with at least one further cationic or polycationic protein, peptide or polymer as cationic component of the polymeric carrier as mentioned herein.

Each disulfide-crosslinked cationic compound is preferably formed by linking a disulfide-crosslinkable component as defined herein to a neighbouring disulfide-crosslinkable component as defined herein or other components via disulfide-crosslinking. Preferably, the disulfide-crosslinking is a (reversible) disulfide bond (-S-S-) between at least one cationic or polycationic protein, peptide or polymer, used as a disulfide-crosslinkable cationic component, and at least one further cationic or polycationic protein, peptide or polymer, likewise used as a disulfide-crosslinkable cationic component or any other component. The disulfide-crosslinking is typically formed by condensation of -SH-moieties of the disulfide-crosslinkable cationic components. Such an -SH-moiety may be part of the structure of the cationic or polycationic protein, peptide or polymer used as a disulfide-crosslinkable cationic component or may be added prior to disulfide-crosslinking by a modification as defined below. In this context, the sulphurs adjacent to one component of the disulfide-crosslinkable cationic component, necessary for providing a disulfide bond, may be provided by the disulfide-crosslinkable cationic component itself, e.g. by a -SH moiety as defined herein or may be provided by modifying the disulfide-crosslinkable cationic component accordingly to exhibit a -SH moiety. These -SH-moieties are typically provided by each of the disulfide-crosslinkable cationic component, e.g. via a cysteine or any further (modified) amino acid or compound of the component, which carries a -SH moiety. In the case that the disulfide-crosslinkable cationic component is a peptide or protein it is preferred that the -SH moiety is provided by at least one cysteine residue. Alternatively, the cationic or polycationic component used as a disulfide-crosslinkable cationic component may be modified accordingly with an -SH moiety, preferably via a chemical reaction with a compound carrying a -SH moiety, such that each of the components carries at least one such -SH moiety. Such a compound carrying a -SH moiety may be e.g. an (additional) cysteine or any further (modified) amino acid or compound, which carries a -SH moiety. Such a compound may also be any non-amino acid compound or moiety, which contains or allows to introduce a -SH moiety into the disulfide-crosslinkable cationic component as defined herein. Such non-amino acid compounds may be attached to the component of the polymeric carrier according to the present invention via chemical reactions or binding of compounds, e.g. by binding of a 3-thio propionic acid or 2-iminothiolane (Traut's reagent), by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. In some cases the -SH moiety may be masked by protecting groups during chemical attachment to the component. Such protecting groups are known in the art and may be removed after chemical coupling. In each case, the -SH moiety, e.g. of a cysteine or of any further (modified) amino acid or compound, may be present at (one or both of) the terminal ends or internally at any position of the disulfide-crosslinkable cationic component. As defined herein, each of the disulfide-crosslinkable cationic components typically exhibits at least one -SH-moiety, but may also contain two, three, four, five, or even more -SH-moieties. Additionally to binding of other disulfide-crosslinkable cationic components a -SH moiety may be used to attach further components to the disulfide-crosslinkable cationic component as defined herein, particularly an amino acid component, e.g. antigen epitopes, antigens, antibodies, cell penetrating peptides (e.g. TAT), ligands, etc.

In the inventive vaccine composition, at least one disulfide-crosslinkable cationic component may be selected from cationic or polycationic peptides or proteins as defined herein. Such cationic or polycationic peptides or proteins typically exhibit a length of about 3 to 200, preferably 3 to 100 amino acids, more preferably a length of about 3 to 50 amino acids, even more preferably a length of about 3 to 25 amino acids, e.g. a length of about 3 to 10, 5 to 15, 10 to 20 or 15 to 25 amino acids. Alternatively or additionally, such cationic or polycationic peptides or proteins may exhibit a molecular weight of about 0.3 kDa to about 20 kDa, preferably 0.3 kDa to about 10 kDa, including a molecular weight of about 0.4 kDa to about 10 kDa, 0.5 kDa to about 10 kDa, preferably of about 0.5 kDa to about 7.5 kDa, 0.5 kDa to about 5 kDa, 0.5 kDa to about 4 kDa, 0.5 kDa to about 3 kDa, or even 0.67 kDa to about 2.7 kDa.

In the specific case that the disulfide-crosslinkable cationic component comprises a cationic or polycationic peptide or protein, the cationic properties of a disulfide-crosslinkable cationic component (or of the disulfide-crosslinked cationic compound formed by the disulfide-crosslinkable cationic component), may be determined upon its content of cationic amino acids. Preferably, the content of cationic amino acids in such a cationic or polycationic peptide or protein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even more preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in such a disulfide-crosslinked cationic compound is 100%.

Preferably, such disulfide-crosslinkable cationic components, which comprise or are additionally modified to comprise at least one -SH moiety, are selected from, without being restricted thereto, cationic peptides or proteins such as protamine, nucleoline, spermine or spermidine, oligo- or poly-L-lysine (PLL), basic polypeptides, oligo or poly-arginine, cell penetrating peptides (CPPs), chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia*)*,* pAntp, plsl, etc., antimicrobial-derived CPPs e.g. Buforin-2, Bac715-24, SynB, SynB(1), pVEC, hCT-derived peptides, SAP, MAP, KALA, PpTG20, Loligomere, FGF, Lactoferrin, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc.

Alternatively or additionally, such disulfide-crosslinkable cationic components, which comprise or are additionally modified to comprise at least one -SH moiety, are selected from, without being restricted thereto, oligopeptides having the sum formula (V) as defined above.

According to a further particular preferred aspect, disulfide-crosslinkable cationic components, preferably oligopeptides having the sum formula (V) as shown above, and which comprise or are additionally modified to comprise at least one -SH moiety, may be, without being restricted thereto, selected from an oligopeptide of subformula (Va):

{(Arg)ₗ;(Lys)ₘ;(His)ₙ;(Orn)ₒ;(Xaa')ₓ(Cys)_{y}} formula (Va)

wherein (Arg)ₗ;(Lys)ₘ;(His)ₙ;(Orn)ₒ; and x is preferably are as defined above for formula (V), Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide.

This aspect may apply to situations, wherein the disulfide-crosslinkable cationic component, e.g. when defined according to empirical formula (Arg)ₗ;(Lys)ₘ;(His)ₙ;(Orn)ₒ;(Xaa)ₓ (formula (V)) as shown above, comprises or has been modified with at least one cysteine as -SH moiety in the above meaning such that the disulfide-crosslinkable cationic component as cationic or polycationic component carries at least one cysteine, which is capable to form a disulfide bond with other cationic or polycationic components.

According to another particular preferred aspect, the disulfide-crosslinkable cationic component may be, without being restricted thereto, selected from subformula (Vb):

Cys¹{(Arg)ₗ;(Lys)ₘ;(His)ₙ;(Orn)ₒ;(Xaa)ₓ}Cys² (formula (Vb))

wherein component {(Arg)ₗ;(Lys)ₘ;(His)ₙ;(Orn)ₒ;(Xaa)ₓ} (formula (V)) within formula (Vb) is as defined herein and forms a core of subformula (Vb), and wherein Cys¹ and Cys² are Cysteines proximal to, or terminal to (Arg)ₗ;(Lys)ₘ;(His)ₙ;(Orn)ₒ;(Xaa)ₓ. Exemplary examples may comprise any of the above sequences flanked by two Cys and following sequences:
Cys(Arg₇)Cys, Cys(Arg₈)Cys, Cys(Arg₉)Cys, Cys(Arg₁₀)Cys, Cys(Arg₁₁)Cys, Cys(Arg₁₂)Cys, Cys(Arg₁₃)Cys, Cys(Arg₁₄)Cys, Cys(Arg₁₅)Cys, Cys(Arg₁₆)Cys, Cys(Arg₁₇)Cys, Cys(Arg₁₈)Cys, Cys(Arg₁₉)Cys, Cys(Arg₂₀)Cys (SEQ ID NOs: 201-214):

Further examples may comprise any of the following sequences:
Cys(Lys₇)Cys, Cys(Lys₈)Cys, Cys(Lys₉)Cys, Cys(Lys₁₀)Cys, Cys(Lys₁₁)Cys, Cys(Lys₁₂)Cys, Cys(Lys₁₃)Cys, Cys(Lys₁₄)Cys, Cys(Lys₁₅)Cys, Cys(Lys₁₆)Cys, Cys(Lys₁₇)Cys, Cys(Lys₁₈)Cys, Cys(Lys₁₉)Cys, Cys(Lys₂₀)Cys (SEQ ID NOs: 215-228):
Cys(His₇)Cys, Cys(His₈)Cys, Cys(His₉)Cys, Cys(His₁₀)Cys, Cys(His₁₁)Cys, Cys(His₁₂)Cys, Cys(His₁₃)Cys, Cys(His₁₄)Cys, Cys(His₁₅)Cys, Cys(His₁₆)Cys, Cys(His₁₇)Cys, Cys(His₁₈)Cys, Cys(His₁₉)Cys, Cys(His₂₀)Cys (SEQ ID NOs: 229-242):
Cys(Orn7)Cys, Cys(Orn₈)Cys, Cys(Orn₉)Cys, Cys(Orn₁₀)Cys, Cys(Orn₁₁)Cys, Cys(Orn₁₂)Cys, Cys(Orn₁₃)Cys, Cys(Orn₁₄)Cys, Cys(Orn₁₅)Cys, Cys(Orn₁₆)Cys, Cys(Orn₁₇)Cys, Cys(Orn₁₈)Cys, Cys(Orn₁₉)Cys, Cys(Orn₂₀)Cys (SEQ ID NOs: 243-256):
or following exemplary sequences:
CysArg₉Cys, CysArg₉His₃Cys, CysHis₃Arg₉His₃Cys, CysTyrSerSerArg₉SerSerTyrCys, CysHis₃Arg₉SerSerTyrCys, Cys(ArgLysHis)₄Cys, CysTyr(ArgLysHis)₂ArgCys, CysHis₃Arg₉His₃Cys, CysHis₆Arg₉His₆Cys, CysHis3Arg4His3Cys, CysHis6Arg4His6Cys, CysArg₁₂Cys; (SEQ ID NOs: 257-268).

This aspect may apply to situations, wherein the disulfide-crosslinkable cationic components, e.g. when defined according to empirical formula (Arg)ₗ;(Lys)ₘ;(His)ₙ;(Orn)ₒ;(Xaa)ₓ (formula (V)) as shown above, has been modified with at least two cysteines as -SH moieties in the above meaning such that the disulfide-crosslinkable cationic component carries at least two (terminal) cysteines, which are capable to form a disulfide bond with other components.

Alternatively, at least one cationic (or polycationic) component, used as a disulfide-crosslinkable cationic (or polycationic) component to form a disulfide-crosslinked cationic compound to complex the immunostimulatory nucleic acid sequence of the adjuvant component, may comprise e.g. any (non-peptidic) cationic or polycationic polymer suitable in this context, provided that this (non-peptidic) cationic or polycationic polymer exhibits or is modified to exhibit at least one -SH-moiety, which provides for a disulfide bond linking with a further cationic or polycationic polymer or with another component as defined herein, e.g. a further disulfide-crosslinkable cationic (or polycationic) component. Likewise as defined herein, the cationic (or polycationic) component, used as a disulfide-crosslinkable cationic (or polycationic) component to form a disulfide-crosslinked cationic compound to complex the immunostimulatory nucleic acid sequence of the adjuvant component, may comprise the same or different cationic or polycationic polymers, exhibiting or being modified to exhibit at least one -SH-moiety.

In the specific case that the cationic (or polycationic) component, used as a disulfide-crosslinkable cationic (or polycationic) component to form a disulfide-crosslinked cationic compound to complex the immunostimulatory nucleic acid sequence of the adjuvant component as described herein, comprises a (non-peptidic) cationic or polycationic polymer, the cationic properties of this (non-peptidic) cationic or polycationic component (and eventually of the entire disulfide-crosslinked cationic compound, if entirely formed by such (non-peptidic) cationic or polycationic components as defined herein) may be determined upon its content of cationic charges when compared to the overall charges of the cationic (or polycationic) component, preferably the disulfide-crosslinkable cationic (or polycationic) component, or the disulfide-crosslinked cationic compound. Preferably, the content of cationic charges in the cationic (or polycationic) component (or in the disulfide-crosslinked cationic compound) at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 30% to 100%, even preferably in the range of about 50% to 100%, e.g. 50, 60, 70, 80%, 90% or 100%, or in a range formed by any two of the afore mentioned values, provided, that the content of all charges, e.g. positive and negative charges at a (physiological) pH as defined herein, in the entire disulfide-crosslinkable cationic (or polycationic) component or in the entire disulfide-crosslinked cationic compound is 100%.

Preferably, the (non-peptidic) cationic or polycationic component, used as a disulfide-crosslinkable cationic (or polycationic) component to form a disulfide-crosslinked cationic compound to complex the immunostimulatory nucleic acid sequence of the adjuvant component, may be formed by a cationic or polycationic polymer, typically exhibiting a molecular weight of about 0.1 kDa to about 20 kDa, usually a molecular weight of about 0.5 kDa to about 11,5 kDa, preferably of about 1 kDa to about 10 kDa, more preferably of about 0.1 kDa to about 8 kDa, even more preferably of about 0.1 kDa to about 6 kDa, or a molecular weight of about 0.1 kDa to about 5 kDa, even more preferably of about 0.5 kDa to about 5 kDa, likewise preferably a molecular weight of about 0.3 kDa to about 20 kDa, preferably 0.3 kDa to about 10 kDa, including a molecular weight of about 0.4 kDa to about 10 kDa, 0.5 kDa to about 10 kDa, preferably of about 0.5 kDa to about 7.5 kDa, 0.5 kDa to about 5 kDa, 0.5 kDa to about 4 kDa, 0.5 kDa to about 3 kDa, or even 0.67 kDa to about 2.7 kDa.

Additionally, the (non-peptidic) cationic or polycationic polymer typically exhibits at least one (preferably two) -SH-moiety, which is capable to form a disulfide linkage upon condensation with (-SH moieties of) other cationic or polycationic components as defined herein.

In the above context, the (non-peptidic) cationic or polycationic component, which may be used to form a disulfide-crosslinked cationic compound to complex the immunostimulatory nucleic acid sequence of the adjuvant component, may be selected from acrylates, modified acrylates, such as pDMAEMA (poly(dimethylaminoethyl methylacrylate)), chitosanes, aziridines or 2-ethyl-2-oxazoline (forming oligo ethylenimines or modifed oligoethylenimines), polymers obtained by reaction of bisacrylates with amines forming oligo beta aminoesters or poly amido amines, or other polymers like polyesters, polycarbonates, etc. Each molecule of these (non-peptidic) cationic or polycationic polymers typically exhibits at least one -SH-moiety, wherein these at least one -SH-moiety may be introduced into the (non-peptidic) cationic or polycationic polymer by chemical modifications, e.g. using imonothiolan, 3-thio propionic acid or introduction of -SH-moieties containing amino acids, such as cysteine or any further (modified) amino acid. Such -SH-moieties are preferably as already defined above.

In the context of disulfide-crosslinked cationic compounds, used as a complexing agent to complex the immunostimulatory nucleic acid sequence of the adjuvant component, the single disulfide-crosslinkable cationic components thereof forming the disulfide-crosslinked cationic compound may be the same or different from each other. It is also particularly preferred that the disulfide-crosslinked cationic compound comprises mixtures of disulfide-crosslinkable cationic components, i.e. peptides, proteins and optionally polymers or further components as defined herein, which are crosslinked by disulfide bonds as described herein, preferably mixtures of any of the disulfide-crosslinkable cationic components as defined herein.

In this context, the disulfide-crosslinked cationic compounds, used as a complexing agent to complex the immunostimulatory nucleic acid sequence of the adjuvant component, advantageously allow to combine desired properties of different (short) cationic or polycationic peptides, proteins and optionally polymers or other components.

In particular, the disulfide-crosslinked cationic compound, used as a complexing agent to complex the immunostimulatory nucleic acid sequence of the adjuvant component, allow considerably to vary its peptide content and thus to modulate its biophysical/biochemical properties, particularly its cationic properties, quite easily and fast, e.g. by incorporating as (cationic) components the same or different (cationic) peptide(s) and optionally polymer(s) and optionally adding other components. Even though consisting of quite small non-toxic repeat units the disulfide-crosslinked cationic compound, used as a complexing agent to complex the immunostimulatory nucleic acid sequence of the adjuvant component form a long cationic binding sequence providing a strong condensation of the nucleic acid sequence cargo and complex stability. Under the reducing conditions of the cytosol (e.g. cytosolic GSH), the complex formed with the disulfide-crosslinked cationic compound and the immunostimulatory nucleic acid sequence (adjuvant component) is rapidly degraded into its (cationic) components, which are further degraded (e.g. oligopeptides). This supports deliberation of the nucleic acid sequence cargo in the cytosol. Due to degradation into small oligopeptides or polymers in the cytosol, no toxicity is observed as known for high-molecular oligopeptides or polymers, e.g. from high-molecular polyarginine.

Accordingly, the disulfide-crosslinked cationic compound, used as a complexing agent to complex the immunostimulatory nucleic acid sequence of the adjuvant component, may comprise different (short) disulfide-crosslinkable cationic or polycationic components, i.e. peptides or proteins and optionally polymers selected from cationic or polycationic peptides or proteins and optionally (non-peptidic) polymers as defined above, optionally together with further components as defined herein.

Additionally, the disulfide-crosslinked cationic compound, used as a complexing agent to complex the immunostimulatory nucleic acid sequence of the adjuvant component, more preferably at least one of the different (short) disulfide-crosslinkable cationic or polycationic peptides forming basis for the polymeric carrier via disulfide-crosslinking, may be, preferably prior to the disulfide-crosslinking, be modified with at least one further component. Alternatively, the disulfide-crosslinked cationic compound as such may be modified with at least one further component. It may also optionally comprise at least one further component, which typically forms the disulfide-crosslinked cationic compound together with the other the (short) cationic or polycationic peptides as defined above via disulfide crosslinking.

To allow modification of the disulfide-crosslinked cationic compound, used as a complexing agent to complex the immunostimulatory nucleic acid sequence of the adjuvant component, as defined above, each of the disulfide-crosslinkable cationic or polycationic components of the disulfide-crosslinked cationic compound may (preferably already prior to disulfide-crosslinking) also contain at least one further functional moiety, which allows attaching such further components as defined herein. Such functional moieties may be selected from, e.g. functionalities as defined herein, obtained e.g. by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components.

According to another aspect, the disulfide-crosslinked cationic compound or single disulfide-crosslinkable cationic or polycationic components thereof, e.g. of the above mentioned (disulfide-crosslinkable) cationic or polycationic peptides, proteins or polymers or further components, may be further modified with a ligand, preferably a carbohydrate, more preferably a sugar, even more preferably mannose. Preferably this ligand is bound to the disulfide-crosslinked cationic compound or to a (disulfide-crosslinkable) cationic or polycationic component of the disulfide-crosslinked cationic compound via a (reversible) disulfide bond or via Michael addition. In the case that the ligand is bound by a disulfide bond the ligand additionally comprises at least one -SH-moiety. These ligands may be used to direct the adjuvant component or the inventive vaccine composition to specific target cells (e.g. hepatocytes or antigen-presenting cells). In this context mannose is particular preferred as ligand in the case that dendritic cells are the target especially for vaccination or adjuvant purposes.

According to one specific aspect, the entire adjuvant component may be formed by a polymerization condensation (of at least one) of the above mentioned (disulfide-crosslinkable) cationic or polycationic components, i.e. peptides or proteins and optionally polymers or further components as defined above, via their -SH-moieties in a first step to form the disulfide-crosslinked cationic compound and complexing the immunostimulatory nucleic acid sequence to such a disulfide-crosslinked cationic compound in a second step. The disulfide-crosslinked cationic compound may thus contain a number of at least one or even more of the same or different of the above defined cationic or polycationic peptides or proteins and optionally polymers or further components, the number preferably determined by the above range.

According to one alternative specific aspect, the entire adjuvant component may be formed by carrying out the polymerization condensation of at least one of the above mentioned (disulfide-crosslinkable) cationic components, preferably cationic or polycationic peptides, proteins or polymers or further components, via their -SH-moieties simultaneously to complexing the immunostimulatory nucleic acid sequence to the (in situ prepared) disulfide-crosslinked cationic compound. Likewise, the disulfide-crosslinked cationic compound may thus also here contain a number of at least one or even more of the same or different of the above defined (disulfide-crosslinkable) cationic or polycationic components, preferably peptides, proteins or polymers or further components, the number preferably determined by the above range.

The inventive vaccine composition furthermore comprises a nucleic acid sequence encoding an antigen. According to the present invention, the term "antigen" refers to a substance which is recognized by the immune system and is capable of triggering an antigen-specific immune response, e.g. by formation of antibodies or antigen-specific T-cells as part of an adaptive immune response. In this context, the first step of an adaptive immune response is the activation of naive antigen-specific T cells by antigen-presenting cells. This occurs in the lymphoid tissues and organs through which naïve T cells are constantly passing. The three cell types that can serve as antigen-presenting cells are dendritic cells, macrophages, and B cells. Each of these cells has a distinct function in eliciting immune responses. Tissue dendritic cells take up antigens by phagocytosis and macropinocytosis and are stimulated by infection to migrate to the local lymphoid tissue, where they differentiate into mature dendritic cells. Macrophages ingest particulate antigens such as bacteria and are induced by infectious agents to express MHC class II molecules. The unique ability of B cells to bind and internalize soluble protein antigens via their receptors may be important to induce T cells. By presenting the antigen on MHC molecules leads to activation of T cells which induces their proliferation and differentiation into armed effector T cells. The most important function of effector T cells is the killing of infected cells by CD8⁺ cytotoxic T cells and the activation of macrophages by TH1 cells which together make up cell-mediated immunity, and the activation of B cells by both TH2 and TH1 cells to produce different classes of antibody, thus driving the humoral immune response. T cells recognize an antigen by their T cell receptors which does not recognize and bind antigen directly, but instead recognize short peptide fragments e.g. of pathogens' protein antigens, which are bound to MHC molecules on the surfaces of other cells.

T cells fall into two major classes that have different effector functions. The two classes are distinguished by the expression of the cell-surface proteins CD4 and CD8. These two types of T cells differ in the class of MHC molecule that they recognize. There are two classes of MHC molecules - MHC class I and MHC class II molecules - which differ in their structure and expression pattern on tissues of the body. CD4⁺ T cells bind to a MHC class II molecule and CD8⁺ T cells to a MHC class I molecule. MHC class I and MHC class II molecules have distinct distributions among cells that reflect the different effector functions of the T cells that recognize them. MHC class I molecules present peptides from pathogens, commonly viruses to CD8⁺ T cells, which differentiate into cytotoxic T cells that are specialized to kill any cell that they specifically recognize. Almost all cells express MHC class I molecules, although the level of constitutive expression varies from one cell type to the next. But not only pathogenic peptides from viruses are presented by MHC class I molecules, also self-antigens like tumour antigens are presented by them. MHC class I molecules bind peptides from proteins degraded in the cytosol and transported in the endoplasmic reticulum. Thereby MHC class I molecules on the surface of cells infected with viruses or other cytosolic pathogens display peptides from these pathogens. The CD8⁺ T cells that recognize MHC class I:peptide complexes are specialized to kill any cells displaying foreign peptides and so rid the body of cells infected with viruses and other cytosolic pathogens. The main function of CD4⁺ T cells (CD4⁺ helper T cells) that recognize MHC class II molecules is to activate other effector cells of the immune system. Thus MHC class II molecules are normally found on B lymphocytes, dendritic cells, and macrophages, cells that participate in immune responses, but not on other tissue cells. Macrophages, for example, are activated to kill the intravesicular pathogens they harbour, and B cells to secrete immunoglobulins against foreign molecules. MHC class II molecules are prevented from binding to peptides in the endoplasmic reticulum and thus MHC class II molecules bind peptides from proteins which are degraded in endosomes. They can capture peptides from pathogens that have entered the vesicular system of macrophages, or from antigens internalized by immature dendritic cells or the immunoglobulin receptors of B cells. Pathogens that accumulate in large numbers inside macrophage and dendritic cell vesicles tend to stimulate the differentiation of TH1 cells, whereas extracellular antigens tend to stimulate the production of TH2 cells. TH1 cells activate the microbicidal properties of macrophages and induce B cells to make IgG antibodies that are very effective of opsonising extracellular pathogens for ingestion by phagocytic cells, whereas TH2 cells initiate the humoral response by activating naive B cells to secrete IgM, and induce the production of weakly opsonising antibodes such as IgG1 and IgG3 (mouse) and IgG2 and IgG4 (human) as well as IgA and IgE (mouse and human).

In the context of the present invention, "antigens" generally comprise any antigen, antigenic epitope or antigenic peptide, falling under the above definition, more preferably protein and peptide antigens or their encoding nucleic acid sequences, e.g. tumour antigens, allergenic antigens, auto-immune self-antigens, pathogenic antigens, etc. In particular antigens , either as a protein or peptide or encoded by a nucleic acid sequence may be selected from antigens generated outside the cell, more typically from antigens not derived from the host organism (e.g. a human) itself (i.e. non-self antigens) but rather derived from host cells outside the host organism, e.g. viral antigens, bacterial antigens, fungal antigens, protozoological antigens, animal antigens, allergenic antigens, etc. Allergenic antigens (allergy antigens) are typically antigens, which cause an allergy in a human and may be derived from either a human or other sources. Additionally, antigens may be furthermore antigens generated inside the cell, the tissue or the body. Such antigens include antigens derived from the host organism (e.g. a human) itself, e.g. tumour antigens, self-antigens or auto-antigens, such as auto-immune self-antigens, etc., but also (non-self) antigens as defined herein, which have been originally been derived from host cells outside the host organism, but which are fragmented or degraded inside the body, tissue or cell, e.g. by (protease) degradation, metabolism, etc.

One class of antigens as contained in the inventive vaccine composition may comprise tumour antigens. ,,"Tumour antigens",, are preferably located on the surface of the (tumour) cell. Tumour antigens may also be selected from proteins, which are overexpressed in tumour cells compared to a normal cell. Furthermore, tumour antigens also include antigens expressed in cells which are (were) not themselves (or originally not themselves) degenerated but are (were) associated with the supposed tumour. Antigens which are connected with tumour-supplying vessels or (re)formation thereof, in particular those antigens which are associated with neovascularization, e.g. growth factors, such as VEGF, bFGF etc., are also included herein. Antigens connected with a tumour furthermore include antigens from cells or tissues, typically embedding the tumour. Further, some substances (usually proteins or peptides) are expressed in patients suffering (knowingly or not-knowingly) from a cancer disease and they occur in increased concentrations in the body fluids of said patients. These substances are also referred to as "tumour antigens", however, they may not be antigens in the stringent meaning of an immune response inducing substance. The class of tumour antigens can be divided further into tumour-specific antigens (TSAs) and tumour-associated-antigens (TAAs). TSAs can only be presented by tumour cells and never by normal "healthy" cells. They typically result from a tumour specific mutation. TAAs, which are more common, are usually presented by both tumour and healthy cells. These antigens are recognized and the antigen-presenting cell can be destroyed by cytotoxic T cells. Additionally, tumour antigens can also occur on the surface of the tumour in the form of, e.g., a mutated receptor. In this case, they can be recognized by antibodies. Particular preferred tumour antigens are selected from the group consisting of 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGF, VEGFR-2/FLK-1, and WT1. Such tumour antigens preferably may be selected from the group consisting of MAGE-A1 (e.g. MAGE-A1 according to accession number M77481), MAGE-A2, MAGE-A3, MAGE-A6 (e.g. MAGE-A6 according to accession number NM_005363), MAGE-C1, MAGE-C2, melan-A (e.g. melan-A according to accession number NM_005511), GP100 (e.g. GP100 according to accession number M77348), tyrosinase (e.g. tyrosinase according to accession number NM_000372), surviving (e.g. survivin according to accession number AF077350), CEA (e.g. CEA according to accession number NM_004363), Her-2/neu (e.g. Her-2/neu according to accession number M11730), WT1 (e.g. WT1 according to accession number NM_000378), PRAME (e.g. PRAME according to accession number NM_006115), EGFRI (epidermal growth factor receptor 1) (e.g. EGFRI (epidermal growth factor receptor 1) according to accession number AF288738), MUC1, mucin-1 (e.g. mucin-1 according to accession number NM_002456), SEC61G (e.g. SEC61G according to accession number NM_014302), hTERT (e.g. hTERT accession number NM_198253), 5T4 (e.g. 5T4 according to accession number NM_006670), NY-Eso-1 (e.g. NY-Eso1 according to accession number NM_001327), TRP-2 (e.g. TRP-2 according to accession number NM_001922), STEAP, PCA, PSA, PSMA, etc.

According to another alternative, one further class of antigens as contained in the inventive vaccine composition may comprise allergenic antigens. Such allergenic antigens may be selected from antigens derived from different sources, e.g. from animals, plants, fungi, bacteria, etc. Allergens in this context include e.g. grasses, pollens, molds, drugs, or numerous environmental triggers, etc. Allergenic antigens typically belong to different classes of compounds, such as nucleic acid sequences and their fragments, or proteins or peptides and their fragments, carbohydrates, polysaccharides, sugars, lipids, phospholipids, etc.

Antigens may generally occur as a protein or peptide antigen, or a fragment, variant or epitope thereof and/or may occur as a nucleic acid sequence encoding said protein or peptide antigen or a fragment, variant or epitope thereof.

According to one alternative, antigens may occur as a protein or peptide antigen, or as a fragment, variant or epitope thereof. In this context, fragments and/or variants may have a sequence identity to one of the aforementioned protein or peptide antigens of at least 5%, 10%, 20%, 30%, 40%, 50%, 60%, 70%, 80% or 85%, preferably at least 90%, more preferably at least 95% and most preferably at least 99% over the whole length of these protein or peptide antigens. In the context of the present invention a fragment of such a protein or peptide antigen is to be understood as a truncated protein or peptide thereof, i.e. an amino acid sequence, which is N-terminally, C-terminally and/or intrasequentially truncated compared to the amino acid sequence of the original (native) protein or peptide antigen. Especially, fragments including an antigenic epitope are preferred. In this context, fragments and epitopes are preferably as specifically defined herein for antigens.

"Fragments" of protein or peptide antigens in the context of the present invention may comprise a sequence of a protein or peptide antigen as defined herein, which is, with regard to its amino acid sequence (or its encoded nucleic acid sequence), N-terminally, C-terminally and/or intrasequentially truncated compared to the amino acid sequence of the original (native) protein or peptide antigen (or its encoded nucleic acid sequence). Such truncation may thus occur either on the amino acid level or correspondingly on the nucleic acid sequence level. A sequence identity with respect to such a fragment as defined herein may therefore preferably refer to the entire protein or peptide antigen as defined herein or to the entire (coding) nucleic acid sequence of such a protein or peptide antigen. The same applies accordingly to nucleic acid sequences.

Such fragments of protein or peptide antigens in the context of the present invention may furthermore comprise a sequence of a protein or peptide antigen as defined herein, which has a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form.

The fragments of protein or peptide antigens as defined herein may also comprise epitopes of those protein or peptide antigens. Epitopes (also called "antigen determinants") in the context of the present invention are typically fragments located on the outer surface of (native) proteins or peptides as defined herein, preferably having 5 to 15 amino acids, more preferably having 5 to 12 amino acids, even more preferably having 6 to 9 amino acids, which may be recognized by antibodies or B-cell receptors, i.e. in their native form. Such epitopes of protein or peptide antigens may furthermore be selected from any of the herein mentioned variants of such protein or peptide antigens. In this context antigenic determinants can be conformational or discontinous epitopes which are composed of segments of the protein or peptide antigens as defined herein that are discontinuous in the amino acid sequence of the protein or peptide antigens as defined herein but are brought together in the three-dimensional structure or continuous or linear epitopes which are composed of a single polypeptide chain.

"Variants" of protein or peptide antigens as defined herein may be encoded by a nucleic acid sequence as defined herein, wherein nucleotides of the nucleic acid sequence, encoding the protein or peptide antigen as defined herein, are exchanged. Thereby, a protein or peptide antigen may be generated, having an amino acid sequence which differs from the original sequence in one or more mutation(s), such as one or more substituted, inserted and/or deleted amino acid(s). Preferably, these fragments and/or variants have the same biological function or specific activity compared to the full-length native protein or peptide antigen, e.g. its specific antigenic property.

Protein or peptide antigens, or a fragment, variant or epitope thereof as defined herein, which have (a) conservative substitution(s) compared to the physiological, i.e. native and non-modified, sequence in particular fall under the term "variants". Substitutions in which encoded amino acids which originate from the same class are exchanged for one another are called conservative substitutions. In particular, these are encoded amino acids, encoded aliphatic side chains, positively or negatively charged side chains, aromatic groups in the side chains or encoded amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification of the three-dimensional structure or do not affect the binding region or the catalytic domain. Modifications of the three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed), Elsevier, Amsterdam).

According to the invention, the antigen of the inventive vaccine composition occurs as a nucleic acid sequence encoding said protein or peptide antigen or a fragment, variant or epitope thereof as defined above. In the context of the present disclosure, such a nucleic acid sequence may generally be any suitable nucleic acid sequence, selected e.g. from any (single-stranded or double-stranded) DNA, preferably, without being limited thereto, e.g. genomic DNA, single-stranded DNA molecules, double-stranded DNA molecules, coding DNA, DNA primers, DNA probes, a (short) DNA oligonucleotide ((short) oligodesoxyribonucleotides), or may be selected e.g. from any PNA (peptide nucleic acid sequence) or may be selected e.g. from any (single-stranded or double-stranded) RNA, preferably, without being limited thereto, a (short) RNA oligonucleotide ((short) oligoribonucleotide), a coding RNA, a messenger RNA (mRNA), etc. In the inventive vaccine composition, the nucleic acid sequence encoding said protein or peptide antigen or a fragment, variant or epitope thereof is an RNA, preferably a (linear) single-stranded RNA, more preferably an mRNA. In the context of the present invention, an mRNA is typically an RNA, which is composed of several structural elements, e.g. an optional 5'-CAP structure, an optional 5'-UTR region, an upstream positioned ribosomal binding site followed by a coding region, an optional 3'-UTR region, which may be followed by a poly-A tail (and/or a poly-C-tail). An mRNA may occur as a mono-, di-, or even multicistronic RNA, i.e. a RNA which carries the coding sequences of one, two or more proteins or peptides, e.g. one, two or more protein or peptide antigens or fragments, variants or epitopes thereof. Such coding sequences in di-, or even multicistronic mRNA may be separated by at least one IRES (internal ribosomal entry site) sequence, e.g. as defined herein. Examples of IRES sequences which may be used include those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

Furthermore, the nucleic acid sequence encoding said protein or peptide antigen or a fragment, variant or epitope thereof as defined above, may be a single- or a double-stranded nucleic acid sequence (molecule) (which may also be regarded as a nucleic acid sequence (molecule) due to non-covalent association of two single-stranded nucleic acid sequence(s) (molecules)) or a partially double-stranded or partially single stranded nucleic acid sequence, which are at least partially self complementary (both of these partially double-stranded or partially single stranded nucleic acid sequences are typically formed by a longer and a shorter single-stranded nucleic acid sequence or by two single stranded nucleic acid sequences, which are about equal in length, wherein one single-stranded nucleic acid sequence is in part complementary to the other single-stranded nucleic acid sequence and both thus form a double-stranded nucleic acid sequence in this region, i.e. a partially double-stranded or partially single stranded nucleic acid sequence (molecule). Preferably, the nucleic acid sequence (molecule) may be a single-stranded nucleic acid sequence. Furthermore, the nucleic acid sequence (molecule) may be a circular or linear nucleic acid sequence, preferably a linear nucleic acid sequence.

In order to determine the percentage to which two sequences are identical, particularly the sequences of protein or peptide antigens, or a fragment, variant or epitope thereof as defined herein or the sequences of nucleic acid sequences encoding said protein or peptide antigens or a fragment, variant or epitope thereof, as defined herein, or sequences of any further protein or nucleic acid sequence as defined herein, the sequences can be aligned in order to be subsequently compared to one another. Therefore, as an example, e.g. gaps can be inserted into the sequence of the first sequence (e.g. (m)RNA or mRNA) and the component at the corresponding position of the second sequence (e.g. (m)RNA or mRNA) can be compared. If a position in the first sequence (e.g. (m)RNA or mRNA) sequence is occupied by the same component as is the case at a position in the second sequence (e.g. (m)RNA or mRNA), the two sequences are identical at this position. The percentage to which two (m)RNA (or mRNA) sequences are identical is a function of the number of identical positions divided by the total number of positions. The same, of course also applies accordingly to DNA sequences or the encoded amino acid sequences. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic acid sequences Res, 25:3389-3402. Such an algorithm is integrated e.g. in the BLAST or NBLAST program.

In a further preferred aspect, a nucleic acid sequence as herein defined may also occur in the form of a modified nucleic acid sequence.

According to a first specific aspect, a nucleic acid sequence as herein defined may be provided as a "stabilized nucleic acid sequence", preferably as a stabilized RNA, more preferably as an RNA that is essentially resistant to *in vivo* degradation (e.g. by an exo- or endonuclease).

In this context, a nucleic acid sequence as herein defined may contain backbone modifications, sugar modifications or base modifications. A backbone modification in connection with the present invention is a modification in which phosphates of the backbone of the nucleotides contained in the nucleic acid sequence are chemically modified. A sugar modification in connection with the present invention is a chemical modification of the sugar of the nucleotides of a nucleic acid sequence as defined herein. Furthermore, a base modification in connection with the present invention is a chemical modification of the base moiety of the nucleotides of a nucleic acid sequence as defined herein.

According to a further aspect, a nucleic acid sequence as defined herein can contain a lipid modification. Such a lipid-modified nucleic acid sequence typically further comprises at least one linker covalently linked with that nucleic acid sequence, and at least one lipid covalently linked with the respective linker. Alternatively, the lipid-modified nucleic acid sequence comprises at least one nucleic acid sequence as defined herein and at least one (bifunctional) lipid covalently linked (without a linker) with that nucleic acid sequence. According to a third alternative, the lipid-modified nucleic acid sequence comprises a nucleic acid sequence as defined herein, at least one linker covalently linked with that nucleic acid sequence, and at least one lipid covalently linked with the respective linker, and also at least one (bifunctional) lipid covalently linked (without a linker) with that nucleic acid sequence.

The nucleic acid sequence as defined herein may likewise be stabilized in order to prevent degradation of the nucleic acid sequence by various approaches, particularly, when RNA or mRNA is used as a nucleic acid sequence for the inventive purpose. It is known in the art that instability and (fast) degradation of RNA in general may represent a serious problem in the application of RNA based composition s. This instability of RNA is typically due to RNA-degrading enzymes, "RNAases" (ribonucleases), wherein contamination with such ribonucleases may sometimes completely degrade RNA in solution. Accordingly, the natural degradation of RNA in the cytoplasm of cells is very finely regulated and RNase contaminations may be generally removed by special treatment prior to use of said composition s, in particular with diethyl pyrocarbonate (DEPC). A number of mechanisms of natural degradation are known in this connection in the prior art, which may be utilized as well. E.g., the terminal structure is typically of critical importance particularly for an mRNA. As an example, at the 5' end of naturally occurring mRNAs there is usually a so-called "cap structure" (a modified guanosine nucleotide), and at the 3' end is typically a sequence of up to 200 adenosine nucleotides (the so-called poly-A tail).

According to another aspect, a nucleic acid sequence as defined herein may be modified, and thus stabilized, especially if the nucleic acid sequence is in the form of a coding nucleic acid sequence e.g. an mRNA, by modifying the G/C content of the nucleic acid sequence, particularly an mRNA, preferably of the coding region thereof.

In a particularly preferred aspect of the present invention, the G/C content of the coding region of a nucleic acid sequence as defined herein, especially if the nucleic acid sequence is in the form of an mRNA, is modified, particularly increased, compared to the G/C content of the coding region of its particular wild type coding sequence, i.e. the unmodified mRNA. The encoded amino acid sequence of the nucleic acid sequence is preferably not modified compared to the coded amino acid sequence of the particular wild type mRNA.

The modification of the G/C-content of a nucleic acid sequence as defined herein, especially if the nucleic acid sequence is in the form of an mRNA or codes for an mRNA, is based on the fact that the sequence of any mRNA region to be translated is important for efficient translation of that mRNA. Thus, the composition and the sequence of various nucleotides are important. In particular, sequences having an increased G (guanosine)/C (cytosine) content are more stable than sequences having an increased A (adenosine)/U (uracil) content. According to the invention, the codons of the coding sequence or mRNA are therefore varied compared to its wild type coding sequence or mRNA, while retaining the translated amino acid sequence, such that they include an increased amount of G/C nucleotides. In respect to the fact that several codons code for one and the same amino acid (so-called degeneration of the genetic code), the most favourable codons for the stability can be determined (so-called alternative codon usage).

Preferably, the G/C content of the coding region of a nucleic acid sequence as defined herein, especially if the nucleic acid sequence is in the form of an mRNA or codes for an mRNA, is increased by at least 7%, more preferably by at least 15%, particularly preferably by at least 20%, compared to the G/C content of the coded region of the wild type mRNA. According to a specific aspect at least 5%, 10%, 20%, 30%, 40%, 50%, 60%, more preferably at least 70 %, even more preferably at least 80% and most preferably at least 90%, 95% or even 100% of the substitutable codons in the region coding for a protein or peptide as defined herein or its fragment or variant thereof or the whole sequence of the wild type mRNA sequence or coding sequence are substituted, thereby increasing the G/C content of said sequence.

In this context, it is particularly preferable to increase the G/C content of a nucleic acid sequence as defined herein, especially if the nucleic acid sequence is in the form of an mRNA or codes for an mRNA, to the maximum (i.e. 100% of the substitutable codons), in particular in the region coding for a protein, compared to the wild type sequence.

According to the invention, a further preferred modification of a nucleic acid sequence as defined herein, especially if the nucleic acid sequence is in the form of an mRNA or codes for an mRNA, is based on the finding that the translation efficiency is also determined by a different frequency in the occurrence of tRNAs in cells. Thus, if so-called "rare codons" are present in the nucleic acid sequence, especially if the nucleic acid sequence is in the form of an mRNA or codes for an mRNA, to an increased extent, the corresponding modified nucleic acid sequence is translated to a significantly poorer degree than in the case where codons coding for relatively "frequent" tRNAs are present.

Especially if the modified nucleic acid sequence of a nucleic acid sequence as defined is in the form of an mRNA or codes for an mRNA, the coding region of the modified nucleic acid sequence is preferably modified compared to the corresponding region of the wild type mRNA or coding sequence such that at least one codon of the wild type sequence which codes for a tRNA which is relatively rare in the cell is exchanged for a codon which codes for a tRNA which is relatively frequent in the cell and carries the same amino acid as the relatively rare tRNA. By this modification, the sequences of the nucleic acid sequence, especially if the nucleic acid sequence is in the form of an mRNA or codes for an mRNA, is modified such that codons for which frequently occurring tRNAs are available are inserted. In other words, according to the invention, by this modification all codons of the wild type sequence which code for a tRNA which is relatively rare in the cell can in each case be exchanged for a codon which codes for a tRNA which is relatively frequent in the cell and which, in each case, carries the same amino acid as the relatively rare tRNA.

Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons which use for the particular amino acid the tRNA which occurs the most frequently, e.g. the Gly codon, which uses the tRNA which occurs the most frequently in the (human) cell, are particularly preferred.

According to the invention, it is particularly preferable to link the sequential G/C content which is increased, in particular maximized, in the modified nucleic acid sequence as herein defined, especially if the nucleic acid sequence is in the form of an mRNA or codes for an mRNA, with the "frequent" codons without modifying the amino acid sequence of a protein encoded by the coding region of such a nucleic acid sequence. This preferred aspect allows provision of a particularly efficiently translated and stabilized (modified) nucleic acid sequence, especially if the nucleic acid sequence is in the form of an mRNA or codes for an mRNA.

According to a further preferred aspect of the invention, a nucleic acid sequence as defined herein, especially if the nucleic acid sequence is in the form of a coding nucleic acid sequence, preferably has at least one 5' and/or 3' stabilizing sequence. These stabilizing sequences in the 5' and/or 3' untranslated regions have the effect of increasing the half-life of the nucleic acid sequence in the cytosol. These stabilizing sequences can have 100% sequence identity to naturally occurring sequences which occur in viruses, bacteria and eukaryotes, but can also be partly or completely synthetic. The untranslated sequences (UTR) of the (alpha-)globin gene, e.g. from *Homo sapiens* or *Xenopus laevis* may be mentioned as an example of stabilizing sequences which can be used in the present invention for a stabilized nucleic acid sequence. Another example of a stabilizing sequence has the general formula (C/U)CCANₓCCC(U/A)PyₓUC(C/U)CC (SEQ ID NO: 543), which is contained in the 3'UTR of the very stable RNA which codes for (alpha-)globin, type(I)-collagen, 15-lipoxygenase or for tyrosine hydroxylase (cf. Holcik et al., Proc. Natl. Acad. Sci. USA 1997, 94: 2410 to 2414). Such stabilizing sequences can of course be used individually or in combination with one another and also in combination with other stabilizing sequences known to a person skilled in the art.

Nevertheless, substitutions, additions or eliminations of bases are preferably carried out with a nucleic acid sequence as defined herein, especially if the nucleic acid sequence is in the form of an mRNA, using a DNA matrix for preparation of the nucleic acid sequence by techniques of the well known site directed mutagenesis or with an oligonucleotide ligation strategy (see e.g. Maniatis et al., Molecular Cloning: A Laboratory Manual, Cold Spring Harbor Laboratory Press, 3rd ed., Cold Spring Harbor, NY, 2001). In such a process, for preparation of the nucleic acid sequence, especially if the nucleic acid sequence is in the form of an mRNA, a corresponding DNA molecule may be transcribed *in vitro.* This DNA matrix preferably comprises a suitable promoter, e.g. a T7 or SP6 promoter, for *in vitro* transcription, which is followed by the desired nucleotide sequence for the nucleic acid sequence, e.g. mRNA, to be prepared and a termination signal for *in vitro* transcription. The DNA molecule, which forms the matrix of the at least one RNA of interest, may be prepared by fermentative proliferation and subsequent isolation as part of a plasmid which can be replicated in bacteria. Plasmids which may be mentioned as suitable for the present invention are e.g. the plasmids pT7Ts (GenBank accession number U26404; Lai et al., Development 1995, 121: 2349 to 2360), pGEM® series, e.g. pGEM®-1 (GenBank accession number X65300; from Promega) and pSP64 (GenBank accession number X65327); cf. also Mezei and Storts, Purification of PCR Products, in: Griffin and Griffin (ed), PCR Technology: Current Innovation, CRC Press, Boca Raton, FL, 2001.

Nucleic acid sequences as defined and used herein may be prepared using any method known in the art, including synthetic methods such as e.g. solid phase synthesis, as well as *in vitro* methods, such as *in vitro* transcription reactions.

The inventive vaccine composition furthermore comprises a carrier molecule for combined packaging the adjuvant component and the RNA encoding the antigen as defined herein. In this context, a carrier molecule is a polymeric carrier molecule according to generic formula (VI):

L-P¹-S-[S-P²-S]ₙ-S-P³-L

wherein,
- P¹ and P³: are different or identical to each other and represent a linear or branched hydrophilic polymer chain, each P¹ and P³ exhibiting at least one -SH-moiety, capable to form a disulfide linkage upon condensation with component P², or alternatively with (AA), (AA)ₓ, or [(AA)ₓ]_{z} if such components are used as a linker between P¹ and P² or P³ and P²) and/or with further components (e.g. (AA), (AA)ₓ, [(AA)ₓ]_{z} or L), the linear or branched hydrophilic polymer chain selected independent from each other from polyethylene glycol (PEG), wherein the hydrophilic polymer chain exhibits a molecular weight of about 1 kDa to about 100 kDa, preferably of about 2 kDa to about 25 kDa; or more preferably of about 2 kDa to about 10 kDa, e.g. about 5 kDa to about 25 kDa or 5 kDa to about 10 kDa;
- P²: is a cationic or polycationic peptide or protein, e.g. as defined herein, and preferably having a length of about 3 to about 100 amino acids, more preferably having a length of about 3 to about 50 amino acids, even more preferably having a length of about 3 to about 25 amino acids, e.g. a length of about 3 to 10, 5 to 15, 10 to 20 or 15 to 25 amino acids, more preferably a length of about 5 to about 20 and even more preferably a length of about 10 to about 20;
is a cationic or polycationic polymer, e.g. as defined herein, typically having a molecular weight of about 0.5 kDa to about 30 kDa, including a molecular weight of about 1 kDa to about 20 kDa, even more preferably of about 1.5 kDa to about 10 kDa, or having a molecular weight of about 0.5 kDa to about 100 kDa, including a molecular weight of about 10 kDa to about 50 kDa, even more preferably of about 10 kDa to about 30 kDa; each P² exhibiting at least two -SH-moieties, capable to form a disulfide linkage upon condensation with further components P² or component(s) P¹ and/or P³ or alternatively with further components (e.g. (AA), (AA)ₓ, or [(AA)ₓ]_{z});
- -S-S-: is a (reversible) disulfide bond (the brackets are omitted for better readability), wherein S preferably represents sulphur or a -SH carrying moiety, which has formed a (reversible) disulfide bond. The (reversible) disulfide bond is preferably formed by condensation of -SH-moieties of either components P¹ and P², P² and P², or P² and P³, or optionally of further components as defined herein (e.g. L, (AA), (AA)ₓ, [(AA)ₓ]_{z}, etc); The -SH-moiety may be part of the structure of these components or added by a modification as defined below;
- L: is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, (e.g. TAT or KALA), a ligand of a receptor (e.g. cytokines, hormones, growth factors etc), small molecules (e.g. carbohydrates like mannose or galctose or synthetic ligands), small molecule agonists, inhibitors or antagonists of receptors (e.g. RGD peptidomimetic analogues) etc.;

- n: is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 4 to 9, 4 to 10, 3 to 20, 4 to 20, 5 to 20, or 10 to 20, or a range of about 3 to 15, 4 to 15, 5 to 15, or 10 to 15, or a range of about 6 to 11 or 7 to 10. Most preferably, n is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

The polymeric carrier molecule according to generic formula (VI) is preferably prepared by a new synthesis strategy and advantageously allows to define the length of the polymer chain and to combine desired properties of different (short) polymers in one polymer, e.g. to efficiently compact cargos for the purpose of efficient transfection of said (nucleic acid) cargo. Such a (nucleic acid) cargo may be e.g. the adjuvant component of the inventive vaccine composition comprising or consisting of at least one immunostimulatory nucleic acid, complexed with a complexing agent as defined herein; and/or the antigen of the inventive vaccine composition, which is a nucleic acid encoding an antigen as defined herein. The (nucleic acid) cargo, and preferably the entire inventive vaccine composition is preferably efficiently transfected into different cell lines *in vitro* but also *in vivo* due to the properties of the polymeric carrier molecule without loss of activity. The polymeric carrier molecule used for this purpose is furthermore not toxic to cells and provides for efficient release of its (nucleic acid) cargo, i.e. the adjuvant component of the inventive vaccine composition comprising or consisting of at least one immunostimulatory nucleic acid, complexed with a complexing agent as defined herein; and/or the antigen of the inventive vaccine composition. Finally, the polymeric carrier molecule and thus the inventive vaccine composition shows improved resistance to agglomeration due to the reversible addition of hydrophilic polymer chains (e.g. PEG-monomers) particularly to the terminal ends of the polymeric carrier molecule according to generic formula (VI). The polymeric carrier molecule additionally confers enhanced stability to the (nucleic acid) cargo with respect to serum containing media.

Even more advantageously, the polymeric carrier molecule according to generic formula (VI) allows to considerably vary its peptide or polymeric content and thus to modulate its biophysical/biochemical properties, particularly the cationic properties of component [S-P²-S]ₙ, quite easily and fast, e.g. by incorporating as components P² the same or different cationic peptide(s), protein(s) or polymer(s) and optionally adding other components, e.g. amino acid component(s) (AA) or (AA)ₓ, into the repetitive component [S-P²-S] to form a modified repetitive component such as {[S-P²-S]ₐ/[S-(AA)ₓ-S]_{b}} as a core motif of the polymeric carrier (wherein a + b = n, see below). Even though consisting of quite small nontoxic repeat units the polymeric carrier molecule allows effectively packaging the components of the inventive vaccine composition. Additionally, under the reducing conditions of the cytosol (e.g. cytosolic GSH), the polymeric carrier molecule of the entire complex of the inventive vaccine composition is rapidly degraded into its monomers, which are further degraded (e.g. oligopeptides) or secreted (e.g. PEG). This supports deliberation of the components of the inventive vaccine composition in the cytosol. Due to degradation into small oligopeptides in the cytosol, no toxicity is observed as known for high-molecular oligopeptides, e.g. from high-molecular oligoarginine. The PEG-"coating" also allows to somehow "coat" the polymeric carrier and thus the entire "cargo" (the adjuvant component and/or the antigen of the inventive vaccine composition) with a hydrophilic coating at its terminal ends, which prevents salt-mediated agglomeration and undesired interactions with serum contents. In the cytosol, this "coating" is easily removed under the reducing conditions of the cell. Also, this effect promotes deliberation of the cargo in the cytosol.

As defined above, ligands (L), may be optionally used in the polymeric carrier molecule according to generic formula (VI), e.g. for direction of the carrier polymer and its entire "cargo" (the adjuvant component and/or the antigen of the inventive vaccine composition) into specific cells. They may be selected independent from the other from RGD, Transferrin, Folate, a signal peptide or signal sequence, a localization signal or sequence, a nuclear localization signal or sequence (NLS), an antibody, a cell penetrating peptide (CPP), (e.g. TAT, KALA), a ligand of a receptor (e.g. cytokines, hormones, growth factors etc), small molecules (e.g. carbohydrates like mannose or galactose or synthetic ligands), small molecule agonists, inhibitors or antagonists of receptors (e.g. RGD peptidomimetic analogues) or any such molecule as further defined below. etc. Particularly preferred are cell penetrating peptides (CPPs), which induce a pH-mediated conformational change in the endosome and lead to an improved release of the polymeric carrier (in complex with a nucleic acid) from the endosome by insertion into the lipid layer of the liposome. Such called CPPs or cationic peptides for transportation, may include, without being limited thereto protamine, nucleoline, spermine or spermidine, poly-L-lysine (PLL), basic polypeptides, poly-arginine, cell penetrating peptides (CPPs), chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia*)*,* pAntp, plsl, etc., antimicrobial-derived CPPs e.g. Buforin-2, Bac715-24, SynB, SynB(1), pVEC, hCT-derived peptides, SAP, MAP, KALA, PpTG20, Proline-rich peptides, Loligomers, Arginine-rich peptides, Calcitonin-peptides, FGF, Lactoferrin, , poly-L-Lysine, poly-Arginine, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc. Particularly preferred in this context is mannose as ligand to target antigen presenting cells which carries on their cell membrane mannose receptors. In a further preferred aspect of the first embodiment of the present invention galactose as optional ligand can be used to target hepatocytes. Such ligands may be attached to component P¹ and/or P³ by reversible disulfide bonds as defined below or by any other possible chemical attachement, e.g. by amide formation (e.g. carboxylic acids, sulphonic acids, amines, etc), by Michael addition (e.g. maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components.

In the context of formula (VI) of the present invention components P¹ and P³ represent a linear or branched hydrophilic polymer chain, containing at least one -SH-moiety, each P¹ and P³ independently selected from each other, e.g. from polyethylene glycol (PEG), poly-*N*-(2-hydroxypropyl)methacrylamide, poly-2-(methacryloyloxy)ethyl phosphorylcholines, poly(hydroxyalkyl L-asparagine) or poly(hydroxyalkyl L-glutamine). P¹ and P³ may be identical or different to each other. Preferably, each of hydrophilic polymers P¹ and P³ exhibits a molecular weight of about 1 kDa to about 100 kDa, preferably of about 1 kDa to about 75 kDa, more preferably of about 5 kDa to about 50 kDa, even more preferably of about 5 kDa to about 25 kDa. Additionally, each of hydrophilic polymers P¹ and P³ typically exhibits at least one -SH-moiety, wherein the at least one -SH-moiety is capable to form a disulfide linkage upon reaction with component P² or with component (AA) or (AA)ₓ, if used as linker between P¹ and P² or P³ and P² as defined below and optionally with a further component, e.g. L and/or (AA) or (AA)ₓ, e.g. if two or more -SH-moieties are contained. The following subformulae "P¹-S-S-P²" and "P²-S-S-P³" within generic formula (VI) above (the brackets are omitted for better readability), wherein any of S, P¹ and P³ are as defined herein, typically represent a situation, wherein one-SH-moiety of hydrophilic polymers P¹ and P³ was condensed with one -SH-moiety of component P² of generic formula (VI) above, wherein both sulphurs of these -SH-moieties form a disulfide bond -S-S- as defined herein in formula (VI). These -SH-moieties are typically provided by each of the hydrophilic polymers P¹ and P³, e.g. via an internal cysteine or any further (modified) amino acid or compound which carries a -SH moiety. Accordingly, the subformulae "P¹-S-S-P²" and "P²-S-S-P³" may also be written as "P¹-Cys-Cys-P²" and "P²-Cys-Cys-P³", if the - SH- moiety is provided by a cysteine, wherein the term Cys-Cys represents two cysteines coupled via a disulfide bond, not via a peptide bond. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" also may be understood generally as "-(Cys-S)-(S-Cys)-", wherein in this specific case S indicates the sulphur of the -SH-moiety of cysteine. Likewise, the terms "-S-Cys" and "-Cys-S" indicate a disulfide bond between a -SH containing moiety and a cysteine, which may also be written as "-S-(S-Cys)" and "-(Cys-S)-S". Alternatively, the hydrophilic polymers P¹ and P³ may be modified with a -SH moiety, preferably via a chemical reaction with a compound carrying a -SH moiety, such that each of the hydrophilic polymers P¹ and P³ carries at least one such -SH moiety. Such a compound carrying a -SH moiety may be e.g. an (additional) cysteine or any further (modified) amino acid, which carries a -SH moiety. Such a compound may also be any non-amino compound or moiety, which contains or allows to introduce a -SH moiety into hydrophilic polymers P¹ and P³ as defined herein. Such non-amino compounds may be attached to the hydrophilic polymers P¹ and P³ of formula (VI) of the polymeric carrier according to the present invention via chemical reactions or binding of compounds, e.g. by binding of a 3-thio propionic acid or thioimolane, by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. A particularly preferred PEG derivate in this context is alpha-Methoxy-omega-mercapto poly(ethylene glycol). In each case, the SH-moiety, e.g. of a cysteine or of any further (modified) amino acid or compound, may be present at the terminal ends or internally at any position of hydrophilic polymers P¹ and P³. As defined herein, each of hydrophilic polymers P¹ and P³ typically exhibits at least one -SH-moiety preferably at one terminal end, but may also contain two or even more -SH-moieties, which may be used to additionally attach further components as defined herein, preferably further functional peptides or proteins e.g. a ligand, an amino acid component (AA) or (AA)ₓ, antibodies, cell penetrating peptides or enhancer peptides (e.g. TAT, KALA), etc.

According to one preferred alternative, such further functional peptides or proteins may comprise so called cell penetrating peptides (CPPs) or cationic peptides for transportation. Particularly preferred are CPPs, which induce a pH-mediated conformational change in the endosome and lead to an improved release of the polymeric carrier (in complex with a nucleic acid) from the endosome by insertion into the lipid layer of the liposome. Such called cell penetrating peptides (CPPs) or cationic peptides for transportation, may include, without being limited thereto protamine, nucleoline, spermine or spermidine, poly-L-lysine (PLL), basic polypeptides, poly-arginine, cell penetrating peptides (CPPs), chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia),* pAntp, plsl, etc., antimicrobial-derived CPPs e.g. Buforin-2, Bac715-24, SynB, SynB(1), pVEC, hCT-derived peptides, SAP, MAP, KALA, PpTG20, Proline-rich peptides, Loligomers, Arginine-rich peptides, Calcitonin-peptides, FGF, Lactoferrin, , poly-L-Lysine, poly-Arginine, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc.

According to a further preferred aspect of the first embodiment of the present invention, each of hydrophilic polymers P¹ and P³ of formula (VI) of the polymeric carrier used according to the present invention may also contain at least one further functional moiety, which allows attaching further components as defined herein, e.g. a ligand, an amino acid component (AA) or (AA)ₓ, etc. Such functional moieties may be selected from functionalities which allow the attachment of further components, e.g. functionalities as defined herein, e.g. by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components.

Component P² of formula (VI) of the polymeric carrier used according to the first embodiment of the present invention preferably represents a cationic or polycationic peptide or protein or alternatively a cationic or polycationic polymer. Each component P² typically exhibits at least two -SH-moieties, capable to form a disulfide linkage upon condensation with further components P², component(s) P¹ and/or P³ or alternatively with further components, e.g. amino acid components (AA)ₓ. Component P² typically occurs within the repetitive component [-S-P²-S-]ₙ of formula (VI) of the present invention. The term "cationic or polycationic" typically refers to a charged molecule, which is positively charged (cation) at a pH value of about 1 to 9, preferably 4 to 9, 5 to 8 or even 6 to 8, more preferably of a pH value of or below 9, of or below 8, of or below 7, most preferably at physiological pH values, e.g. about 7.3 to 7.4. Accordingly, a cationic or polycationic peptide or protein as component P² or alternatively a cationic or polycationic polymer as component P² of formula (VI) of the polymeric carrier according to the present invention is positively charged under physiological conditions, particularly under physiological salt conditions of the cell *in vivo.*

In the specific case that component P² of formula (VI) of the present invention is a cationic or polycationic peptide or protein the cationic properties of component [S-P²-S]ₙ or {[S-P²-S]ₐ/[S-(AA)ₓ-S]_{b} (as defined below) may be determined upon its content of cationic amino acids in the entire component [S-P²-S]ₙ or {[S-P²-S]ₐ/[S-(AA)ₓ-S]_{b}}. Preferably, the content of cationic amino acids in component [S-P²-S]ₙ or {[S-P²-S]ₐ/]S-(AA)ₓ-S]_{b}} is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even more preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in the entire component [S-P²-S]ₙ or {[S-P²-S]ₐ/[S-(AA)ₓ-S]_{b}} is 100%.

In the specific case that component P² of formula (VI) of the polymeric carrier of the present invention is a cationic or polycationic polymer the cationic properties of component [S-P²-S]ₙ or {[S-P²-S]ₐ/[S-(AA)ₓ-S]_{b}} may be determined upon its content of cationic charges in the entire component [S-P²-S]ₙ or {[S-P²-S]ₐ/[S-(AA)ₓ-S]_{b}} when compared to the overall charges of component [S-P²-S]ₙ or {[S-P²-S]ₐ/[S-(AA)ₓ-S]_{b}}. Preferably, the content of cationic charges in component [S-P²-S]ₙ or {[S-P²-S]ₐ/[S-(AA)ₓ-S]_{b}} at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even preferably in the range of about 20% to 50%, e.g. 20, 30, 40 or 50%, or in a range formed by any two of the afore mentioned values, provided, that the content of all charges, e.g. positive and negative charges at a (physiological) pH as defined herein, in the entire component [S-P²-S]ₙ or {[S-P²-S]ₐ/[S-(AA)ₓ-S]_{b}} is 100%.

In the specific context of a complex formed by the adjuvant component, the antigen and the polymeric carrier molecule according to generic formula (VI) L-P¹-S-[S-P²-S]ₙ-S-P³-L as defined herein (or according to any of its subformulae herein), forming the inventive vaccine composition, it is particularly preferred that at least 10% of all charges in the whole repetitive component [S-P²-S]ₙ or {[S-P²-S]ₐ/[S-(AA)ₓ-S]_{b}} are cationic to allow complexation of the negatively charged "cargo".

The cationic or polycationic peptide or protein as component P² of formula (VI) of the polymeric carrier, or the cationic or polycationic polymer as component P², is preferably a linear molecule, however, branched cationic or polycationic peptides or proteins as component P² or branched cationic or polycationic polymers as component P² may also be used.

Typically, component P² of formula (VI) of the polymeric carrier, e.g. the cationic or polycationic peptide or protein or the cationic or polycationic polymer as defined herein, is linked to its neighbouring components, e.g. components P¹ and P³, and/or as a part of repetitive component [S-P²-S]ₙ to further components P², via disulfide bonds (-S-S-) or to (AA)ₓ components as part of {[S-P²-S]ₐ/[S-(AA)ₓ-S]_{b}}. In this context, the sulphurs adjacent to component P² in the repetitive component [S-P²-S]ₙ and as defined in generic formula (VI) L-P¹-S-[S-P²-S]ₙ-S-P³-L of the polymeric carrier, necessary for providing a disulfide bond, may be provided by component P² itself by a -SH moiety as defined herein or may be provided by modifying component P² accordingly to exhibit a -SH moiety within the above definition of repetitive component [S-P²-S]ₙ. The -SH moieties for component P² are preferably as defined herein for components P¹ and P³. If such -SH-moieties, necessary to form a disulfide bond (-S-S-) within the above meaning, are provided by component P² itself this may occur e.g. by at least two cysteines or any further (modified) amino acids or chemical compounds, which carry a -SH moiety, already occurring within the amino acid sequence of component P² at whatever position of the amino acid sequence of component P². Alternatively, component P² may be modified accordingly with a chemical compound, e.g. a cysteine or any further (modified) amino acid or chemical compound, which carries a (free) -SH moiety. Thereby, component P² preferably carries at least two -SH-moieties, which sulphurs atoms are capable to form a disulfide bond upon condensation with a-SH-moiety of components P¹ or P³ as defined herein, or between a first component P² and a further component P², etc. Such -SH-moieties are preferably as defined herein. Preferably the at least two SH-moieties are located at the terminal ends or near to the terminal ends of component P²

According to one specific aspect component P² of formula (VI) of the polymeric carrier may comprise within repetitive component [S-P²-S]ₙ a cysteine as a -SH moiety, e.g. one or both of S as defined above may be a Cysteine. In this context, repetitive component [S-P²-S]ₙ may thus be written as follows:

[Cys-P²-Cys]ₙ

wherein n and P² are as defined herein and each Cys provides for the -SH-moiety for the disulfide bond. Cys is the amino acid cysteine in its three letter code. (For illustrative purposes, in the present description the disulfide bond -S-S- generally may also be written as -(Cys-S)-(S-Cys)-, wherein Cys-S represents a Cysteine with an naturally occurring -SH moiety, wherein this -SH moiety forms a disulfide bond with a -SH moiety of a second cysteine. Accordingly, repetitive component [Cys-P²-Cys]ₙ may also be written as [(S-Cys)-P²-(Cys-S)]ₙ, which indicates that the -SH-moiety is provided by a cysteine and the Cysteine itself provides for the sulphur of the disulfide bond).

In the context of the entire formula (VI) of the polymeric carrier may thus be defined as follows:

L-P¹-S-[Cys-P²-Cys]ₙ-S-P³-L

wherein L, P¹, P², P³ and n are as defined herein, S is sulphur and each Cys provides for one -SH-moiety for the disulfide bond.

In each case, the SH-moiety, e.g. of a cysteine or any further (modified) amino acid or further compound used for modification of component P², may be present in the cationic or polycationic peptide or protein or cationic or polycationic polymer as component P², internally or at one or both of its terminal ends, e.g. if a cationic or polycationic peptide or protein is used as component P² at the N-terminal end or at the C-terminal end, at both these terminal ends, and/or internally at any position of the cationic or polycationic peptide or protein as component P². Preferably, the -SH moiety may be present in component P² at least at one terminal end, more preferably at both terminal ends, e.g. at the N-terminal end and/or at the C-terminal end, more preferably at both the N-terminal and the C-terminal end of a cationic or polycationic peptide or protein as component P².

Due to its repetitive character component [S-P²-S]ₙ within formula (VI) of the polymeric carrier may represent a situation, wherein one of the at least two -SH-moieties of component P² was condensed with a -SH-moiety of a further component P² of generic formula (VI) above, wherein both sulphurs of these -SH-moieties form a disulfide bond (-S-S-) between a first component P² and at least one further component P².

In this context, the number of repetitions of component P² in formula (VI) according to the present invention is defined by integer n. n is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. If, for example, in repetitive component [S-P²-S]ₙ integer n is 5, repetitive component [S-P²-S]ₙ preferably reads as follows:

[S-P²-S-S-P²-S-S-P²-S-S-P²-S-S-P²-S]

In the above example component P² occurs 5 times (preferably in a linear order), wherein each component P² is linked to its neighbor component by a disulfide bond within the above definition of repetitive component [S-P²-S]ₙ. Any of components P² may be the same or different from each other.

According to one particular aspect, component P² of formula (VI) of the polymeric carrier represents a cationic or polycationic peptide or protein having a length of about 3 to about 100 amino acids, more preferably having a length of about 3 to about 50 amino acids, even more preferably having a length of about 3 to about 25 amino acids, e.g. having a length of about 3 to 10, 5 to 15, 10 to 20 or 15 to 25 amino acids, more preferably a length of about 5 to about 20 and even more preferably a length of about 10 to about 20.

The cationic or polycationic peptide or protein as component P² of formula (VI) of the polymeric carrier may be any protein or peptide suitable for this purpose and exhibiting at least two -SH-moieties, particular any cationic or polycationic peptide or protein capable to complex a nucleic acid as defined according to the present invention, and thereby preferably condensing the nucleic acid.

Likewise preferred, cationic or polycationic peptides or proteins as component P² of formula (VI) of the polymeric carrier exhibiting at least two -SH-moieties may be selected from protamine, nucleoline, spermine or spermidine, poly-L-lysine (PLL), basic polypeptides, poly-arginine, cell penetrating peptides (CPPs), chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia),* pAntp, plsl, etc., antimicrobial-derived CPPs e.g. Buforin-2, Bac715-24, SynB, SynB(1), pVEC, hCT-derived peptides, SAP, MAP, KALA, PpTG20, Proline-rich peptides, Loligomere, Arginine-rich peptides, Calcitonin-peptides, FGF, Lactoferrin, histones, VP22 derived or analog peptides, HSV, VP22 (Herpes simplex), MAP, KALA or protein transduction domains (PTDs, PpT620, prolin-rich peptides, lysine-rich peptides, Pep-1, L-oligomers, Calcitonin peptide(s), etc.

According to one further particular aspect of the first embodiment of the present invention, cationic or polycationic peptides or proteins as component P² of formula (VI) of the polymeric carrier are selected from following cationic peptides having the following total sum formula (V) or of any of subformulae (Va) or (Vb) or specific sequences thereof as defined above, preferably under the proviso that these sequences additionally exhibit at least one or two -SH-moieties (as defined above):

{(Arg)ₗ;(Lys)ₘ;(His)ₙ;(Orn)ₒ;(Xaa)ₓ} (formula (V))

{(Arg)ₗ;(Lys)ₘ;(His)ₙ;(Orn)ₒ;(Xaa')ₓ(Cys)_{y}} formula (Va)

Cys¹{(Arg)ₗ;(Lys)ₘ;(His)ₙ;(Orn)ₒ;(Xaa)ₓ}Cys² (formula (Vb))

Preferably, component P² of formula (VI) of the polymeric carrier represents a cationic or polycationic polymer, typically exhibiting a molecular weight of about 0.5 kDa to about 100 kDa, of about 1 kDa to about 75 kDa, of about 5 kDa to about 50 kDa, of about 5 kDa to about 30 kDa, or a molecular weight of about 10 kDa to about 50 kDa, or of about 10 kDa to about 30 kDa, preferably of about 0.5 kDa to about 30 kDa, more preferably of about 1 kDa to about 20 kDa, and even more preferably of about 1.5 kDa to about 10 kDa, likewise preferably a molecular weight of about 0.3 kDa to about 20 kDa, preferably 0.3 kDa to about 10 kDa, including a molecular weight of about 0.4 kDa to about 10 kDa, 0.5 kDa to about 10 kDa, preferably of about 0.5 kDa to about 7.5 kDa, 0.5 kDa to about 5 kDa, 0.5 kDa to about 4 kDa, 0.5 kDa to about 3 kDa, or even 0.67 kDa to about 2.7 kDa.

Additionally, the cationic or polycationic polymer as component P² typically exhibits at least two -SH moieties, which are capable to form a disulfide linkage upon condensation with either components P¹ or P³ or with other components P² or amino acid components (AA) or (AA)ₓ. as defined herein.

When component P² of formula (VI) of the polymeric carrier represents a cationic or polycationic polymer, such a polymer may likewise be selected from acrylates, modified acrylates, such as pDMAEMA (poly(dimethylaminoethyl methylacrylate)), chitosanes, aziridines or 2-ethyl-2-oxazoline (forming oligo ethylenimines or modifed oligoethylenimines), polymers obtained by reaction of bisacrylates with amines forming oligo beta aminoesters or poly amido amines, or other polymers like polyesters, polycarbonates, etc. Each molecule of these cationic or polycationic polymers typically exhibits at least two -SH-moieties, wherein these at least two -SH-moieties may be introduced into the cationic or polycationic polymer by chemical modifications, e.g. using imonothiolan, 3-thio propionic acid or introduction of -SH-moieties containing amino acids, such as cystein, methionine or any further (modified) amino acid. Such -SH-moieties are preferably as already defined above for components P¹, P² or P³.

Component P² of formula (VI) of the polymeric carrier preferably occurs as repetitive component [-S-P²-S-]ₙ. Such a repetitive component [S-P²-S]ₙ may be prepared using at least one or even more of the same or different of the above defined components P² and polymerizing same in a polymerization condensation reaction via their-SH-moieties.

According to one specific aspect, such a repetitive component [S-P²-S]ₙ within formula (VI) of the polymeric carrier may be prepared using at least one or even more of the same or different of the above defined cationic or polycationic peptides or proteins, and polymerizing same in a polymerization condensation reaction *via* their -SH-moieties. Accordingly, such a repetitive component [S-P²-S]ₙ within formula (VI) of the polymeric carrier contains a number of at least one or even more of the same or different of the above defined cationic or polycationic proteins or peptides determined by integer n.

According to another specific aspect, such a repetitive component [S-P²-S]ₙ within formula (VI) of the polymeric carrier may be prepared using at least one or even more of the same or different of the above defined cationic or polycationic polymers, and polymerizing same in a polymerization condensation reaction via their -SH-moieties. Accordingly, such a repetitive component [S-P²-S]ₙ contains a number of at least one or even more of the same or different of the above defined cationic or polycationic polymers determined by integer n. According to a further specific aspect, such a repetitive component [S-P²-S]ₙ within formula (VI) of the polymeric carrier may be prepared using at least one or even more of the same or different of the above defined cationic or polycationic polymers and at least one or even more of the same or different of the above defined cationic or polycationic proteins or peptides, and polymerizing same in a polymerization condensation reaction via their -SH-moieties. Accordingly, such a repetitive component [S-P²-S]ₙ within formula (VI) of the polymeric carrier contains a number of at least one or even more of the same or different of the above defined cationic or polycationic polymers and at least one or even more of the same or different of the above defined cationic or polycationic proteins or peptides , both together determined by integer n.

According to a particular aspect, the polymeric carrier according to formula (VI) as defined above, may comprise at least one amino acid component (AA), wherein AA is preferably an amino acid as defined in the following or a combination of amino acids as defined in the following, e.g. selected from an aromatic, a hydrophilic, a lipophilic, or a weak basic amino acid or peptide as defined in the following. Amino acid component (AA) preferably allows to (substantially) modify the biophysical/biochemical properties of the polymeric carrier according to formula (VI) as defined herein. Preferably, the amino acid component may occur as amino acid component (AA)ₓ, wherein the number of amino acids in such an amino acid component (AA)ₓ (repetitions) is defined by x. In the above context, x is preferably an integer and may be selected from a range of about 1 to 100, preferably from a range of about 1 to 50, more preferably 1 to 30, and even more preferably selected from a number comprising 1, 2, 3, 4, 5, 6, 7, 8, 9, 10, 11, 12, 13, 14 or 15-30, e.g. from a range of about 1 to 30, from a range of about 1 to 15, or from a number comprising 1, 2, 3, 4, 5, 6, 7, 8, 9, 10, 11, 12, 13, 14 or 15, or may be selected from a range formed by any two of the afore mentioned values. Most preferably, x is 1.

Such an amino acid component (AA) or (AA)ₓ may be contained in every part of the polymeric carrier according to formula (VI) above and therefore may be attached to all components of the polymeric carrier according to formula (VI). It is particularly preferred that amino acid component (AA) or (AA)ₓ is present as a ligand or part of the repetitive component [S-P²-S]ₙ within formula (VI) of the polymeric carrier.

In this context it is particularly preferred that the amino acid component (AA) or (AA)ₓ contains or is flanked (e.g. terminally) by at least one -SH containing moiety, which allows introducing this component (AA) or (AA)ₓ via a disulfide bond into the polymeric carrier according to formula (VI) as defined herein. In this context, the amino acid component (AA) or (AA)ₓ may also be read as a component -S-(AA)ₓ- or -S-(AA)ₓ-S-, wherein S represents a - SH containing moiety (or, of course, one sulphur of a disulfide bond), e.g. a cysteine residue. In the specific case that the -SH containing moiety represents a cysteine, the amino acid component (AA)ₓ may also be read as -Cys-(AA)ₓ- or -Cys-(AA)ₓ-Cys- wherein Cys represents Cysteine and provides for the necessary -SH-moiety for a disulfide bond. (Accordingly, -Cys-(AA)ₓ-Cys- may also be written as -(S-Cys)-(AA)ₓ-(Cys-S)- and -Cys-(AA)ₓ-may also be written as -(S-Cys)-(AA)ₓ-)) The -SH containing moiety may be also introduced into the amino acid component (AA)ₓ using any of modifications or reactions as shown above for components P¹, P² or P³. In the specific case that the amino acid component (AA)ₓ is linked to two components of the polymeric carrier according to formula (VI) it is preferred that (AA) or (AA)ₓ contains at least two -SH-moieties, e.g. at least two Cysteines, preferably at its terminal ends. This is particularly preferred if (AA) or (AA)ₓ is part of the repetitive component [S-P²-S]ₙ.

In an alternative the amino acid component (AA) or (AA)ₓ is introduced into the polymeric carrier according to formula (VI) as defined herein via any chemical possible addition reaction. Therefore the amino acid component (AA) or (AA)ₓ contains at least one further functional moiety, which allows attaching same to a further component as defined herein, e.g. component P¹ or P³, P², L, or a further amino acid component (AA) or (AA)ₓ, etc. Such functional moieties may be selected from functionalities which allow the attachment of further components, e.g. functionalities as defined herein, e.g. by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components.

The amino acid component (AA) or (AA)ₓ may also occur as a mixed repetitive amino acid component [(AA)ₓ]_{z}, wherein the number of amino acid components (AA) or (AA)ₓ is further defined by integer z. In this context, z may be selected from a range of about 1 to 30, preferably from a range of about 1 to 15, more preferably 1 to 10 or 1 to 5 and even more preferably selected from a number selected from 1, 2, 3, 4, 5, 6, 7, 8, 9, 10, 11, 12, 13, 14 or 15, or may be selected from a range formed by any two of the afore mentioned values. Such a mixed repetitive amino acid component [(AA)ₓ]_{z} may be used to integrate several of the same or different amino acid components (AA)ₓ as defined herein in the polymeric carrier. Preferably, in the mixed repetitive amino acid component [(AA)ₓ]_{z} the amino acid component (AA)ₓ may contain or may be flanked (e.g. terminally) by at least one -SH containing moiety, preferably at least two -SH containing moieties as already defined above, which allows coupling the different amino acid components (AA)ₓ using a disulfide bond via a condensation polymerization. Likewise as above, the mixed repetitive amino acid component [(AA)ₓ]_{z} may also be read as [S-(AA)ₓ-S]_{z}, wherein S represents a -SH containing moiety, e.g. a cysteine residue. In the specific case that the -SH containing moiety represents a cysteine, the mixed repetitive amino acid component [(AA)ₓ]_{z} may also be read as [Cys-(AA)ₓ-Cys]_{z}, wherein Cys represents Cysteine and provides for the necessary -SH-moiety for a disulfide bond. The -SH containing moiety may be also introduced into the amino acid component (AA) or (AA)ₓ using any of modifications or reactions as shown above for components P¹, P² or P³.

The amino acid component (AA) or (AA)ₓ or the mixed repetitive amino acid component [(AA)ₓ]_{z} may be provided with at least one -SH-moiety, e.g. in a form represented by formula (AA)ₓ-SH. Then, the component (AA) or (AA)ₓ according to formula (AA)ₓ-SH or the mixed repetitive amino acid component [(AA)ₓ]_{z} according to formula [(AA)ₓ]_{z}-SH, may be bound to any of components L, P¹, P² and/or P³ or another component (AA) or (AA)ₓ via a disulfide bond. If bound to component P¹ and/or component P³, components P¹ and/or P³ preferably exhibit at least two -SH-moieties to allow further binding of components P¹ and/or P³ to a component P² via a -SH-moiety forming a disulfide bond (see above). The amino acid component (AA) or (AA)ₓ in a form represented by formula (AA)ₓ-SH or the mixed repetitive amino acid component [(AA)ₓ]_{z} according to formula [(AA)ₓ]_{z}-SH may be also used to terminate a condensation reaction due to its single -SH moiety. In this case, the amino acid component (AA) or (AA)ₓ in a form represented by formula (AA)ₓ-SH is preferably coupled terminally to components P¹ and/or P³. The amino acid component (AA) or (AA)ₓ in a form represented by formula (AA)ₓ-SH or the mixed repetitive amino acid component [(AA)ₓ]_{z} according to formula [(AA)ₓ]_{z}-SH may be also used to bind internally to any of components L, P¹, P² and/or P³ or a further component (AA) or (AA)ₓ via a further internal -SH-moiety of any of components L, P¹, P² and/or P³ or (AA)ₓ.

Furthermore, the amino acid component (AA) or (AA)ₓ may be provided with two -SH-moieties (or even more), e.g. in a form represented by formula HS-(AA)ₓ-SH. Additionally, the mixed repetitive amino acid component [(AA)ₓ]_{z} may be provided with two -SH-moieties (or even more), e.g. in a form represented by formula HS-[(AA)ₓ]_{z}-SH, to allow binding to two functionalities via disulfide bonds, e.g. if the amino acid component (AA)ₓ or the mixed repetitive amino acid component [(AA)ₓ]_{z} is used as a linker between two further components (e.g. as a linker between components L and P¹, between components P¹ and P², in or as a part of repetitive component [S-P²-S]ₙ, between components P² and P³ and/or between components P³ and L). In this case, one -SH moiety is preferably protected in a first step using a protecting group as known in the art, leading to an amino acid component (AA)ₓ of formula HS-(AA)ₓ-S-protecting group or to a mixed repetitive amino acid component [(AA)ₓ]_{z} of formula HS-[(AA)ₓ]_{z}-S-protecting group. Then, the amino acid component (AA)ₓ or the mixed repetitive amino acid component [(AA)ₓ]_{z} may be bound to a component L, P¹, P² and/or P³, to form a first disulfide bond via the non-protected -SH moiety. The protected-SH-moiety is then typically deprotected and bound to a further free - SH-moiety of a further component L, P¹, P² and/or P³ to form a second disulfide bond. In the case that the amino acid component (AA)ₓ or the mixed repetitive amino acid component [(AA)ₓ]_{z} is part of the repetitive component [S-P²-S]ₙ it is preferred that the formation of the disulfide bonds between (AA) or (AA)ₓ and P² concurrently occurs with the polycondensation reaction of the repetitive component [S-P²-S]ₙ and therefore no protection of the at least two terminal -SH-moieties is not necessary.

Alternatively, the amino acid component (AA) or (AA)ₓ or the mixed repetitive amino acid component [(AA)ₓ]_{z} may be provided with other functionalities as already described above for components P¹ and P² and/or P³, which allow binding of the amino acid component (AA)ₓ or binding of the mixed repetitive amino acid component [(AA)ₓ]_{z} to any of components P¹, P² and/or P³ or (AA) or (AA)ₓ and optionally to component L.

Thus, according to the present invention, the amino acid component (AA) or (AA)ₓ and/or the mixed repetitive amino acid component [(AA)ₓ]_{z} may be bound to P¹, P², P³, (AA) or (AA)ₓ and/or L with or without using a disulfide linkage. Binding without using a disulfide linkage may be accomplished by any of the reactions described above, preferably by binding the amino acid component (AA) or (AA)ₓ or the mixed repetitive amino acid component [(AA)ₓ]_{z} to P¹, P², P³, (AA)ₓ and/or L using an amid-chemistry as defined herein. If desired or necessary, the other terminus of the amino acid component (AA) or (AA)ₓ or the mixed repetitive amino acid component [(AA)ₓ]_{z}, e.g. the N- or C-terminus, may be used to couple another component, e.g. a ligand L. For this purpose, the other terminus of the amino acid component (AA) or (AA)ₓ or the mixed repetitive amino acid component [(AA)ₓ]_{z} preferably comprises or is modified to comprise a further functionality, e.g. an alkyn-species (see above), which may be used to add the other component via e.g. click-chemistry. Such a construct, e.g. L-(AA)ₓ-P¹-S-or L-[(AA)ₓ]_{z}-P¹-S-, may be used to terminate the polymerization condensation reaction of repetitive component [S-P²-S]ₙ. If the ligand is bound via an acid-labile bond, the bond may be cleaved off in the endosome and the polymeric carrier presents amino acid component (AA) or (AA)ₓ or the mixed repetitive amino acid component [(AA)ₓ]_{z} at its surface.

The amino acid component (AA) or (AA)ₓ or the mixed repetitive amino acid component [(AA)ₓ]_{z} may occur as a further component of generic formula (VI) above, e.g. as a linker between components P¹ or P³ and P², as a linker between components L and P¹ or P² or as an additional component of the repetitive component [S-P²-S]ₙ.

According to a first alternative, such an amino acid component (AA) or (AA)ₓ or the mixed repetitive amino acid component [(AA)ₓ]_{z} may be present as a linker between components P¹ or P³ and component P². This is preferably represented in the context of the entire polymeric carrier according to formula (VI) by following formulae:

L-P¹-S-S-(AA)ₓ-S-[S-P²-S]ₙ-S-(AA)ₓ-S-S-P³-L,

or

L-P¹-S-[S-(AA)ₓ-S]_{z}-[S-P²-S]ₙ-[S-(AA)ₓ-S]z-S-P³-L,

wherein n, x, z, S, L, AA, P¹, P² and P³ are preferably as defined herein. In the above formulae, the term "-S-S-" represents a disulfide bond, wherein this at least one sulphur of the disulfide bond may also be provided by a cysteine. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" may also be understood generally as "-(Cys-S)-(S-Cys)-", wherein in this specific case S indicates the sulphur of the -SH-moiety of cysteine. Likewise, the terms "-S-Cys" and "-Cys-S" indicate a disulfide bond between a -SH containing moiety and a cysteine, which may also be written as "-S-(S-Cys)" and "-(Cys-S)-S".

According to a second alternative, such an amino acid component (AA) or (AA)ₓ or the mixed repetitive amino acid component [(AA)ₓ]_{z} may be present as a linker between components P¹ or P³ and component L. This is preferably represented in the context of the entire polymeric carrier according to formula (VI) by following formulae:

L-(AA)ₓ-P¹-S-[S-P²-S]ₙ-S-P³-(AA)ₓ-L,

or

L-[(AA)ₓ]z-P¹-S-[S-P²-S]ₙ-S-P³-[(AA)ₓ]_{z}-L,

or alternatively

L-(AA)ₓ-S-S-P¹-S-[S-P²-S]ₙ-S-P³-S-S-(AA)ₓ-S-S-L,

or

L-S-S-(AA)ₓ-S-S-P¹-S-[S-P²-S]ₙ-S-P³-S-S-(AA)ₓ-S-S-L,

or

L-S-[S-(AA)ₓ-S]_{z}-S-P¹-S-[S-P²-S]ₙ-S-P³-S-[S-(AA)ₓ-S]_{z}-S-L,

etc. wherein n, x, z, S, L, AA, P¹, P² and P³ are preferably as defined herein. In the above formulae, the term "-S-S-" represents a disulfide bond, as already defined above.

According to a third alternative, such an amino acid component (AA) or (AA)ₓ or the mixed repetitive amino acid component [(AA)ₓ]_{z} may be present as a part of components P¹ and/or P³, wherein the amino acid component (AA)ₓ may be directly bound to (e.g. the terminus of) component P¹ and/or P³ without a further ligand L. In this case the (AA) or (AA)ₓ component may be in the form of a ligand as defined above. This is preferably represented in the context of the entire polymeric carrier according to formula (VI) by following formulae:

(AA)ₓ-P¹-S-[S-P²-S]ₙ-S-P³-(AA)ₓ,

or

[(AA)ₓ]_{z}-P¹-S-[S-P²-S]ₙ-S-P³-[(AA)ₓ]_{z},

or or alternatively

(AA)ₓ-S-S-P¹-S-[S-P²-S]ₙ-S-P³-S-S-(AA)ₓ,

or

H-[S-(AA)ₓ-S]_{z}-P¹-S-[S-P²-S]ₙ-S-S-P³-S-[S-(AA)ₓ-S]_{z}-H,

wherein n, x, z, S, AA, P¹, P² and P³ are preferably as defined herein. In the above formulae, the term "-S-S-" represents a disulfide bond, as already defined above. The free -SH moiety at the terminal ends in the last formula may also be terminated using a monothiol compound as defined herein.

According to a fourth and particularly preferred alternative, the amino acid component (AA) or (AA)ₓ, preferably written as S-(AA)ₓ-S or [S-(AA)ₓ-S] may be used to modify component P², particularly the content of component S-P²-S in repetitive component [S-P²-S]ₙ of formula (VI) above. This may be represented in the context of the entire polymeric carrier according to formula (VI) e.g. by following formula (VIa):

L-P¹-S-{[S-P²-S]ₐ[S-(AA)ₓ-S]_{b}}-S-P³-L,

wherein x, S, L, AA, P¹, P² and P³ are preferably as defined herein. In formula (VIa) above, any of the single components [S-P²-S]and [S-(AA)ₓ-S] may occur in any order in the subformula {[S-P²-S]ₐ[S-(AA)ₓ-S]_{b}}. The numbers of single components [S-P²-S]and [S-(AA)ₓ-S] in the subformula {[S-P²-S]ₐ[S-(AA)ₓ-S]_{b}} are determined by integers a and b, wherein a + b = n. n is an integer and is defined as above for formula (VI).
a is an integer, typically selected independent from integer b from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. Most preferably, a is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.
b is an integer, typically selected independent from integer a from a range of about 0 to 50 or 1 to 50, preferably from a range of about 0, 1, 2 or 3 to 30, more preferably from a range of about 0, 1, 2, 3, 4, or 5 to 25, or a range of about 0, 1, 2, 3, 4, or 5 to 20, or a range of about 0, 1, 2, 3, 4, or 5 to 15, or a range of about 0, 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. Most preferably, b is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

In the above formula, the term "-S-S-" (the brackets are omitted for better readability) represents a disulfide bond as already defined above.

The modification of component P², particularly of component S-P²-S of repetitive component [S-P²⁻S]ₙ, by "diluting" same with amino acid components (AA) or (AA)ₓ may be also realized in the context of any of the afore mentioned alternatives of the entire polymeric carrier according to formula (VI),

L-P¹-S-S-(AA)ₓ-S-{[S-P²-S]ₐ[S-(AA)ₓ-S]_{b}}-S-(AA)ₓ-S-S-P³-L,

or

L-P¹-S-[S-(AA)ₓ-S]_{z}-{[S-P²-S]ₐ[S-(AA)ₓ-S]_{b}}-[S-(AA)ₓ-S]_{z}-S-P³-L,

or

L-(AA)ₓ-P¹-S-{[S-P²-S]ₐ[S-(AA)ₓ-S]_{b}}-S-P³-(AA)ₓ-L,

or

L-[(AA)ₓ]_{z}-P¹-S-{[S-P²-S]ₐ[S-(AA)ₓ-S]_{b}}-S-P³-[(AA)ₓ]_{z}-L,

or

L-(AA)ₓ-S-S-P¹-S-{[S-P²-S]ₐ[S-(AA)ₓ-S]_{b}}-S-P³-S-S-(AA)ₓ-S-S-L,

or

L-S-S-(AA)ₓ-S-S-P¹⁻S-{[S-P²-S]ₐ[S-(AA)ₓ-S]_{b}}-S-P³-S-S-(AA)ₓ-S-S-L,

or

L-S-[S-(AA)ₓ-S]_{z}-S-P¹-S-{[S-P²-S]ₐ[S-(AA)ₓ-S]_{b}}-S-P³-S-[S-(AA)ₓ-S]_{z}-S-L,

or

(AA)ₓ-P¹-S-{[S-P²-S]ₐ[S-(AA)ₓ-S]_{b}}-S-P³-(AA)ₓ,

or

[(AA)ₓ]_{z}-P¹-S-{[S-P²-S]ₐ[S-(AA)ₓ-S]_{b}}-S-P³-[(AA)ₓ]_{z},

or

(AA)ₓ-S-S-P¹⁻S-{[S-P²-S]ₐ[S-(AA)ₓ-S]_{b}}-S-P³-S-S-(AA)ₓ,

or

H-[S-(AA)ₓ-S]_{z}-S-P¹-S-{[S-P²-S]ₐ[S-(AA)ₓ-S]_{b}}-S-P³⁻S-[S-(AA)ₓ-S]_{z}-H,

wherein n, x, z, a, b, S, L, AA, P¹, P² and P³ are preferably as defined herein. Likewise, the term "-S-S-" represents a disulfide bond and is preferably as defined herein.

In the above alternatives, wherein the component [S-P²-S] is preferably "diluted" with amino acid components [S-(AA)ₓ-S], the ratio is determined by integers a and b, wherein a + b = n. Preferably, integers a and b are selected such that the cationic binding properties of component [S-P²-S] are not lost but remain to a minimum extent in subformula/component {[S-P²-S]ₐ[S-(AA)ₓ-S]_{b}}. This allows to weaken ("dilute") the cationic binding strength of component [S-P²-S]in repetitive component [S-P²-S]ₙ of polymeric carrier of formula (VI) to a desired extent.

In this specific context the (desired) cationic binding strength of subformula/component {[S-P²-S]ₐ[S-(AA)ₓ-S]_{b}} may be determined using different methods.

According to a first alternative, component P² of formula (VI) of the present invention is particularly preferable a cationic or polycationic peptide as defined herein. Furthermore, the amino acid component (AA) or (AA)ₓ, preferably written as [S-(AA)ₓ-S], typically resembles a peptide sequence. In this specific case, the cationic properties of subformula/component {[S-P²-S]ₐ[S-(AA)ₓ-S]_{b}} may be determined upon their content of cationic amino acids in the entire subformula/component. Preferably, the content of cationic amino acids in subformula/component {[S-P²-S]ₐ[S-(AA)ₓ-S]_{b}} is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in the entire subformula/component {[S-P²-S]ₐ[S-(AA)ₓ-S]_{b}} is 100%.

According to a second alternative, component P² of formula (VI) of the present invention is particularly preferable a cationic or polycationic polymer as defined herein. The amino acid component (AA) or (AA)ₓ, preferably written as [S-(AA)ₓ-S], typically resembles a peptide sequence. In this specific case, the cationic properties of subformula/component {[S-P²-S]ₐ[S-(AA)ₓ-S]_{b}} may be determined upon their content of cationic charges in the entire subformula/component. Preferably, the content of cationic charges in subformula/component {[S-P²-S]ₐ[S-(AA)ₓ-S]_{b}} at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even preferably in the range of about 20% to 50%, e.g. 20, 30, 40 or 50%, or in a range formed by any two of the afore mentioned values, provided, that the content of all charges, e.g. positive and negative charges at a (physiological) pH as defined herein, in the entire subformula/component {[S-P²-S]ₐ[S-(AA)ₓ-S]_{b}} is 100%.

According to the present invention, the amino acid component (AA) preferably comprises a number of amino acids preferably in a range of about 1 to 100, preferably in a range of about 1 to 50, more preferably selected from a number comprising 1, 2, 3, 4, 5, 6, 7, 8, 9, 10, 11, 12, 13, 14 or 15-20, or may be selected from a range formed by any two of the afore mentioned values. In this context the amino acids of amino acid component (AA) can be chosen independently from each other. For example if in the polymeric carrier according to formula (VI) above (or according to any of its subformulae herein) two or more (AA) components are present they can be the same or can be different from each other.

The amino acid component (AA) may contain or may be flanked (e.g. terminally) by an -SH containing moiety, which allows introducing this amino acid component (AA) via a disulfide bond into the polymeric carrier according to formula (VI) above (or according to any of its subformulae herein) as defined herein. In the specific case that the -SH containing moiety represents a cysteine, the amino acid component (AA) may also be read as -Cys-(AA)-Cys- or -Cys-(AA), wherein Cys represents Cysteine and provides for the necessary - SH-moiety for a disulfide bond. The -SH containing moiety may be also introduced into amino acid component (AA) itself using any of modifications or reactions as shown above for the polymeric carrier according to formula (VI) above (or according to any of its subformulae herein).

Furthermore, the amino acid component (AA) may be provided with two -SH-moieties (or even more), e.g. in a form represented by formula HS-(AA)-SH to allow binding to two functionalities via disulfide bonds, e.g. if the amino acid component (AA) is used as a linker between two further components (e.g. as a linker between two disulfide-crosslinked cationic compounds, e.g. of the polymeric carrier according to formula (VI) above (or according to any of its subformulae herein)). In this case, one -SH moiety is preferably protected in a first step using a protecting group as known in the art, leading to an amino acid component (AA) of formula HS-(AA)-S-protecting group. Then, the amino acid component (AA) may be bound to a further component of the polymeric carrier according to formula (VI) above (or according to any of its subformulae herein), to form a first disulfide bond via the non-protected -SH moiety. The protected-SH-moiety is then typically deprotected and bound to a further free -SH-moiety of a further component of the polymeric carrier to form a second disulfide bond.

Alternatively, the amino acid component (AA) may be provided with other functionalities as already described above for the other components of the polymeric carrier according to formula (VI) above (or according to any of its subformulae herein), which allow binding of the amino acid component (AA) to any of components of the polymeric carrier.

Thus, according to the present invention, the amino acid component (AA) as defined above may likewise be bound to further components of the polymeric carrier according to formula (VI) above (or according to any of its subformulae herein) with or without using a disulfide linkage. Binding without using a disulfide linkage may be accomplished by any of the reactions described above, preferably by binding the amino acid component (AA) to the other component of the polymeric carrier according to formula (VI) above (or according to any of its subformulae herein) using an amid-chemistry as defined herein. If desired or necessary, the other terminus of the amino acid component (AA), e.g. the N- or C-terminus, may be used to couple another component. For this purpose, the other terminus of the amino acid component (AA) preferably comprises or is modified to comprise a further functionality, e.g. an alkyn-species (see above), which may be used to add the other component via e.g. click-chemistry. If the other component is bound via an acid-labile bond, the bond is preferably cleaved off in the endosome and the polymeric carrier according to formula (VI) above (or according to any of its subformulae herein) presents an amino acid component (AA) at its surface.

The amino acid component (AA) may also occur as a further component of the polymeric carrier according to formula (VI) above (or according to any of its subformulae herein) as defined above, e.g. as a linker between cationic components, e.g. as a linker between one cationic peptide and a further cationic peptide, as a linker between one cationic polymer and a further cationic polymer, as a linker between one cationic peptide and a cationic polymer, all preferably as defined herein, or as an additional component of the polymeric carrier according to formula (VI) above (or according to any of its subformulae herein), e.g. by binding the amino acid component (AA) to the polymeric carrier or a component thereof, e.g. via side chains, SH-moieties or via further moieties as defined herein, wherein the amino acid component (AA) is preferably accordingly modified.

According to a particularly preferred alternative, the amino acid component (AA), may be used to modify the polymeric carrier according to formula (VI) above (or according to any of its subformulae herein), particularly the content of cationic components in the polymeric carrier as defined above.

In this context it is preferable, that the content of cationic components in the polymeric carrier according to formula (VI) above (or according to any of its subformulae herein) is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 30% to 100%, more preferably in the range of about 50% to 100%, even preferably in the range of about 70% to 100%, e.g. 70, 80, 90 or 100%, or in a range formed by any two of the afore mentioned values, provided, that the content of all components in the polymeric carrier according to formula (VI) above (or according to any of its subformulae herein) is 100%.

In the context of the present invention, the amino acid component(s) (AA) (likewise in the context of (AA)ₓ or [(AA)ₓ]_{y} as defined above) may be independently selected from the following alternatives.

According to a first alternative, the amino acid component (AA) may be an aromatic amino acid component (AA). The incorporation of aromatic amino acids or sequences as amino aromatic acid component (AA) into the polymeric carrier molecule according to formula (VI) above (or according to any of its subformulae herein) of the present invention enables a different (second) binding of the polymeric carrier to the nucleic acid sequence due to interactions of the aromatic amino acids with the bases of the "cargo" of the polymeric carrier (e.g. the adjuvant component and/or the antigen as defined herein) in contrast to the binding thereof via cationic charged sequences of the polymeric carrier molecule to e.g. the phosphate backbone of a nucleic acid of the "cargo". This interaction may occur e.g. by intercalations or by minor or major groove binding. This kind of interaction is not prone to decompaction by anionic complexing partners (e.g. Heparin, hyaluronic acid, etc) which are found mainly in the extracellular matrix *in vivo* and is also less susceptible to salt effects.

For this purpose, the amino acids in the aromatic amino acid component (AA) may be selected from either the same or different aromatic amino acids e.g. selected from Trp, Tyr, or Phe. Alternatively, the amino acids (or the entire aromatic amino acid component (AA)) may be selected from following peptide combinations Trp-Tyr, Tyr-Trp, Trp-Trp, Tyr-Tyr, Trp-Tyr-Trp, Tyr-Trp-Tyr, Trp-Trp-Trp, Tyr-Tyr-Tyr, Trp-Tyr-Trp-Tyr, Tyr-Trp-Tyr-Trp, Trp-Trp-Trp-Trp, Phe-Tyr, Tyr-Phe, Phe-Phe, Phe-Tyr-Phe, Tyr-Phe-Tyr, Phe-Phe-Phe, Phe-Tyr-Phe-Tyr, Tyr-Phe-Tyr-Phe, Phe-Phe-Phe-Phe, Phe-Trp, Trp-Phe, Phe-Phe, Phe-Trp-Phe, Trp-Phe-Trp, Phe-Trp-Phe-Trp, Trp-Phe-Trp-Phe, or Tyr-Tyr-Tyr-Tyr, etc. (SEQ ID NOs: 269-296). Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the aromatic amino acid component (AA) may contain or may be flanked by a -SH containing moiety, which allows introducing this component via a disulfide bond as a (further) part of the polymeric carrier according to formula (VI) above (or according to any of its subformulae herein) as defined above, e.g. as a component of the polymeric carrier or as a linker. Such a -SH containing moiety may be any moiety as defined herein suitable to couple one component as defined herein to a further component as defined herein. As an example, such a -SH containing moiety may be a cysteine. Then, e.g. the aromatic amino acid component (AA) may be selected from e.g. peptide combinations Cys-Tyr-Cys, Cys-Trp-Cys, Cys-Trp-Tyr-Cys, Cys-Tyr-Trp-Cys, Cys-Trp-Trp-Cys, Cys-Tyr-Tyr-Cys, Cys-Trp-Tyr-Trp-Cys, Cys-Tyr-Trp-Tyr-Cys, Cys-Trp-Trp-Trp-Cys, Cys-Tyr-Tyr-Tyr-Cys, Cys-Trp-Tyr-Trp-Tyr-Cys, Cys-Tyr-Trp-Tyr-Trp-Cys, Cys-Trp-Trp-Trp-Trp-Cys, Cys-Tyr-Tyr-Tyr-Tyr-Cys, Cys-Phe-Cys, Cys-Phe-Tyr-Cys, Cys-Tyr-Phe-Cys, Cys-Phe-Phe-Cys, Cys-Tyr-Tyr-Cys, Cys-Phe-Tyr-Phe-Cys, Cys-Tyr-Phe-Tyr-Cys, Cys-Phe-Phe-Phe-Cys, Cys-Tyr-Tyr-Tyr-Cys, Cys-Phe-Tyr-Phe-Tyr-Cys, Cys-Tyr-Phe-Tyr-Phe-Cys, or Cys-Phe-Phe-Phe-Phe-Cys, Cys-Phe-Trp-Cys, Cys-Trp-Phe-Cys, Cys-Phe-Phe-Cys, Cys-Phe-Trp-Phe-Cys, Cys-Trp-Phe-Trp-Cys, Cys-Phe-Trp-Phe-Trp-Cys, Cys-Trp-Phe-Trp-Phe-Cys, etc. (SEQ ID NOs: 297-329). Each Cys above may also be replaced by any modified peptide or chemical compound carrying a free -SH-moiety as defined herein. Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the aromatic amino acid component (AA) may contain or represent at least one proline, which may serve as a structure breaker of longer sequences of Trp, Tyr and Phe in the aromatic amino acid component (AA), preferably two, three or more prolines.

According to a second alternative, the amino acid component (AA) may be a hydrophilic (and preferably non charged polar) amino acid component (AA). The incorporation of hydrophilic (and preferably non charged polar) amino acids or sequences as amino hydrophilic (and preferably non charged polar) acid component (AA) into the polymeric carrier according to formula (VI) above (or according to any of its subformulae herein) of the present invention enables a more flexible binding of the polymeric carrier to the "cargo" of the polymeric carrier, e.g. the adjuvant component and/or the antigen as defined above. This preferably leads to a more effective compaction of the components of the cargo and hence to a better protection against nucleases and unwanted decompaction. It also allows provision of a (long) polymeric carrier according to formula (VI) above (or according to any of its subformulae herein) which exhibits a reduced cationic, preferably a neutral or even a negative charge over the entire polymeric carrier and in this context to better adjusted binding properties, if desired or necessary.

For this purpose, the amino acids in the hydrophilic (and preferably non charged polar) amino acid component (AA) may be selected from either the same or different hydrophilic (and preferably non charged polar) amino acids e.g. selected from Thr, Ser, Asn or Gln. Alternatively, the amino acids (or the entire hydrophilic (and preferably non charged polar) amino acid component (AA) may be selected from following peptide combinations Ser-Thr, Thr-Ser, Ser-Ser, Thr-Thr, Ser-Thr-Ser, Thr-Ser-Thr, Ser-Ser-Ser, Thr-Thr-Thr, Ser-Thr-Ser-Thr, Thr-Ser-Thr-Ser, Ser-Ser-Ser-Ser, Thr-Thr-Thr-Thr, Gln-Asn, Asn-Gln, Gln-Gln, Asn-Asn, Gln-Asn-Gln, Asn-Gln-Asn, Gln-Gln-Gln, Asn-Asn-Asn, Gln-Asn-Gln-Asn, Asn-Gln-Asn-Gln, Gln-Gln-Gln-Gln, Asn-Asn-Asn-Asn, Ser-Asn, Asn-Ser, Ser-Ser, Asn-Asn, Ser-Asn-Ser, Asn-Ser-Asn, Ser-Ser-Ser, Asn-Asn-Asn, Ser-Asn-Ser-Asn, Asn-Ser-Asn-Ser, Ser-Ser-Ser-Ser, or Asn-Asn-Asn-Asn, etc. (SEQ ID NOs: 330-365). Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the hydrophilic (and preferably non-charged polar) amino acid component (AA) may contain or may be flanked by a -SH containing moiety, which allows introducing this component via a disulfide bond as a further part of generic formula (V) above, e.g. as a linker. Such a -SH containing moiety may be any moiety as defined herein suitable to couple one component as defined herein to a further component as defined herein. As an example, such a -SH containing moiety may be a cysteine. Then, e.g. the hydrophilic (and preferably non-charged polar) amino acid component (AA) may be selected from e.g. peptide combinations Cys-Thr-Cys, Cys-Ser-Cys, Cys-Ser-Thr-Cys, Cys-Thr-Ser-Cys, Cys-Ser-Ser-Cys, Cys-Thr-Thr-Cys, Cys-Ser-Thr-Ser-Cys, Cys-Thr-Ser-Thr-Cys, Cys-Ser-Ser-Ser-Cys, Cys-Thr-Thr-Thr-Cys, Cys-Ser-Thr-Ser-Thr-Cys, Cys-Thr-Ser-Thr-Ser-Cys, Cys-Ser-Ser-Ser-Ser-Cys, Cys-Thr-Thr-Thr-Thr-Cys, Cys-Asn-Cys, Cys-Gln-Cys, Cys-Gln-Asn-Cys, Cys-Asn-Gln-Cys, Cys-Gln-Gln-Cys, Cys-Asn-Asn-Cys, Cys-Gln-Asn-Gln-Cys, Cys-Asn-Gln-Asn-Cys, Cys-Gln-Gln-Gln-Cys, Cys-Asn-Asn-Asn-Cys, Cys-Gln-Asn-Gln-Asn-Cys, Cys-Asn-Gln-Asn-Gln-Cys, Cys-Gln-Gln-Gln-Gln-Cys, Cys-Asn-Asn-Asn-Asn-Cys, Cys-Asn-Cys, Cys-Ser-Cys, Cys-Ser-Asn-Cys, Cys-Asn-Ser-Cys, Cys-Ser-Ser-Cys, Cys-Asn-Asn-Cys, Cys-Ser-Asn-Ser-Cys, Cys-Asn-Ser-Asn-Cys, Cys-Ser-Ser-Ser-Cys, Cys-Asn-Asn-Asn-Cys, Cys-Ser-Asn-Ser-Asn-Cys, Cys-Asn-Ser-Asn-Ser-Cys, Cys-Ser-Ser-Ser-Ser-Cys, or Cys-Asn-Asn-Asn-Asn-Cys, etc. (SEQ ID NOs: 366-407). Each Cys above may also be replaced by any modified peptide or chemical compound carrying a free -SH-moiety as defined herein. Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the hydrophilic (and preferably non-charged polar) amino acid component (AA) may contain at least one proline, which may serve as a structure breaker of longer sequences of Ser, Thr and Asn in the hydrophilic (and preferably non charged polar) amino acid component (AA), preferably two, three or more prolines.

According to a third alternative, the amino acid component (AA) may be a lipophilic amino acid component (AA). The incorporation of lipophilic amino acids or sequences as lipophilic amino acid component (AA) into the polymeric carrier according to formula (VI) above (or according to any of its subformulae herein) of the present invention enables a stronger compaction of the "cargo" (e.g. the adjuvant component and/or the antigen as defined above) when forming a complex. This is particularly due to interactions of one or more components of the polymeric carrier, particularly of lipophilic sections of lipophilic amino acid component (AA) and the components of the cargo, e.g. a nucleic acid sequence encoding the antigen. This interaction will preferably add an additional stability to the complex between the polymeric carrier as a sort of outer shell and the complexed adjuvant component together with the antigen as defined herein. This stabilization may somehow be compared to a sort of non covalent crosslinking between different polymer strands. Especially in aqueous environment this interaction is typically strong and provides a significant effect.

For this purpose, the amino acids in the lipophilic amino acid component (AA) may be selected from either the same or different lipophilic amino acids, e.g., selected from Leu, Val, Ile, Ala, Met. Alternatively, the amino acid AA (or the entire lipophilic amino acid component (AA)) may be selected from following peptide combinations Leu-Val, Val-Leu, Leu-Leu, Val-Val, Leu-Val-Leu, Val-Leu-Val, Leu-Leu-Leu, Val-Val-Val, Leu-Val-Leu-Val, Val-Leu-Val-Leu, Leu-Leu-Leu-Leu, Val-Val-Val-Val, Ile-Ala, Ala-Ile, Ile-Ile, Ala-Ala, Ile-Ala-Ile, Ala-Ile-Ala, Ile-Ile-Ile, Ala-Ala-Ala, Ile-Ala-Ile-Ala, Ala-Ile-Ala-Ile, Ile-Ile-Ile-Ile, Ala-Ala-Ala-Ala, Met-Ala, Ala-Met, Met-Met, Ala-Ala, Met-Ala-Met, Ala-Met-Ala, Met-Met-Met, Ala-Ala-Ala, Met-Ala-Met-Ala, Ala-Met-Ala-Met, or Met-Met-Met-Met etc. (SEQ ID NOs: 408-442) Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the lipophilic amino acid component (AA) may contain or may be flanked by a -SH containing moiety, which allows introducing this component via a disulfide bond as a further part of the polymeric carrier according to formula (VI) above (or according to any of its subformulae herein) as defined above, e.g. as a linker. Such a -SH containing moiety may be any moiety as defined herein suitable to couple one component as defined herein to a further component as defined herein. As an example, such a -SH containing moiety may be a cysteine. Then, e.g. the lipophilic amino acid component (AA) may be selected from e.g. peptide combinations Cys-Val-Cys, Cys-Leu-Cys, Cys-Leu-Val-Cys, Cys-Val-Leu-Cys, Cys-Leu-Leu-Cys, Cys-Val-Val-Cys, Cys-Leu-Val-Leu-Cys, Cys-Val-Leu-Val-Cys, Cys-Leu-Leu-Leu-Cys, Cys-Val-Val-Val-Cys, Cys-Leu-Val-Leu-Val-Cys, Cys-Val-Leu-Val-Leu-Cys, Cys-Leu-Leu-Leu-Leu-Cys, Cys-Val-Val-Val-Val-Cys, Cys-Ala-Cys, Cys-Ile-Cys, Cys-Ile-Ala-Cys, Cys-Ala-Ile-Cys, Cys-Ile-Ile-Cys, Cys-Ala-Ala-Cys, Cys-Ile-Ala-Ile-Cys, Cys-Ala-Ile-Ala-Cys, Cys-Ile-Ile-Ile-Cys, Cys-Ala-Ala-Ala-Cys, Cys-Ile-Ala-Ile-Ala-Cys, Cys-Ala-Ile-Ala-Ile-Cys, Cys-Ile-Ile-Ile-Ile-Cys, or Cys-Ala-Ala-Ala-Ala-Cys, Cys-Met-Cys, Cys-Met-Ala-Cys, Cys-Ala-Met-Cys, Cys-Met-Met-Cys, Cys-Ala-Ala-Cys, Cys-Met-Ala-Met-Cys, Cys-Ala-Met-Ala-Cys, Cys-Met-Met-Met-Cys, Cys-Ala-Ala-Ala-Cys, Cys-Met-Ala-Met-Ala-Cys, Cys-Ala-Met-Ala-Met-Cys, Cys-Met-Met-Met-Met-Cys, or Cys-Ala-Ala-Ala-Ala-Cys, etc. Each Cys above may also be replaced by any modified peptide or chemical compound carrying a free -SH-moiety as defined herein. (SEQ ID NOs: 443-483) Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the lipophilic amino acid component (AA) may contain at least one proline, which may serve as a structure breaker of longer sequences of Leu, Val, Ile, Ala and Met in the lipophilic amino acid component (AA), preferably two, three or more prolines.

Finally, according to a fourth alternative, the amino acid component (AA) may be a weak basic amino acid component (AA). The incorporation of weak basic amino acids or sequences as weak basic amino acid component (AA) into the polymeric carrier according to formula (VI) above (or according to any of its subformulae herein) may serve as a proton sponge and facilitates endosomal escape (also called endosomal release) (proton sponge effect). Incorporation of such a weak basic amino acid component (AA)preferably enhances transfection efficiency.

For this purpose, the amino acids in the weak basic amino acid component (AA) may be selected from either the same or different weak amino acids e.g. selected from histidine or aspartate (aspartic acid). Alternatively, the weak basic amino acids (or the entire weak basic amino acid component (AA)) may be selected from following peptide combinations Asp-His, His-Asp, Asp-Asp, His-His, Asp-His-Asp, His-Asp-His, Asp-Asp-Asp, His-His-His, Asp-His-Asp-His, His-Asp-His-Asp, Asp-Asp-Asp-Asp, or His-His-His-His, etc. (SEQ ID NOs: 484-495) Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the weak basic amino acid component (AA) may contain or may be flanked by a -SH containing moiety, which allows introducing this component via a disulfide bond as a further part of generic formula (V) above, e.g. as a linker. Such a -SH containing moiety may be any moiety as defined herein suitable to couple one component as defined herein to a further component as defined herein as used in the polymeric carrier according to formula (VI) above (or according to any of its subformulae herein). As an example, such a -SH containing moiety may be a cysteine. Then, e.g. the weak basic amino acid component (AA) may be selected from e.g. peptide combinations Cys-His-Cys, Cys-Asp-Cys, Cys-Asp-His-Cys, Cys-His-Asp-Cys, Cys-Asp-Asp-Cys, Cys-His-His-Cys, Cys-Asp-His-Asp-Cys, Cys-His-Asp-His-Cys, Cys-Asp-Asp-Asp-Cys, Cys-His-His-His-Cys, Cys-Asp-His-Asp-His-Cys, Cys-His-Asp-His-Asp-Cys, Cys-Asp-Asp-Asp-Asp-Cys, or Cys-His-His-His-His-Cys, etc. Each Cys above may also be replaced by any modified peptide or chemical compound carrying a free -SH-moiety as defined herein. (SEQ ID NOs: 496-509) Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the weak basic amino acid component (AA) may contain at least one proline, which may serve as a structure breaker of longer sequences of histidine or aspartate (aspartic acid) in the weak basic amino acid component (AA), preferably two, three or more prolines.

The polymeric carrier according to formula (VI) above (or according to any of its subformulae herein) may also comprise as an additional component, preferably as a ligand L or as an amino acid component (AA) or (AA)ₓ a signal peptide or signal sequence, a localization signal or sequence, a nuclear localization signal or sequence (NLS), an antibody, a cell penetrating peptide (CPP), (e.g. TAT), etc. Likewise preferably such an additional component may occur as component L as defined herein. Alternatively, such an additional component may also be bound e.g. to a component L, P¹, P², P³, (AA) or (AA)ₓ as defined herein, e.g. to a side chain of any of components L, P¹, P², P³, (AA) or (AA)ₓ, preferably via a side chain of component P², or optionally as a linker between components L and P¹ or P³ and L. The binding to any of components L, P¹, P², or P³ may also be accomplished using an acid-labile bond, preferably via a side chain of any of components L, P¹, P², P³, which allows to detach or release the additional component at lower pH-values, e.g. at physiological pH-values as defined herein. Preferably such an additional component is bound to the polymeric carrier according to formula (VI) above (or according to any of its subformulae herein) or to another component of the polymeric carrier via a (reversible) disulfide bond. In the above context the signal peptide or signal sequence, a localization signal or sequence, a nuclear localization signal or sequence (NLS), an antibody, a cell penetrating peptide, (e.g. TAT), etc., additionally comprises at least one - SH-moiety. Preferably, a signal peptide, a localization signal or sequence or a nuclear localization signal or sequence (NLS), may be used to direct the complex formed by the polymeric carrier and its "cargo" (preferably the adjuvant component and/or the antigen as defined herein) to specific target cells (e.g. hepatocytes or antigen-presenting cells) and preferably allows a translocalization of the polymeric carrier or such a complex to a specific target, e.g. into the cell, into the nucleus, into the endosomal compartment, sequences for the mitochondrial matrix, localisation sequences for the plasma membrane, localisation sequences for the Golgi apparatus, the nucleus, the cytoplasm and the cytosceleton, etc. Such signal peptide, a localization signal or sequence or a nuclear localization signal may be used for the transport of any of the herein defined components of the complex, e.g. the polymeric carrier, its "cargo" (preferably the adjuvant component and/or the antigen as defined herein), preferably nucleic acid sequences, preferably an RNA or a DNA as defined and/or used herein, e.g. into the nucleus. Without being limited thereto, particular localization signals or sequences or a nuclear localization signals may include e.g. KDEL (SEQ ID NO: 510), DDEL (SEQ ID NO: 511), DEEL (SEQ ID NO: 512), QEDL (SEQ ID NO: 513), RDEL (SEQ ID NO: 514), and GQNLSTSN (SEQ ID NO: 515), nuclear localisation sequences, including PKKKRKV (SEQ ID NO: 516), PQKKIKS (SEQ ID NO: 517), QPKKP (SEQ ID NO: 518), RKKR (SEQ ID NO: 519), RKKRRQRRRAHQ (SEQ ID NO: 520), RQARRNRRRRWRERQR (SEQ ID NO: 521), MPLTRRRPAASQALAPPTP (SEQ ID NO: 522), GAALTILV (SEQ ID NO: 523), and GAALTLLG (SEQ ID NO: 524), localisation sequences for the endosomal compartment, including MDDQRDLISNNEQLP (SEQ ID NO: 525), localisation sequences for the mitochondrial matrix, including MLFNLRXXLNNAAFRHGHNFMVRNFRCGQPLX (SEQ ID NO: 526), localisation sequences for the plasma membrane: GCVCSSNP (SEQ ID NO: 527), GQTVTTPL (SEQ ID NO: 528), GQELSQHE (SEQ ID NO: 529), GNSPSYNP (SEQ ID NO: 530), GVSGSKGQ (SEQ ID NO: 531), GQTITTPL (SEQ ID NO: 532), GQTLTTPL (SEQ ID NO: 533), GQIFSRSA (SEQ ID NO: 534), GQIHGLSP (SEQ ID NO: 535), GARASVLS (SEQ ID NO: 536), and GCTLSAEE (SEQ ID NO: 537), localisation sequences for the endoplasmic reticulum and the nucleus, including GAQVSSQK (SEQ ID NO: 538), and GAQLSRNT (SEQ ID NO: 539), localisation sequences for the Golgi apparatus, the nucleus, the cytoplasm and the cytosceleton, including GNAAAAKK (SEQ ID NO: 540), localisation sequences for the cytoplasm and cytosceleton, including GNEASYPL (SEQ ID NO: 541), localisation sequences for the plasma membrane and cytosceleton, including GSSKSKPK (SEQ ID NO: 542), etc. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulins as defined herein, signal sequences of the invariant chain of immunoglobulins or antibodies as defined herein, signal sequences of Lamp1, Tapasin, Erp57, Calreticulin, Calnexin, and further membrane associated proteins or of proteins associated with the endoplasmic reticulum (ER) or the endosomal-lysosomal compartment. Particularly preferably, signal sequences of MHC class I molecule HLA-A*0201 may be used according to the present invention. Such an additional component may be bound e.g. to a cationic polymer or to any other component of the polymeric carrier as defined herein or may be added to the polymeric carrier when forming the complex with its "cargo" as defined above. Preferably this signal peptide, localization signal or sequence or nuclear localization signal or sequence (NLS), is bound to the polymeric carrier or to another component of the polymeric carrier via a (reversible) disulfide bond. For this purpose the (AA) component additionally comprises at least one -SH moiety as defined herein. The binding to any of components of the polymeric carrier may also be accomplished using an acid-labile bond, preferably via a side chain of any of components of the polymeric carrier, which allows to detach or release the additional component at lower pH-values, e.g. at physiological pH-values as defined herein.

Additionally, the polymeric carrier according to formula (VI) above (or according to any of its subformulae herein), may comprise further functional peptides or proteins preferably as ligand or amino acid component (AA) or (AA)ₓ, which may modulate the functionality of the polymeric carrier accordingly, preferably so called cell penetrating peptides (CPPs) or cationic peptides for transportation as defined herein. Likewise, such an additional component may occur as component L or (AA) or (AA)ₓ as defined herein. Alternatively, such an additional component may also be bound to a component L, P¹, P², P³, (AA) or (AA)ₓ as defined herein, e.g. to a side chain of any of components L, P¹, P², P³, (AA) or (AA)ₓ preferably via a side chain of component P², or optionally as a linker between components L and P¹ or P³ and L. The binding to any of components L, P¹, P², P³, (AA) or (AA)ₓ may also be accomplished using an acid-labile bond, preferably via a side chain of any of components L, P¹, P², P³, (AA) or (AA)ₓ which allows to detach or release the additional component at lower pH-values, e.g. at physiological pH-values as defined herein. In this context it is particularly preferred that this additional component occurs as ligand L or as amino acid component (AA) or (AA)ₓ of the repetitive component [S-P²-S]ₙ of formula (VI). Such functional peptides or proteins preferably comprise any peptides or proteins as defined herein, e.g. so called cell penetrating peptides (CPPs) or cationic peptides for transportation. Particularly preferred are CPPs, which induce a pH-mediated conformational change in the endosome and lead to an improved release of the complex formed by the polymeric carrier according to formula (VI) above (or according to any of its subformulae herein) or its components from the endosome by insertion into the lipid layer of the liposome. These cell penetrating peptides (CPPs) or cationic peptides for transportation, may include, without being limited thereto protamine, nucleoline, spermine or spermidine, oligo- or poly-L-lysine (PLL), basic polypeptides, oligo or poly-arginine, cell penetrating peptides (CPPs), chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia*)*,* pAntp, plsl, etc., antimicrobial-derived CPPs e.g. Buforin-2, Bac715-24, SynB, SynB(1), pVEC, hCT-derived peptides, SAP, MAP, KALA, PpTG20, Loligomere, FGF, Lactoferrin, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc. Such functional peptides or proteins may also comprise any peptide or protein which can execute any favourable function in the cell. Particularly preferred are peptides or proteins selected from therapeutically active proteins or peptides, from antigens as defined herein, e.g. tumour antigens, pathogenic antigens (animal antigens, viral antigens, protozoan antigens, bacterial antigens, allergic antigens), autoimmune antigens, or further antigens, from allergens, from antibodies, from immunostimulatory proteins or peptides, from antigen-specific T-cell receptors, or from any other protein or peptide suitable for a specific (therapeutic) application. Particularly preferred are peptide epitopes from antigens as defined herein. Such functional peptides or proteins may also be added to the polymeric carrier when forming the complex with its "cargo" as defined above.

The polymeric carrier according to formula (VI) above (or according to any of its subformulae herein) may comprise at least one of the above mentioned cationic or polycationic peptides, proteins or polymers or further components, wherein any of the above defined alternatives and components may be combined with each other, and may be formed by polymerizing same in a polymerization condensation reaction via their -SH-moieties.

The polymeric carrier according to formula (VI) may comprise at least one of the above mentioned cationic or polycationic peptides, proteins or polymers or further components, e.g. (AA) or (AA)ₓ, wherein any of the above alternatives may be combined with each other, and may be formed by polymerizing same in a polymerization condensation reaction via their -SH-moieties.

The polymeric carrier according to formula (VI), which may be used for combined packaging the adjuvant component and the antigen of the inventive vaccine composition as defined above, may be prepared according to the following method. In this context, the present invention also provides and utilizes a polymeric carrier according to formula (VI) (as a product by process) obtained or obtainable by such method steps. The method may be furthermore be extended to obtain the inventive vaccine composition . The method preferably comprises following steps:
a) providing at least one cationic or polycationic protein or peptide as component P² as defined herein and/or at least one cationic or polycationic polymer as component P² as defined herein, and optionally at least one further component (e.g. (AA), (AA)ₓ, [(AAₓ)]_{z}, etc)., preferably in the ratios indicated herein, mixing these components, preferably in a basic milieu as defined herein, preferably in the presence of oxygen or a further starter as defined herein which leads to mild oxidation conditions, preferably at a pH, at a temperature and at time as defined herein, and thereby condensing and thus polymerizing these components with each other via disulfide bonds (in a polymerization condensation or polycondensation) to obtain a repetitive component H-[S-P²-S]ₙ-H or H{[S-P²-S]ₐ[S-(AA)ₓ-S]_{b}}H, etc.;
b) providing a hydrophilic polymer P¹ and/or P³ as defined herein, optionally modified with a ligand L and/or an amino acid component (AA) or (AA)ₓ as defined herein;
c) mixing the hydrophilic polymer P¹ and/or P³ provided according to step b) with the repetitive component H-[S-P²-S]ₙ-H or H{[S-P²-S]ₐ[S-(AA)ₓ-S]_{b}}H, etc. obtained according to step a), typically in a ratio of about 2 : 1, (and thereby typically terminating the polymerization condensation or polycondensation reaction) and obtaining the polymeric carrier, preferably according to formula (VI) as defined herein or according to any subformula thereof as defined herein;
d) optionally purifying the polymeric carrier obtained or obtainable according to step c), preferably using a method as defined herein;
e) optionally adding the polymeric carrier obtained or obtainable according to step c) or d) to a mixture of an adjuvant component and an antigen as defined herein for the inventive vaccine composition , preferably in the herein mentioned ratios, and thereby packaging the (complex of the) adjuvant component and the antigen, optionally in the presence of further components as defined herein, e.g. enhancer peptides, CPPs, etc. as defined herein, with the polymeric carrier to obtain the inventive vaccine composition as defined herein.

The method of preparing the polymeric carrier according to formula (I) as defined herein represents a multi-step condensation polymerization or polycondensation reaction via -SH moieties of the educts, e.g. component(s) P² as defined herein, further components P¹ and/or P³ and optionally further components (AA) or (AA)ₓ. The condensation polymerization or polycondensation reaction preferably leads to the polymeric carrier as a condensation polymer, wherein the single components are linked by disulfide bonds. This condensation polymerization leads to the polymeric carrier according to formula (VI) preparing in a first step a) of the condensation reaction the inventive repetitive component H-[S-P²-S]ₙ-H or a variant thereof as a sort of a "core" or "central motif" of the polymeric carrier. In a second step b) components P¹ and/or P³ are provided, which allow to terminate or to somehow "coat" the inventive repetitive component H-[S-P²-S]ₙ-H or a variant thereof in a third step c) by adding components P¹ and/or P³ as defined herein (optionally modified with a ligand L and/or an amino acid component (AA) or (AA)ₓ as defined herein) to the condensation product obtained according to step a). In subsequent step d), this product may be purified and further in a further step e) the product of step c) or d) may be added to a mixture of an adjuvant component and an antigen as defined herein for the inventive vaccine composition, preferably in the herein mentioned ratios, to allow packaging the adjuvant component and the antigen together with the polymeric carrier to obtain the inventive vaccine composition as defined herein

It is important to understand that the method is based on an equilibrity reaction under mild oxidation conditions in steps a), b) and c), which, upon balancing the equilibrity state, allows to obtain the polymeric carrier according to formula (VI) above or according to any of its subformulae comprising the selected components in the desired molar ratios. For this purpose, long reaction times are envisaged to achieve an equilibrity state in steps a), b) and c). If for example a condensation polymerization is to be carried out using a molar ratio of 5 components P² in step a), the equilibrium is surprisingly settled at a polymer length of about 5 after sufficient time, preferably e.g. > 12 hours. However, due to the equilibrium the polymer length (as defined by n) is not fixed at a specific value, e.g. 5, but may vary accordingly within the equilibrity reaction. Accordingly, about 5 may mean about 4 to 6, or even about 3 to 7. Preferably, the polymer length and thus the integer n (and thus a, b and a + b) varies within a limit of about ±1, or ±2.

As defined herein in a step a) of the method of preparing the polymeric carrier according to formula (VI) at least one cationic or polycationic protein or peptide as component P² as defined herein and/or at least one cationic or polycationic polymer as component P² as defined herein are provided, preferably in the ratios indicated above by formula (VI). These components are mixed, preferably in a basic milieu as defined herein, preferably in the presence of oxygen or a further starter as defined herein which leads to mild oxidation conditions, preferably at a pH, and at a temperature and at a time as defined herein, and thereby condensing and thus polymerizing these components with each other via disulfide bonds (in a polymerization condensation or polycondensation) to obtain a repetitive component H-[S-P²-S]ₙ-H.

According to an alternative, in step a) of the method of preparing the polymeric carrier at least one cationic or polycationic protein or peptide and/or at least one cationic or polycationic polymer are provided and used as component(s) P² as defined herein, and additionally at least one amino acid component (AA) or (AA)ₓ is provided as defined herein, and components P² and (AA) or (AA)ₓ are used for a polymerization condensation or polycondensation according to step a). Preferably, the components are all provided in the ratios indicated above by formula (VIa), mixed, preferably in a basic milieu as defined herein, preferably in the presence of oxygen or a further starter as defined herein which leads to mild oxidation conditions, preferably at a pH, at a temperature and at time as defined herein. Upon mixing and starting the reaction, the components are condensed and thus polymerized with each other via disulfide bonds (in a polymerization condensation or polycondensation) to obtain a repetitive component H-{[S-P²-S]ₐ[S-(AA)ₓ-S]_{b}}-H.

In both of the above alternatives, different component(s) P², particularly different peptides and/or different polymers as component P², may be selected in the condensation polymerization as indicated above. In this context, the selection of different component(s) P² is typically dependent upon the desired properties of the final polymeric carrier and the desired cationic strength of the final polymeric carrier or its central core motif. Accordingly, the repetitive component [S-P²-S]ₙ, may furthermore be "diluted" or modified in the above alternative of step a) e.g. by introducing an amino acid component (AA)ₓ as defined herein, preferably in the above defined ratios. Thereby, a modified central core motif {[S-P²-S]ₐ[S-(AA)ₓ-S]_{b}} may be obtained, wherein the cationic character of (unmodified) repetitive component [S-P²-S]ₙ typically remains in the limitations as defined herein. The properties of the final polymeric carrier may thus be adjusted as desired with properties of components (AA)ₓ by inserting amino acid component (AA)ₓ as defined herein in steps a), b) and/or c).

In all cases, step a) is based on an equilibrity reaction under mild oxidation conditions which, upon balancing the equilibrity state, allows to obtain either inventive repetitive component H-[S-P²-S]ₙ-H or inventive repetitive component H-{[S-P²-S]ₐ[S-(AA)ₓ-S]_{b})-H in the desired molar ratios. In the equilibrity state, n is preferably 1, 2, 3, 4, or 5 to 10, more preferably 4 to 9, and a + b = n is as defined above, preferably a + b = 1, 2, 3, 4, or 5 to 10, more preferably 4 to 9. For this purpose, long reaction times are envisaged to achieve an equilibrity state in step a), most preferably e.g. > 12 hours. Accordingly, step a) of the method of preparing a polymeric carrier typically requires at least about 5 hours, even more preferably at least about 7.5 hours or even 10 hours, most preferably at least about 12 hours, e.g. a reaction time of about 12 to 60 hours, a reaction time of about 12 to 48 hours, a reaction time of about 12 to 36 hours, or a reaction time of about 12 to 24 hours, etc, wherein the lower border of 12 hours of the latter ranges may also be adjusted to 10, 7.5, or even 5 hours. Advantageously, the equilibrity state can be balanced using the method.

In step a), the at least one cationic or polycationic protein or peptide as component P² as defined herein and/or at least one cationic or polycationic polymer as component P² as defined herein, and optionally at least one amino acid component (AA) or (AA)ₓ as defined herein, are preferably contained in a basic milieu in the step a) of the method of preparing the polymeric carrier according to formula (VI) (or any of its subformulae, e.g. (Via)). Such a basic milieu typically exhibits a pH range of about 6 to about 12, preferably a pH range of about 7 to about 10, more preferably a pH range of about 8 to about 10, e.g. about 8, 8.5, 9, 9.5, or 10 or any range selected from any two of these or the aforementioned values.

Furthermore, the temperature of the solution in step a) is preferably in a range of about 5°C to about 60°C, more preferably in a range of about 15°C to about 40°C, even more preferably in a range of about 20°C to about 30°C, and most preferably in a range of about 20°C to about 25°C, e.g. about 25°C.

In step a) of the method of preparing the polymeric carrier according to formula (VI) (or any of its subformulae, e.g. (VIa)) as defined herein buffers may be used as suitable. Preferred buffers may comprise, but are not limited to carbonate buffers, borate buffers, Bicine buffer, CHES buffer, CAPS buffer, Ethanolamine containing buffers, HEPES, MOPS buffer, Phosphate buffer, PIPES buffer, Tris buffer, Tricine buffer, TAPS buffer, and/or TES buffer as buffering agents. Particularly preferred is a carbonate buffer.

Upon mixing the components, preferably in the presence of oxygen, preferably in the presence of a basic milieu as defined herein, the condensation polymerization or polycondensation reaction is started. For this purpose, the mixture in step a) is preferably exposed to oxygen or may be started using a further starter, e.g. a catalytic amount of an oxidizing agent, e.g. DMSO, etc. To determine the desired polymer chain length the condensation reaction has to be carried out under mild oxidation conditions, preferably in the presence of less than 30% DMSO, more preferably in the presence of less than 20% DMSO and most preferably in the presence of less than 10% DMSO. Upon start of the condensation polymerization or polycondensation reaction the at least one cationic or polycationic protein or peptide and/or at least one cationic or polycationic polymer as component P² and optionally at least one amino acid component (AA) or (AA)ₓ as defined herein, are condensed and thus polymerized with each other via disulfide bonds (polymerization condensation or polycondensation). In this reaction step a) preferably linear polymers are created using monomers with at least two reactive -SH moieties, i.e. at least one cationic or polycationic protein or peptide and/or at least one cationic or polycationic polymer as component P² as defined herein, each component P² exhibiting at least two free -SH-moieties as defined herein, e.g. at their terminal ends. However, components P² with more than two free -SH-moieties may be used, which may lead to branched polymers. According to one other specific aspect, the condensation product obtained according to step a) may be modified (e.g. in a step a1)) by adding an amino acid component (AA) or (AA)ₓ or a mixed repetitive amino acid component [(AA)ₓ]_{z} as defined herein e.g. to the terminal ends of the condensation product of step a). This may occur via any functionality as defined herein, e.g. a -SH moiety or any further functionality described herein, preferably a -SH moiety. For this purpose, amino acid component (AA) or (AA)ₓ or a mixed repetitive amino acid component [(AA)ₓ]_{z} may be provided with two (or even more) -SH-moieties, e.g. in a form represented by formulae "H(S-AA-S)ₓH" or "H[S-(AA)ₓ-S]_{z}H". Then, a polycondensation raction may be carried out with the products of step a), i.e. inventive repetitive component H-[S-P²-S]ₙ-H or inventive repetitive component H-{[S-P²-S]ₐ[S-(AA)ₓ-S]_{b}}-H, leading to intermediate components

H(S-AA-S)ₓ-[S-P²-S]ₙ-(S-AA-S)ₓH,

or

H[S-(AA)ₓ-S]_{z}-[S-P²-S]ₙ-[S-(AA)ₓ-S]_{z}H,

or

H(S-AA-S)ₓ-{[S-P²⁻S]ₐ[S-(AA)ₓ-S]_{b}}-(S-AA-S)ₓH,

or

H[S-(AA)ₓ-S]_{z}-{[S-P²-S]ₐ[S-(AA)ₓ-S]_{b}}-[S-(AA)ₓ-S]_{z}H.

Any single or all of these intermediate components or the inventive repetitive component

H-[S-P²-S]ₙ-H

or the inventive repetitive component

H-{[S-P²-S]ₐ[S-(AA)ₓ-S]_{b}}-H

obtained according to step a), may be used to be coupled to the polymers provided in step b) of the method.

According to a second step b) of the method of preparing the polymeric carrier according to formula (VI) as defined herein (or according to any of its subformulae), a hydrophilic polymer P¹ and/or P³ as defined herein is added to the condensation product obtained according to step a). In this context, the hydrophilic polymers P¹ and/or P³ as defined herein, preferably exhibit at least one -SH-moiety, more preferably only one -SH-moiety per hydrophilic polymers P¹ and/or P³ as defined herein, thereby terminally stopping the polymerization condensation or polycondensation according to step a) in step c). Hydrophilic polymers P¹ and/or P³ as defined herein may be the same or different, wherein these polymers may be selected according to the desired properties. Typically, hydrophilic polymers P¹ and/or P³ as a whole may be added to the condensation product obtained according to step a) in a ratio of about 2 : 1 hydrophilic polymer P¹ and/or P³ : condensation product obtained according to step a). According to one alternative, the hydrophilic polymer(s) P¹ and/or P³ additionally may be modified with either a component L (ligand) as defined herein or a component (AA) or (AA)ₓ or [(AA)ₓ]_{z} as defined herein or both a component L (ligand) as defined herein and a component (AA) or (AA)ₓ or [(AA)ₓ]_{z} as defined herein.

According to a first example, a ligand is attached to component(s) P¹ and/or P³ as component L prior to providing component(s) P¹ and/or P³ in step b) via any functionality as defined herein, e.g. a -SH moiety. This ligand is preferably attached to the hydrophilic polymer(s) P¹ and/or P³ at one terminus of these polymers. If the attachment is carried out via -SH bonds, the hydrophilic polymer(s) P¹ and/or P³ are preferably provided with two (or even more) -SH-moieties., e.g. in a form represented by formulae HS-P¹-SH or HS-P³-SH. Ligand L preferably carries only one -SH moiety. In this case, one -SH moiety of hydrophilic polymer(s) P¹ and/or P³ is preferably protected in a first step using a protecting group as known in the art. Then, the hydrophilic polymer(s) P¹ and/or P³ may be bound to a component L to form a first disulfide bond via the non-protected -SH moiety. The protected -SH-moiety of hydrophilic polymer(s) P¹ and/or P³ is then typically deprotected for further reactions. This preferably leads to following intermediate components

L-S-S-P¹-SH,

or

HS-P³-S-S-L.

Alternatively, the above intermediate components may be provided similarly without the necessity of blocking the free -SH-moieties. These intermediate components may be used in step c) to be coupled with the condensation products obtained according to step a) above, e.g. to form a second disulfide bond with inventive repetitive component H-[S-P²-S]ₙ-H or inventive mixed repetitive component H-{[S-P²-S]ₐ[S-(AA)ₓ-S]_{b}}-H obtained according to step a) or any of its modifications, e.g. according to step a1). If the attachment is carried out via other moieties, any of the reactions as defined herein may be used accordingly.

According to a further example, an amino acid component (AA) or (AA)ₓ or a mixed repetitive amino acid component [(AA)ₓ]_{z} as defined herein may be attached to component(s) P¹ and/or P³ prior to providing component(s) P¹ and/or P³ via any functionality as defined herein, e.g. a -SH moiety. The amino acid component (AA) or (AA)ₓ or a mixed repetitive amino acid component [(AA)ₓ]_{z} may be attached to the hydrophilic polymer(s) P¹ and/or P³ at any position within these polymers or at one or both termini of these polymers. In one specific case, the amino acid component (AA)ₓ or a mixed repetitive amino acid component [(AA)ₓ]_{z} may be provided as a linker between component(s) P¹ and/or P³ and the condensation product obtained according to step a) above or as a linker between component(s) P¹ and/or P³ and a further component, e.g. a linker L, or according to another alternative, as a terminating component at one terminus of component(s) P¹ and/or P³. In any of these cases, the attachment preferably may carried out via -SH bonds, wherein the hydrophilic polymer(s) P¹ and/or P³ are preferably provided with two (or even more) -SH-moieties., e.g. in a form represented by formula "HS-P¹-SH" or "HS-P³-SH", wherein preferably one of these to -SH moieties is protected, e.g. in a form represented by formula "HS-P¹-S-protecting group" or "protecting group-S-P³-SH". Furthermore, amino acid component (AA) or (AA)ₓ or a mixed repetitive amino acid component [(AA)ₓ]_{z} are also preferably provided with two (or even more) -SH-moieties, e.g. in a form represented by formulae "H(S-AA-S)ₓ-H" or "H[S-(AA)ₓ-S]_{z}H", wherein preferably one of these to -SH moieties is protected, e.g. in a form represented by formulae "protecting group-(S-AA-S)ₓ-SH" or "H[S-(AA)ₓ-S]_{z}-protecting group". Then, a polycondensation reaction may be carried out with polymers "HS-P¹-S-protecting group" or "protecting group-S-P³-SH" leading to intermediate components
"protecting group-S-P¹-S-(S-AA-S)ₓ-S-protecting group",
"protecting group-(S-AA-S)ₓ-S-S-P³-S-protecting group",
"protecting group-S-P¹-S-[S-(AA)ₓ-S]_{z}-protecting group", or
"protecting group-[S-(AA)ₓ-S]_{z}-S-P³-S-protecting group".

Any single or all of these intermediate components may then be used in step c) of the method to be coupled to the condensation product according to step a).

For this purpose, at least one or both protecting groups (selected upon the desired direction of the component in the final polymeric carrier) of each intermediate compound may be deprotected prior to providing them in step b), preferably leading to following intermediate components

"HS-P¹-S-(S-AA-S)ₓ-SH",

"H(S-AA-S)ₓ-S-S-P³-SH",

"HS-P¹-S-[S-(AA)ₓ-S]_{z}H",

or

"H[S-(AA)ₓ-S]_{z}-S-P³-SH",

Alternatively, the above intermediate components may be provided similarly without the necessity of blocking the free -SH-moieties. Any single or all of these intermediate components may then be provided in step b) of the method to be coupled to the condensation product according to step a).

If any of the afore mentioned intermediate components is provided in step b), this condensation reaction may be terminated in a step c) by adding a linker component as defined herein with one -SH-moiety (e.g. L-SH) or any further component with a single -SH moiety, e.g. as defined herein. In one further specific case, the amino acid component (AA) or (AA)ₓ or a mixed repetitive amino acid component [(AA)ₓ]_{z} may be used as a terminal component at one terminus of component(s) P¹ and/or P³ without adding a further component to the amino acid component (AA) or (AA)ₓ or a mixed repetitive amino acid component [(AA)ₓ]_{z}.

According to a further example, an amino acid component (AA) or (AA)ₓ or a mixed repetitive amino acid component [(AA)ₓ]_{z} as defined herein may be attached to component(s) P¹ and/or P³ prior to step c), wherein component(s) P¹ and/or P³ have been already modified with a linker. For this purpose, component(s) P¹ and/or P³ preferably carry (at least) two -SH moieties as defined herein, wherein a polycondensation is carried out with a linker, carrying e.g. one -SH moiety. This reaction may be carried out by using protecting groups as defined herein, or, preferably, without protecting groups. Alternatively, any further functionality as defined herein except -SH moieties may be used for coupling. Then, the second -SH moiety of component(s) P¹ and/or P³ may be used to couple an amino acid component (AA) or (AA)ₓ or a mixed repetitive amino acid component [(AA)ₓ]_{z} as defined herein via -SH-moieties, e.g. in a form represented by formulae "H(S-AA-S)ₓ-H" or "H[S-(AA)ₓ-S]_{z}H". The reaction preferably leads to following intermediate compounds

"L-S-S-P¹-S-(S-AA-S)ₓ-SH",

"L-S-S-P¹-S-[S-(AA)ₓ-S]_{z}H",

or

"HS-(S-AA-S)ₓ-S-S-P³-S-S-L",

or

"HS-[S-(AA)ₓ-S]_{z}-S-P³-S-S-L";

or, if component L has been linked without a disulfide bond to following intermediate products

"L-P¹-S-(S-AA-S)ₓ-SH",

"L-P¹-S-[S-(AA)ₓ-S]_{z}H",

or

"HS-(S-AA-S)ₓ-S-S-P³-L",

or

"HS-[S-(AA)ₓ-S]_{z}S-P³-L";

In step c) the hydrophilic polymers P¹ and/or P³ (or any of the intermediate components provided according to step b)) as defined herein, are provided and mixed with the repetitive component H-[S-P²-S]ₙ-H, with the mixed repetitive component H-{[S-P²-S]ₐ[S-(AA)ₓ-S]_{b}}-H, or any of the intermediate components obtained according to step a), typically in a ratio of about 2 : 1. The reaction is typically started and carried out under conditions already described above for step a) (pH, temperature, reaction time, buffers, etc). Step c) allows to terminate the polymerization condensation or polycondensation reaction and to obtain the polymeric carrier according to formula (VI) or (VIa) or according to any of subformulae thereof as defined herein, preferably the polymeric carrier according to formula (VI)

L-P¹-S-[S-P²-S]ₙ-S-P³-L

or according to formula (VIa)

L-P¹-S-{[S-P²-S]ₐ[S-(AA)ₓ-S]b}-S-P³-L.

According to a further step d) of the method of preparing the polymeric carrier according to formula (VI) or (Via) as defined herein, or according to any of subformulae thereof as defined herein, the polymeric carrier obtained according to step c) is optionally purified. Purification may occur by using chromatographic methods, such as HPLC, FPLC, GPS, dialysis, etc.

According to optional step e) of the method of preparing the polymeric carrier according to formula (VI) or (VIa) as defined herein, or according to any of subformulae thereof as defined herein, the polymeric carrier, preferably as obtained according to step c) or d) of the method, may be added to a mixture of an adjuvant component and an antigen as defined herein for the inventive vaccine composition . The mixture of an adjuvant component and an antigen as defined herein are preferably present in the herein mentioned ratios Furthermore, such a mixture of an adjuvant component and an antigen as defined herein for the inventive composition may also comprise further functional peptides or proteins as defined herein, e.g. so called cell penetrating peptides (CPPs) or cationic peptides for transportation. Such further functional peptides or proteins as defined herein, however, are preferably added to the mixture of an adjuvant component and an antigen as defined herein for the inventive composition as a last component. Addition of such components, preferably observes the N/P ratio as defined herein for the entire inventive vaccine composition .

In the above context, such further functional peptides or proteins as defined herein comprise e.g. so called cell penetrating peptides (CPPs) or cationic peptides for transportation. particularly CPPs, which induce a pH-mediated conformational change in the endosome and lead to an improved release of the polymeric carrier (in complex with a nucleic acid) from the endosome by insertion into the lipid layer of the liposome. Such called cell penetrating peptides (CPPs) or cationic peptides for transportation, may include, without being limited thereto protamine, nucleoline, spermine or spermidine, poly-L-lysine (PLL), basic polypeptides, poly-arginine, cell penetrating peptides (CPPs), chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia*)*,* pAntp, plsl, etc., antimicrobial-derived CPPs e.g. Buforin-2, Bac715-24, SynB, SynB(1), pVEC, hCT-derived peptides, SAP, MAP, KALA, PpTG20, Proline-rich peptides, Loligomers, Arginine-rich peptides, Calcitonin-peptides, FGF, Lactoferrin, , poly-L-Lysine, poly-Arginine, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc.

Adding the polymeric carrier, preferably as obtained according to step c) or d) of the method, to a mixture of an adjuvant component and an antigen as defined herein for the inventive vaccine composition , optionally in the presence of further components as defined herein, e.g. enhancer peptides, CPPs, etc. as defined herein, as defined above, allows packaging the adjuvant component and the antigen with the polymeric carrier and preferably yields the inventive vaccine composition as defined herein.

The ratio of the components of the entire inventive vaccine composition, i.e. a) an adjuvant component comprising or consisting of at least one immunostimulatory nucleic acid sequence, complexed with a cationic or polycationic peptide or a protein as a complexing agent; b) a nucleic acid sequence encoding an antigen, wherein the nucleic acid is an RNA; and c) a polymeric carrier molecule for combined packaging the adjuvant component and the RNA encoding the antigen, preferably if the complexing agent is present in a protein or peptide form, preferably if component P² of the carrier molecule is present in a protein or peptide form and/or if the antigen is encoded by a nucleic acid, and optionally in the presence of further components as defined above, may also be calculated on the basis of the nitrogen/phosphate ratio (N/P-ratio) of all these components. In the context of the present invention, an N/P-ratio is preferably in the range of about 0.01-2, 0.1-2 or 0.1-1.5 regarding the ratio of nucleic acids: cationic or polycationic peptide in the complex, and most preferably in the range of about 0.1-1. Such an N/P ratio is preferably designed to provide good transfection properties *in vivo* and transport into and through cell membranes.

In this context, the components of such an inventive vaccine composition may be contained in a molar ratio of e.g.
the (preferably entire) nucleic acid as defined herein, preferably isRNA and/or mRNA as defined herein typically in a relative amount of about 1 mol,
the complexing agent typically in a relative amount of about 0 or 1 mol to about 250 mol, preferably about 1 to 250 mol, more preferably about 1 to about 100 mol, most preferably about 10 to about 40 mol, e.g. about 25 mol,
the carrier molecule as defined herein typically in a relative amount of about 5 mol to about 500 mol, preferably about 5 mol to about 250 mol, more preferably about 5 mol to about 100 mol, even more preferably about 20 mol to about 80 mol, and optionally any further component as defined herein, preferably an enhancer peptide or a CPP as defined herein, e.g. KALA, in a relative amount of about 0 or 1 mol to about 50 mol, preferably about 0 or 1 mol to about 25 mol, more preferably about 0 or 1 mol to about 10 mol, e.g. about 5 mol. Further components may also be contained, if necessary.

### Particularly preferred is following ratio

| Component | mRNA | Carrier | Complexation reagent | Enhancer Peptide (e.g. KALA) |
|---|---|---|---|---|
| Ratio (mol) | 1 | about 5-500, preferably about 50 | about 0-250, preferably about 25 | about 0-50, preferably about 5 |

The method of preparing the polymeric carrier according to formula (VI) or (VIa) or according to any of subformulae thereof as defined herein and further the entire inventive vaccine composition is particularly suitable to adapt the chemical properties of the desired polymeric carrier and of the entire inventive vaccine composition due to specific selection of its components P², L, (AA), (AA)ₓ, or [(AA)ₓ]_{z} thereby avoiding agglomeration and toxicity *in vivo.*

Furthermore, a skilled person would not have expected to obtain such a polymeric carrier according to formula (VI) or (VIa) or according to any of subformulae thereof as defined herein and thus would not have suggested preparing the inventive vaccine composition using the above method. A skilled person would always have expected that the polymeric carrier according to formula (VI) or (VIa) or according to any of subformulae thereof, e.g. as obtained according to the method, due to general rules of equilibrity reactions leads to a monomeric content of component P², flanked by monomeric components P¹ and/or P³, wherein the linkages are formed by disulfide bonds. In contrast, the present inventors were surprisingly able to show that when using a specific ratio of polymers and method steps as defined herein, particularly mild oxidation conditions during the polymerization reaction, the polymerization condensation can be directed to specifically obtain a desired distribution of polymers and a desired average length and the desired polymeric carrier according to generic formula (VI) or (VIa) or according to any of subformulae thereof as defined herein without the necessity of blocking the free -SH-moieties. This was not expected by a skilled person.

In a further preferred aspect of the present invention the inventive vaccine composition may be formulated with or comprises a pharmaceutically acceptable carrier and/or vehicle. In the context of the present invention, a pharmaceutically acceptable carrier typically includes the liquid or non-liquid basis of the inventive vaccine composition. If such a composition is provided in liquid form, the carrier typically will be pyrogen-free water, isotonic saline or buffered (aqueous) solutions, e.g. phosphate, citrate, etc. or further buffered solutions. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis if the at least one antigen comprised in the inventive vaccine composition is provided as nucleic acid sequence.

However, one or more compatible solid or liquid fillers or diluents or encapsulating compounds, which are suitable for administration to a patient to be treated, may be used as well for the inventive vaccine composition. The term "compatible" as used here means that these constituents of the inventive vaccine composition are capable of being mixed in such a manner that no interaction occurs which would substantially reduce the effectiveness of the inventive vaccine composition under typical use conditions.

According to a specific aspect, the inventive vaccine composition may comprise an adjuvant (additional to the adjuvant component of the inventive vaccine composition). In this context, an adjuvant may be understood as any compound, which is suitable to initiate or increase an immune response of the innate immune system, i.e. a non-specific immune response. With other words, when administered, the vaccine preferably also elicits an innate immune response due to the adjuvant, optionally contained therein. Preferably, such an adjuvant may be selected from an adjuvant known to a skilled person and suitable for the present case, i.e. supporting the induction of an innate immune response in a mammal.

Preferably, the adjuvant may be selected from the group consisting of, without being limited thereto, cationic or polycationic compounds as defined above, from chitosan, TDM, MDP, muramyl dipeptide, pluronics, alum solution, aluminium hydroxide, ADJUMER™ (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINE™ (propanediamine); BAY R1005™ ((N-(2-deoxy-2-L-leucylaminob- D-glucopyranosyl)-N-octadecyl-dodecanoyl-amide hydroacetate); CALCITRIOL™ (1-alpha,25-dihydroxy-vitamin D3); calcium phosphate gel; CAP™ (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D35 glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L47 alanyl-D-isoglutamine); imiquimod (1-(2-methypropyl)-1H-imidazo[4,5-c]quinoline-4-amine); ImmTher™ (N-acetylglucosaminyl-N-acetylmuramyl-L-Ala-D-isoGlu-L-Ala-glycerol dipalmitate); DRVs (immunoliposomes prepared from dehydration-rehydration vesicles); interferongamma; interleukin-1beta; interleukin-2; interleukin-7; interleukin-12; ISCOMS™; ISCOPREP 7.0.3. ™; liposomes; LOXORIBINE™ (7-allyl-8-oxoguanosine); LT 5 oral adjuvant (*E.coli* labile enterotoxin-protoxin); microspheres and microparticles of any composition ; MF59TM; (squalenewater emulsion); MONTANIDE ISA 51™ (purified incomplete Freund's adjuvant); MONTANIDE ISA 720™ (metabolisable oil adjuvant); MPL™ (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE™ (Nac-Mur-L-Ala-D-Gln-OCH3); MURAPALMITINE™ and DMURAPALMITINE™ (Nac-Mur-L-Thr-D-isoGln-sn-glyceroldipalmitoyl); NAGO (neuraminidase- galactose oxidase); nanospheres or nanoparticles of any composition ; NISVs (non-ionic surfactant vesicles); PLEURAN™ (-glucan); PLGA, PGA and PLA (homo- and co-polymers of lactic acid and glycolic acid; microspheres/nanospheres); PLURONIC L121™; PMMA (polymethylmethacrylate); PODDS™ (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON™ (QS-21); Quil-A (Quil-A saponin); S-28463 (4-amino-otec-dimethyl-2-ethoxymethyl-1H-imidazo[4,5-c]quinoline-1-ethanol); SAF-1™ ("Syntex adjuvant formulation"); Sendai proteoliposomes and Sendaicontaining lipid matrices; Span-85 (sorbitan trioleate); Specol (emulsion of Marcol 52, Span 85 and Tween 85); squalene or Robane® (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid® (N-acetylglucosaminyl-N-acetylmuramyl-L-Ala-D-isoGlu-L-Aladipalmitoxypropylamide); Theronyl-MDP (Termurtide™ or [thr 1]-MDP; N-acetylmuramyl-Lthreonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin, microbe derived adjuvants, including Romurtide, DETOX, MPL, CWS, Mannose, CpG nucleic acid sequences, CpG7909, ligands of human TLR 1-10, ligands of murine TLR 1-13, ISS-1018, 35 IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

The inventive vaccine composition can additionally contain one or more auxiliary substances in order to increase its immunogenicity or immunostimulatory capacity, if desired. A synergistic action of the components of the inventive vaccine composition as defined herein and of an auxiliary substance, which may be optionally contained in the inventive vaccine composition or may be formulated therewith, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

The inventive vaccine composition can also additionally contain any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

In this context especially preferred as immunostimulating compounds are immunostimulatory nucleic acids which are known to bind to TLR receptors. Such an immunostimulatory nucleic acid can be in the form of a(n) (immunostimulatory) CpG nucleic acid, in particular CpG-RNA or CpG-DNA, which preferably induces an innate immune response. A CpG-RNA or CpG-DNA used according to the invention can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid used according to the invention is preferably in the form of CpG-RNA, more preferably in the form of single-stranded CpG-RNA (ss CpG-RNA). Also preferably, such CpG nucleic acids have a length as described above. Preferably the CpG motifs are unmethylated.

In this context, the adjuvant may be an immunostimulatory nucleic acid as defined herein, preferably an immunostimulatory RNA (isRNA) as defined herein, which preferably elicits an innate immune response. Such an immunostimulatory nucleic acid may furthermore comprise or be complexed with a cationic or polycationic compound as described for the adjuvant component as defined herein. Alternatively, the adjuvant component as defined herein may be used an adjuvant.

According to a particularly preferred aspect, such immunostimulatory nucleic acid sequences, preferably an isRNA, consists of or comprises a nucleic acid of formula (I), (II), (III) or (IV) as defined above.

Further additives which may be included in the inventive vaccine composition are emulsifiers, such as, for example, Tween®, wetting agents, such as, for example, sodium lauryl sulfate; colouring agents; taste-imparting agents, pharmaceutical carriers; tablet-forming agents; stabilizers; antioxidants; preservatives.

Additionally, the inventive vaccine composition may comprise further functional peptides or proteins as already defined herein, e.g. so called cell penetrating peptides (CPPs) or cationic peptides for transportation.

The inventive vaccine composition may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Preferably, the inventive vaccine composition may be administered intradermally to reach APCs in the dermis.

The inventive vaccine composition may also be administered orally in any orally acceptable dosage form including, but not limited to, capsules, tablets, aqueous suspensions or solutions.

The inventive vaccine composition may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the inventive vaccine composition may be formulated in a suitable ointment, containing the components of the inventive vaccine composition and optionally further compounds as defined herein suspended or dissolved in one or more carriers.

The inventive vaccine composition typically comprises a "safe and effective amount" of the components of the inventive vaccine composition as defined herein. As used herein, a "safe and effective amount" preferably means an amount of these components, preferably the immunostimulatory nucleic acid, the adjuvant component and/or the antigen as defined herein, that is sufficient to significantly induce a positive modification of a disease or disorder as defined herein. At the same time, however, a "safe and effective amount" is small enough to avoid serious side-effects and to permit a sensible relationship between advantage and risk. The determination of these limits typically lies within the scope of sensible medical judgment.

The inventive vaccine composition as defined herein may be used for human and also for veterinary medical purposes, preferably for human medical purposes. More preferably, the inventive vaccine composition may be used for treating a mammal for diseases as mentioned herein. In the context of the present invention, a mammal may be selected from any mammal, preferably from a mammal, selected from the group comprising, without being limited thereto, e.g. goat, cattle, swine, dog, cat, donkey, monkey, ape, a rodent such as a mouse, hamster, rabbit, and, in particular, human. Administration modes may be as defined herein.

According to one further specific embodiment, the present invention is directed to the first medical use of the inventive vaccine composition as defined herein as a medicament.

According to another embodiment, the present invention is directed to the second medical use of the inventive vaccine composition as defined herein, optionally in form of a kit, for the treatment of diseases as defined herein. Alternatively or additionally, the second medical use of the compounds of the inventive vaccine composition as defined herein is also envisaged, i.e. a) an adjuvant component comprising or consisting of at least one immunostimulatory nucleic acid sequence, complexed with a cationic or polycationic peptide or a protein as a complexing agent; b) a nucleic acid sequence encoding an antigen, wherein the nucleic acid is an RNA antigenantigen; and c) a polymeric carrier molecule for combined packaging the adjuvant component and the RNA encoding the antigen, all as defined herein, for the preparation of a vaccine composition , preferably as defined herein, for the treatment of diseases as defined herein.

Preferably, diseases as mentioned herein are selected from cancer or tumour diseases, infectious diseases, preferably (viral, bacterial or protozoological) infectious diseases, autoimmune diseases, allergies or allergic diseases, cardiovascular diseases, or neuronal diseases.

Such diseases include cancer or tumour diseases, preferably selected from melanomas, malignant melanomas, colon carcinomas, lymphomas, sarcomas, blastomas, renal carcinomas, gastrointestinal tumours, gliomas, prostate tumours, bladder cancer, rectal tumours, stomach cancer, oesophageal cancer, pancreatic cancer, liver cancer, mammary carcinomas (= breast cancer), uterine cancer, cervical cancer, acute myeloid leukaemia (AML), acute lymphoid leukaemia (ALL), chronic myeloid leukaemia (CML), chronic lymphocytic leukaemia (CLL), hepatomas, various virus-induced tumours such as, for example, papilloma virus-induced carcinomas (e.g. cervical carcinoma = cervical cancer), adenocarcinomas, herpes virus-induced tumours (e.g. Burkitt's lymphoma, EBV-induced B-cell lymphoma), heptatitis B-induced tumours (hepatocell carcinomas), HTLV-1- and HTLV-2-induced lymphomas, acoustic neuroma, lung carcinomas (= lung cancer = bronchial carcinoma), small-cell lung carcinomas, pharyngeal cancer, anal carcinoma, glioblastoma, rectal carcinoma, astrocytoma, brain tumours, retinoblastoma, basalioma, brain metastases, medulloblastomas, vaginal cancer, pancreatic cancer, testicular cancer, Hodgkin's syndrome, meningiomas, Schneeberger disease, hypophysis tumour, Mycosis fungoides, carcinoids, neurinoma, spinalioma, Burkitt's lymphoma, laryngeal cancer, renal cancer, thymoma, corpus carcinoma, bone cancer, non-Hodgkin's lymphomas, urethral cancer, CUP syndrome, head/neck tumours, oligodendroglioma, vulval cancer, intestinal cancer, colon carcinoma, oesophageal carcinoma (= oesophageal cancer), wart involvement, tumours of the small intestine, craniopharyngeomas, ovarian carcinoma, genital tumours, ovarian cancer (= ovarian carcinoma), pancreatic carcinoma (= pancreatic cancer), endometrial carcinoma, liver metastases, penile cancer, tongue cancer, gall bladder cancer, leukaemia, plasmocytoma, lid tumour, prostate cancer (= prostate tumours), etc.

According to one further specific aspect, diseases as defined herein comprise infectious diseases, preferably (viral, bacterial or protozoological) infectious diseases. Such infectious diseases, preferably viral, bacterial or protozoological infectious diseases, are typically selected from viral infectious diseases such as influenza, preferably influenza-A, influenza-B, influenza-C or thogotovirus, more preferably influenza-A comprising e.g. haemagglutinin subtypes H1, H2, H3, H4, H5, H6, H7, H8, H9, H10, H11, H12, H13, H14 or H15, and/or neuroamidase subtypes N1, N2, N3, N4, N5, N6, N7, N8 or N9, or preferably influenza-A subtypes H1N1, H1N2, H2N2, H2N3, H3N1, H3N2, H3N3, H5N1, H5N2, H7N7 or H9N2, etc., or any further combination,, malaria, severe acute respiratory syndrome (SARS), yellow fever, AIDS, Lyme borreliosis, Leishmaniasis, anthrax, meningitis, Condyloma acuminata, hollow warts, Dengue fever, three-day fever, Ebola virus, cold, early summer meningoencephalitis (FSME), shingles, hepatitis, herpes simplex type I, herpes simplex type II, Herpes zoster, Japanese encephalitis, Arenavirus-associated diseases (Lassa fever infection), Marburg virus, measles, foot-and-mouth disease, mononucleosis infectiosa (Pfeiffer's glandular fever), mumps, Norwalk virus infection, smallpox, polio (childhood lameness), pseudo-croup, Erythema infectiosum (fifth disease), rabies, warts, West Nile fever, chickenpox, cytomegalic virus (CMV), bacterial infectious diseases such as miscarriage (prostate inflammation), anthrax, appendicitis, borreliosis, botulism, Camphylobacter, Chlamydia trachomatis (inflammation of the urethra, conjunctivitis), cholera, diphtheria, donavanosis, epiglottitis, typhus fever, gas gangrene, gonorrhoea, rabbit fever, Heliobacter pylori, whooping cough, climatic bubo, osteomyelitis, Legionnaire's disease, leprosy, listeriosis, pneumonia, meningitis, bacterial meningitis, anthrax, otitis media, Mycoplasma hominis, neonatal sepsis (Chorioamnionitis), noma, paratyphus, plague, Reiter's syndrome, Rocky Mountain spotted fever, Salmonella paratyphus, Salmonella typhus, scarlet fever, syphilis, tetanus, tripper, tsutsugamushi disease, tuberculosis, typhus, vaginitis (colpitis), soft chancre, and infectious diseases caused by parasites, protozoa or fungi, such as amoebiasis, bilharziosis, Chagas disease, Echinococcus, fish tapeworm, fish poisoning (Ciguatera), fox tapeworm, athlete's foot, canine tapeworm, candidosis, yeast fungus spots, scabies, cutaneous Leishmaniosis, lambliasis (giardiasis), lice, malaria, microscopy, onchocercosis (river blindness), fungal diseases, bovine tapeworm, schistosomiasis, porcine tapeworm, toxoplasmosis, trichomoniasis, trypanosomiasis (sleeping sickness), visceral Leishmaniosis, nappy/diaper dermatitis or miniature tapeworm.

According to another specific aspect, diseases as defined herein comprise autoimmune diseases as defined in the following. Autoimmune diseases can be broadly divided into systemic and organ-specific or localised autoimmune disorders, depending on the principal clinico-pathologic features of each disease. Autoimmune diseases may be divided into the categories of systemic syndromes, including systemic lupus erythematosus (SLE), Sjögren's syndrome, Scleroderma, Rheumatoid Arthritis and polymyositis or local syndromes which may be endocrinologic (type I diabetes (Diabetes mellitus Type 1), Hashimoto's thyroiditis, Addison's disease etc.), dermatologic (pemphigus vulgaris), haematologic (autoimmune haemolytic anaemia), neural (multiple sclerosis) or can involve virtually any circumscribed mass of body tissue. The autoimmune diseases to be treated may be selected from the group consisting of type I autoimmune diseases or type II autoimmune diseases or type III autoimmune diseases or type IV autoimmune diseases, such as, for example, multiple sclerosis (MS), rheumatoid arthritis, diabetes, type I diabetes (Diabetes mellitus Type 1), chronic polyarthritis, Basedow's disease, autoimmune forms of chronic hepatitis, colitis ulcerosa, type I allergy diseases, type II allergy diseases, type III allergy diseases, type IV allergy diseases, fibromyalgia, hair loss, Bechterew's disease, Crohn's disease, Myasthenia gravis, neurodermitis, Polymyalgia rheumatica, progressive systemic sclerosis (PSS), Reiter's syndrome, rheumatic arthritis, psoriasis, vasculitis, etc, or type II diabetes. While the exact mode as to why the immune system induces an immune reaction against autoantigens has not been elucidated so far, there are several findings with regard to the etiology. Accordingly, the autoreaction may be due to a T cell bypass. A normal immune system requires the activation of B-cells by T cells before the former can produce antibodies in large quantities. This requirement of a T cell can be by-passed in rare instances, such as infection by organisms producing super-antigens, which are capable of initiating polyclonal activation of B-cells, or even of T cells, by directly binding to the β-subunit of T cell receptors in a non-specific fashion. Another explanation deduces autoimmune diseases from a "Molecular Mimicry": an exogenous antigen may share structural similarities with certain host antigens; thus, any antibody produced against this antigen (which mimics the self-antigens) can also, in theory, bind to the host antigens and amplify the immune response. Autoimmune diseases based on molecular mimicry are known to a skilled person for various viral and bacterial antigens. The most striking form of molecular mimicry is observed in Group A beta-haemolytic streptococci, which shares antigens with human myocardium, and is responsible for the cardiac manifestations of rheumatic fever.

Additionally, according to one further specific aspect, diseases as defined herein comprise allergies or allergic diseases, i.e. diseases related to allergies. Allergy is a condition that typically involves an abnormal, acquired immunological hypersensitivity to certain foreign antigens or allergens, such as the allergy antigens as defined herein. Such allergy antigens or allergens may be selected from allergy antigens as defined herein antigens derived from different sources, e.g. from animals, plants, fungi, bacteria, etc. Allergens in this context include e.g. danders, grasses, pollens, molds, drugs, or numerous environmental triggers, etc. Allergies normally result in a local or systemic inflammatory response to these antigens or allergens and lead to immunity in the body against these allergens. Without being bound to theory, several different disease mechanisms are supposed to be involved in the development of allergies. According to a classification scheme by P. Gell and R. Coombs the word "allergy" was restricted to type I hypersensitivities, which are caused by the classical IgE mechanism. Type I hypersensitivity is characterised by excessive activation of mast cells and basophils by IgE, resulting in a systemic inflammatory response that can result in symptoms as benign as a runny nose, to life-threatening anaphylactic shock and death. Well known types of allergies include, without being limited thereto, asthma, allergic asthma (leading to swelling of the nasal mucosa), allergic conjunctivitis (leading to redness and itching of the conjunctiva), allergic rhinitis ("hay fever"), anaphylaxis, angiodema, atopy, atopic dermatitis (eczema), urticaria (hives), eosinophilia, respiratory, allergies to insect stings, skin allergies (leading to and including various rashes, such as eczema, hives (urticaria) and (contact) dermatitis), food allergies, allergies to medicine, etc.;etc. Treatment of such allergic disorders or diseases may occur preferably by desensitizing the immune reaction which triggers a specific immune response. Such a desensitizing may be carried out by administering an effective amount of the allergen or allergic antigen encoded by the nucleic acid as defined herein, preferably, when formulated as a pharmaceutical composition, to induce a slight immune reaction. The amount of the allergen or allergic antigen may then be raised step by step in subsequent administrations until the immune system of the patient to be treated tolerates a specific amount of allergen or allergic antigen.

Diseases in the context of the present invention may also include type II hypersensitivity reactions (cytotoxic, antibody-dependent), including e.g. autoimmune hemolytic anemia, thrombocytopenia, erythroblastosis fetalis, Goodpasture's syndrome, Graves' disease, Myasthenia Gravis, etc.; type III hypersensitivity reactions (immune complex disease), , including e.g. serum sickness, Arthus reaction, Systemic lupus erythematosus (SLE), etc.; type IV hypersensitivity reactions (delayed-type hypersensitivity (DTH), cell-mediated immune memory response, antibody-independent), including e.g. contact dermatitis, Mantoux test, chronic transplant rejection, multiple sclerosis, etc.; and type V hypersensitivity reactions (receptor mediated autoimmune disease), including e.g. Graves' disease, Myasthenia Gravis, etc.;

Likewise, diseases in the context of the present invention may include cardiovascular diseases chosen from, without being limited thereto, coronary heart disease, arteriosclerosis, apoplexy and hypertension, etc.

Finally, diseases in the context of the present invention may be chosen from neuronal diseases including e.g. Alzheimer's disease, amyotrophic lateral sclerosis, dystonia, epilepsy, multiple sclerosis and Parkinson's disease etc.

According to a final embodiment, the present invention also provides a kit, which may comprise the inventive vaccine composition as defined herein and optional technical instructions with information on the administration and dosage of the inventive vaccine composition as defined herein or of any of its components. The present application may also provide a kit of parts, which may comprise in one part of the kit the inventive vaccine composition as defined herein, and optionally in at least one further part of the kit additional components of the inventive vaccine composition as defined herein, and optionally in one further part technical instructions with information on the administration and dosage of the inventive vaccine composition as defined herein or of any of its components. Preferably, such an inventive kit of parts may comprise one inventive vaccine composition as defined herein, wherein the inventive vaccine composition preferably comprises or encodes at least one antigen as defined herein, e.g. one or more (preferably different) antigens as defined herein, preferably a cocktail of antigens as defined herein. The inventive vaccine composition may be contained in one part of the kit. Further parts of the kit may preferably contain additional components, additives, etc., preferably together in one part or each of the additional components, additives, etc., in a separate part of the kit. The kit may also comprise in a further part of the kit technical instructions with information on the administration and dosage of the inventive vaccine composition as defined herein or of any of its components. The inventive kit may also comprise different inventive vaccine compositions as defined herein, each inventive vaccine composition preferably comprising or encoding a different antigen as defined herein. Such a kit may also comprise in a further part of the kit technical instructions with information on the administration and dosage of the inventive vaccine composition as defined herein or of any of its components.

In the present invention, if not otherwise indicated, different features of alternatives and embodiments may be combined with each other. Furthermore, the term "comprising" shall not be construed as meaning "consisting of", if not specifically mentioned. However, in the context of the present invention, term "comprising" may be substituted with the term "consisting of", where applicable.

### Figures:

The following Figures are intended to illustrate the invention further. They are not intended to limit the subject matter of the invention thereto.

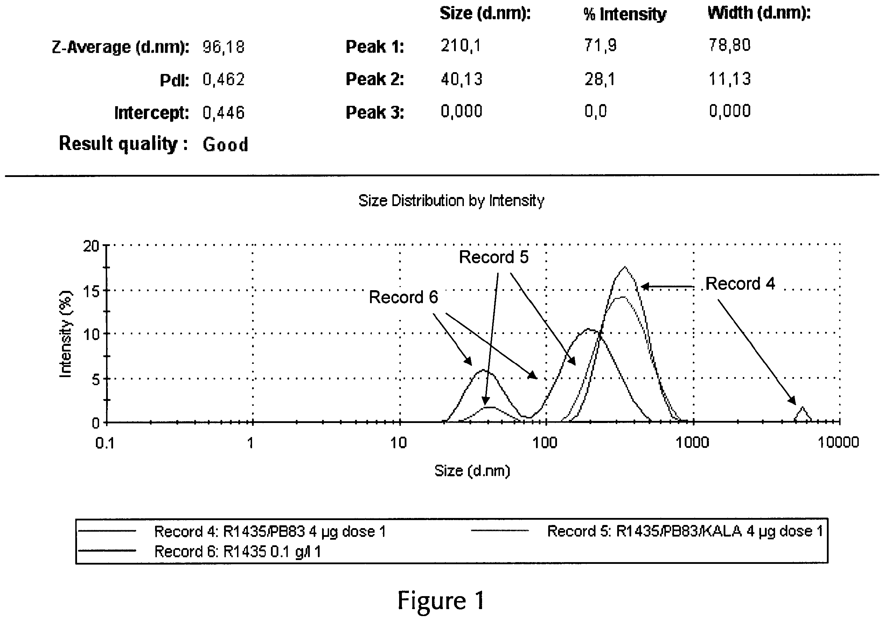

- **Figure 1:**: shows the particle size measurement of inventive complexes. The single measurements are displayed by Records 4 to 6, wherein
Record 4 shows the inventive vaccine R1435/PB83, wherein
the adjuvant component is mRNA R1435 coding for *Gallus gallus* ovalbumine complexed with protamine (2:1; (w/w);
the antigen is free mRNA R1435 (ratio 1:1; complexed RNA:free RNA); and
the carrier is PB83: HO-PEG₅₀₀₀-S-(S-CHHHHHHRRRRHHHHHHC-S-)₇-S-PEG₅₀₀₀-OH;
Record 5 shows the inventive vaccine R1435/PB83/KALA, wherein
the adjuvant component is mRNA R1435 coding for *Gallus gallus* ovalbumine complexed with protamine (2:1; (w/w));
the antigen is free mRNA R1435 (ratio 1:1; complexed RNA:free RNA); and
the carrier is PB83: HO-PEG₅₀₀₀-S-(S-CHHHHHHRRRRHHHHHHC-S-)₇-S-PEG₅₀₀₀-OH, with enhancer peptide KALA;
Record 6 shows as a control the "parent" formulation R1435, wherein
the adjuvant component consists of mRNA coding for *Gallus gallus* ovalbumine complexed with protamine (2:1; (w/w));
the antigen is free mRNA coding for *Gallus gallus* ovalbumine (ratio 1:1; complexed RNA:free RNA).
As can be seen, the parent formulation R1435 shows two components, the adjuvant component consisting of complexed RNA with an average particle size of 200 nm and the antigen component consisting of free mRNA with an average particle size of 40 nm. The complexation of the parent formulation R1435 with the carrier PB83 leads to generation of a uniform component with an average particle size of 300 nm. As can be seen, the addition of KALA to such a complex does not change the particle properties.

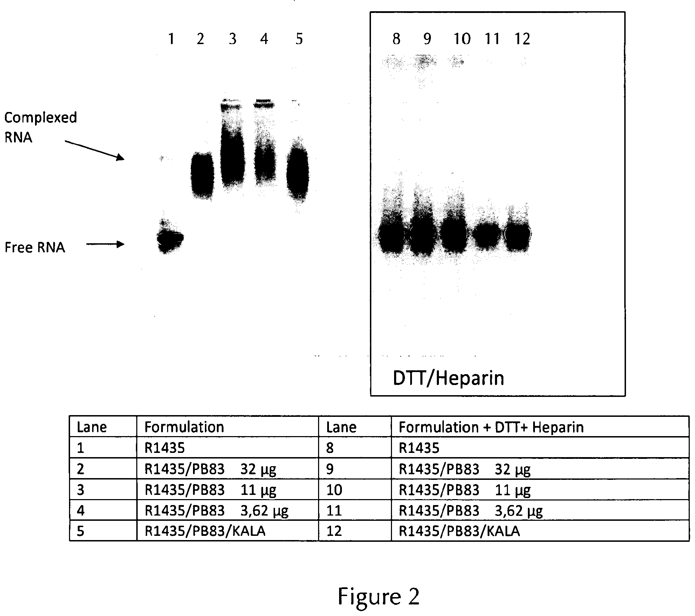

- **Figure 2:**: shows an agarose gel shift assay, wherein the left part of Figure 2 shows the agarose gel shift of the "parent" formulation R1435 or different amounts of the inventive vaccine formulations with the carrier PB83 (R1425/PB83). As can be seen, the parent formulation consists of two components, the complexed RNA (upper band) and free mRNA (lower band). By contrast the inventive vaccine formulation only shows one band and no band of free mRNA. The right part of Figure 2 shows the agarose gel shift of the formulations after 30 minutes treatment with heparin/DTT at 37°C. As can be easily seen the RNA is electrostatically replaced by heparin and therefore leads to the same gel shift as the free RNA. Consequently, complexation of the RNA as carried out in the inventive vaccine formulation is reversible under reducible conditions as present in the endosome.

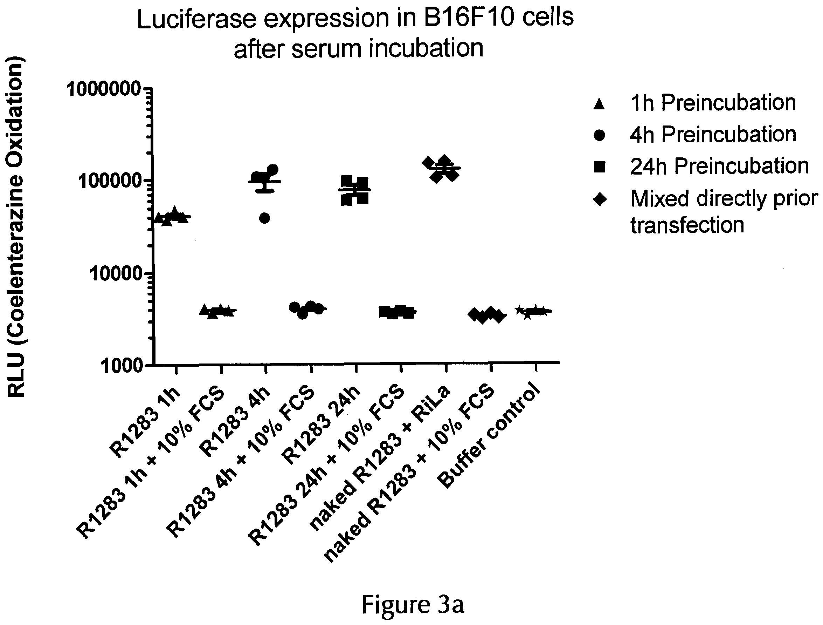

- **Figure 3a:**: shows results from serum stability experiments of "parent" formulations of R1283 consisting of naked mRNA and protamine complexed RNA. As can be seen serum incubation leads to loss of luciferase activity in all parent formulations (R1283). Naked RNA (naked R1283) is even degraded after several seconds of incubation with serum. Thus, RNA comprised in the parent formulation is not protected against nucleases.

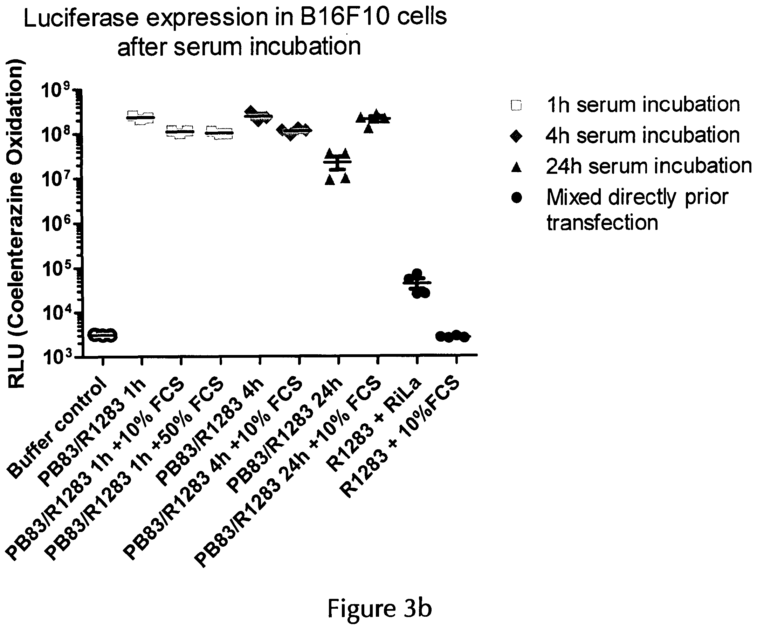

- **Figure 3b:**: shows results from serum stability experiments of inventive vaccine formulations based on a R1283 parent formulation, which has been further formulated with the carrier PB83. As can be seen, the carrier PB83 (R1283/PB83) confers protection on the RNA against RNAses compared to the (unprotected) parent formulation (R1283).

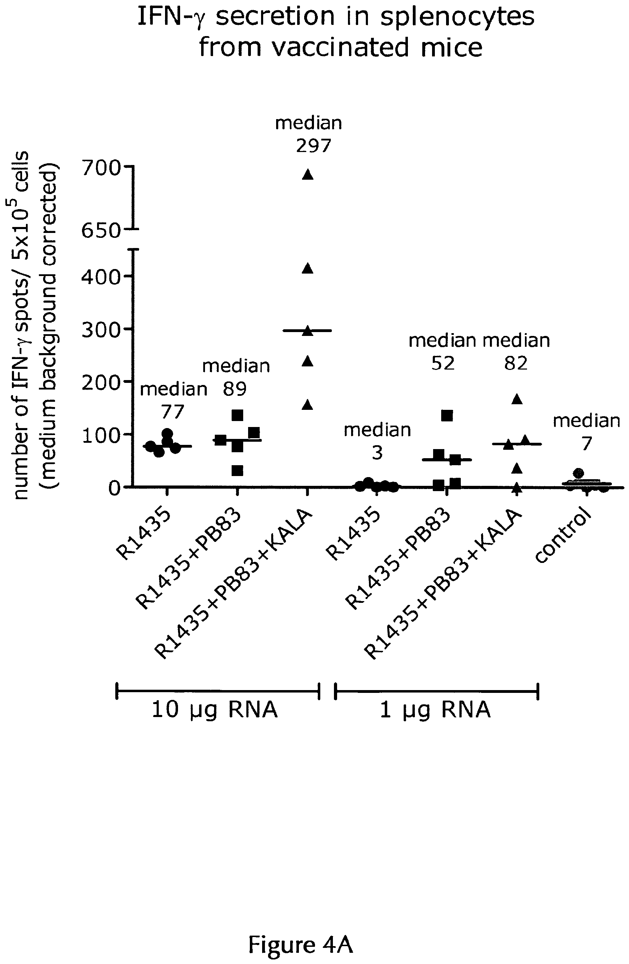

- **Figure 4A:**: shows the results from an IFN-γ ELISPOT assay. As can be seen, the inventive vaccine formulations (R1435/PB83) show significantly higher induction of ovalbumine specific cytotoxic T cells (CTLs) at low doses (1 µg) compared to the parent formulations (R1435) which is inactive at this dose level. Thus, the inventive vaccine formulation improves the CTL specific immune response against the antigen ovalbumine compared to the parent formulation. Advantageously, addition of the enhancer peptide KALA further improves the antigen-specific immune response.

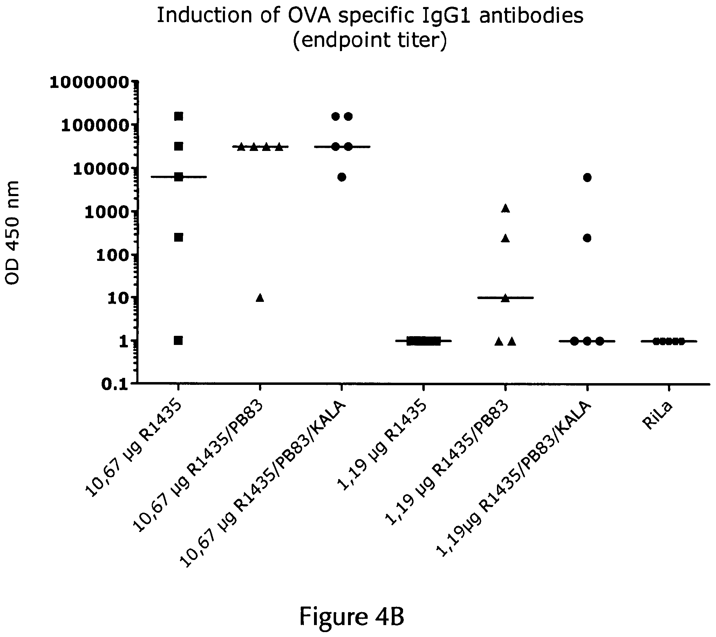

- **Figure 4B:**: shows the results from vaccination of mice with different formulations of R1435. Shown is the induction of OVA specific IgG1 antibodies (endpoint titer). As can be seen, complexation of the parent formulation with carrier PB83 improves the antibody response against the antigen ovalbumine. It can be seen that at the dose of 1.19 µg mRNA there is no response in the parent formulation group (R1435). In contrast, complexation with the carrier leads to an at least partially immune response (R1435/PB83).

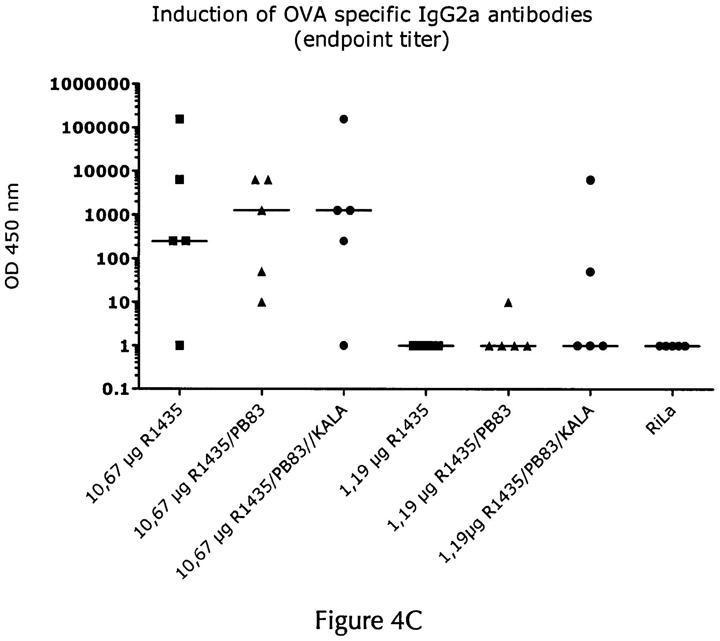

- **Figure 4C:**: shows the results from vaccination of mice with different formulations of R1435. Shown is the induction of OVA specific IgG2 antibodies (endpoint titer). As can be seen, complexation of the parent formulation with carrier PB83 improves the antibody response against the antigen ovalbumine. It can be seen that at the dose of 1.19 µg mRNA there is no response in the parent formulation group (R1435). In contrast, complexation with the carrier leads to an at least partially immune response (R1435/PB83).

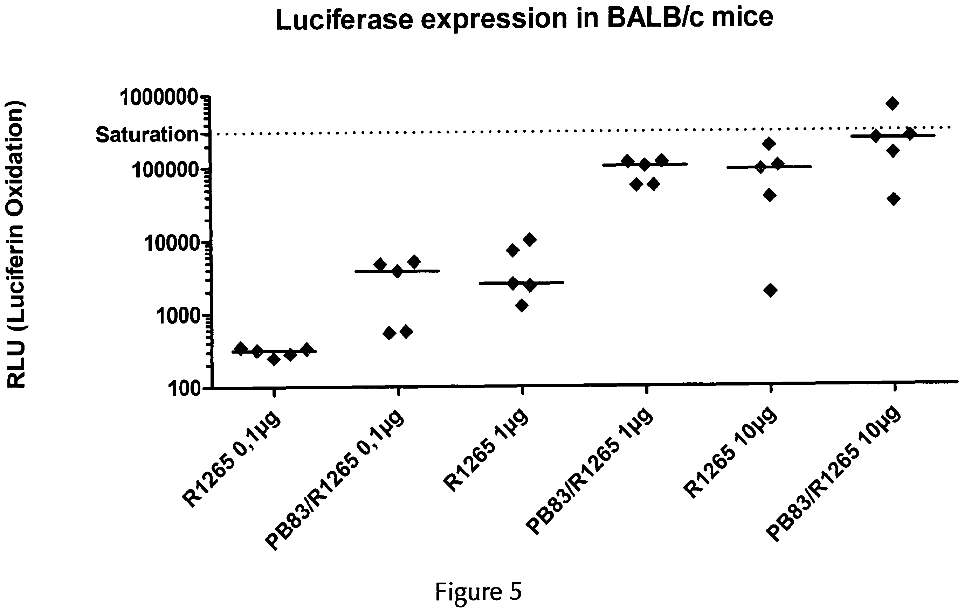

- **Figure 5:**: shows the effect of the complexation of a "parent" formulation (R1265) with a carrier on the efficiency of reporter mRNA expression after ID injection into BALB/c mice. As can be seen complexation of the "parent" formulation (R1265) with a carrier (R1265/PB83) significantly increases the expression of the encoded reporter protein luciferase. The achieved levels are even higher than levels achieved with naked mRNA (R1265 naked).

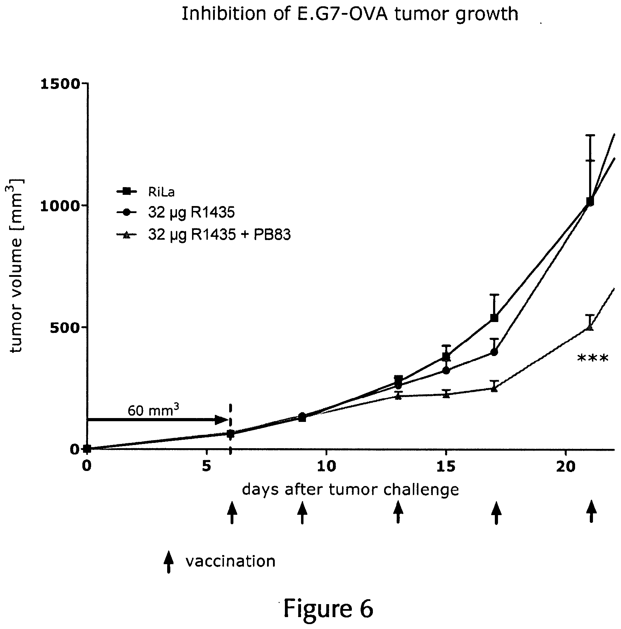

- **Figure 6:**: shows the effect of the inventive vaccine formulation of 32 µg mRNA coding for the antigen ovalbumine on tumour growth. As can be seen, vaccination of mice in a therapeutic setting using the inventive vaccine formulation decelerates tumour growth significantly. This was absolutely surprising as the vaccination schedule used herein usually does not lead to an influence on E.G7-OVA tumour growth.

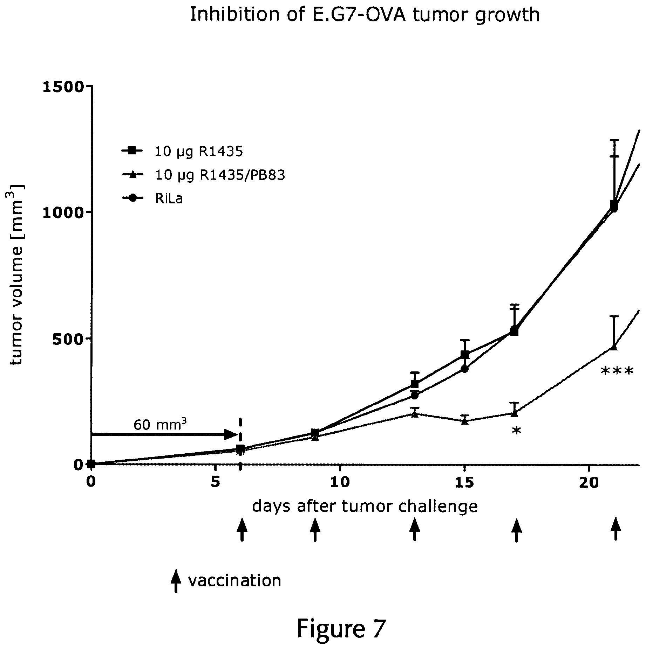

- **Figure 7:**: shows the effect of the inventive vaccine formulation of 10 µg mRNA coding for the antigen ovalbumine on tumour growth. As can be seen, vaccination of mice in a therapeutic setting using the inventive vaccine formulation decelerates the tumour growth significantly. This was absolutely surprising as the vaccination schedule used herein normally does not lead to an influence on E.G7-OVA tumour growth. Furthermore it was absolutely surprising that already 10 µg RNA are sufficient to significantly decelerate tumour growth.

### Examples:

The following examples are intended to further illustrate the invention. They are not intended to limit the subject matter of the invention thereto.

### 1. Preparation of DNA and mRNA constructs

For the present examples DNA sequences, encoding *Photinus pyralis* luciferase (R1265), *Gaussia princeps* luciferase (R1283), or *Callus gallus* ovalbumin (R1435), were prepared and used for subsequent *in vitro* transcription reactions.

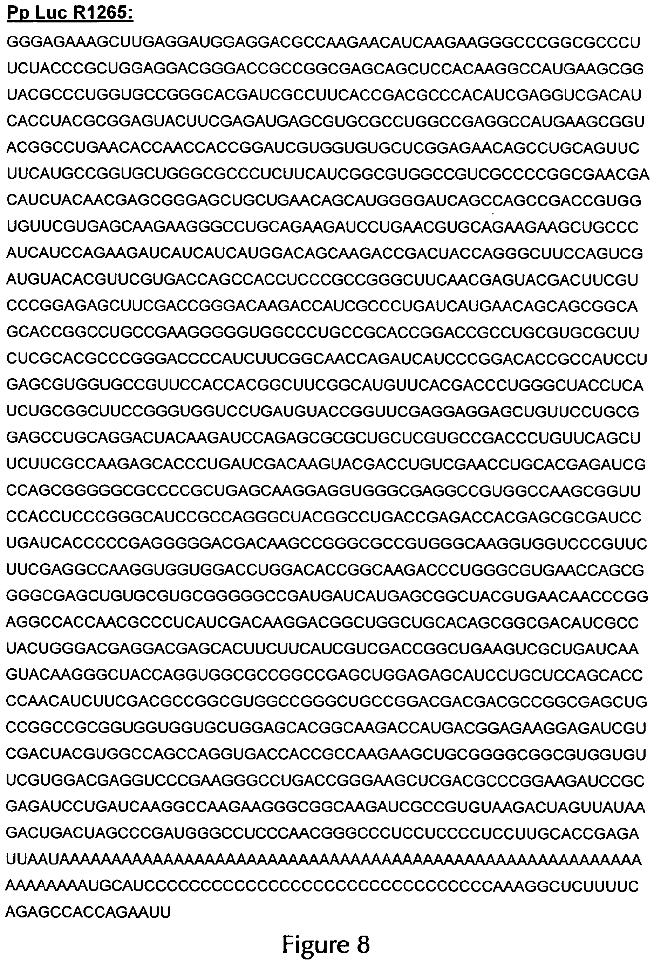

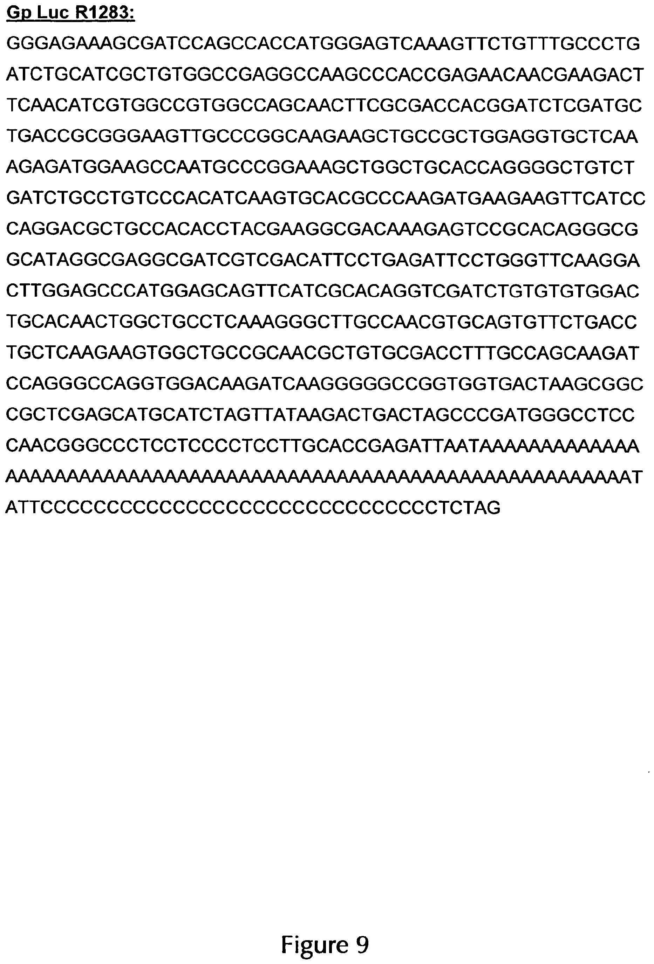

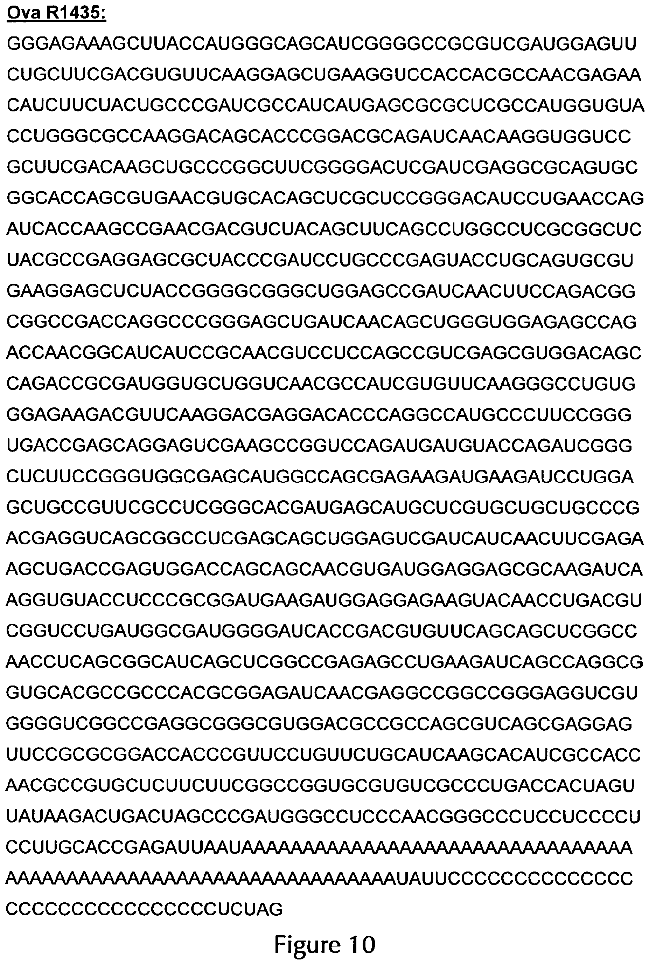

According to a first preparation, the DNA sequences coding for the mRNA R1265, R1283 or R1435 were prepared. The constructs were prepared by modifying the wild type *Photinus pyralis* luciferase, *Gaussia princeps* luciferase or *Gallus gallus* ovalbumine encoding DNA sequences by introducing a GC-optimized sequence for a better codon usage and stabilization, stabilizing sequences derived from alpha-globin-3'-UTR (muag (mutated alpha-globin-3'-UTR)), a stretch of 70 × adenosine at the 3'-terminal end (poly-A-tail), a stretch of 30 × cytosine at the 3'- terminal end (poly-C-tail), and a histone stem-loop sequence leading to SEQ ID NO: 544 (see Figure 8) for mRNA R1265, to SEQ ID NO: 545 (see Figure 9) for mRNA R1283, and to SEQ ID NO: 546 (see Figure 10) for mRNA R1435. In SEQ ID NOs: 544-546 (see Figures 8, 9 and 10) the sequence of the corresponding mRNAs are shown.

### 2. In vitro transcription:

The respective DNA plasmid prepared according to Example 1 was transcribed *in vitro* using T7 polymerase. Subsequently the mRNA was purified using PureMessenger® (CureVac, Tübingen, Germany).

### 3. Reagents:

### Complexation Reagent:

### Protamine

### Carrier:

PB83: HO- PEG₅₀₀₀-S-(S-CHHHHHHRRRRHHHHHHC-S-)₇-S- PEG₅₀₀₀-OH
PB83 (SEQ ID NO: 547) represents an exemplary carrier as described in the present invention.

### 4. Preparation of the adjuvant component and addition of the antigen:

The mRNA to be used as immunostimulatory nucleic acid sequence in the experiments below was complexed with protamine by addition of protamine to the mRNA in the indicated ratios (1:2) (w/w). After incubation for 10 min, the free mRNA to be used as antigen was added.

These combinations of an adjuvant component and an antigen mRNA without a further carrier were termed "parent" formulations: Following "parent" formulations were prepared:
**R1265:** adjuvant component consisting of mRNA coding *for Photinus pyrialis* Luciferase according to SEQ ID NO: 544 complexed with protamine in a ratio of 2:1 (w/w) and free mRNA coding for *Photinus pyrialis* Luciferase (antigen mRNA) according to SEQ ID NO 544 (ratio 1:1; complexed RNA:free RNA).

**R1283:** adjuvant component consisting of mRNA coding for *Caussia princeps* luciferase according to SEQ ID NO: 545 complexed with protamine in a ratio of 2:1 (w/w) and free mRNA coding for Luciferase (antigen mRNA) according to SEQ ID NO 545 (ratio 1:1; complexed RNA:free RNA).

**R1435:** adjuvant component consisting of mRNA coding for *Gallus gallus* ovalbumine according to SEQ ID NO: 546 complexed with protamine in a ratio of 2:1 (w/w) and free mRNA coding for *Gallus gallus* ovalbumine (antigen mRNA) according to SEQ ID NO 546 (ratio 1:1; complexed RNA:free RNA).

### 5. Synthesis of the carrier:

The condensation reaction was performed with the calculated amount of peptide (component P²) which was dissolved in a mixture of a buffered aqueous solution at pH 8,5 with an optional additive of 5% (v/v) Dimethylsulfoxide (DMSO) (which represent mild oxidation conditions and therefore allow the establishement of an equilibrium) and stirred for 18h at ambient temperature. Afterwards the calculated amount of a thiol group containing PEG derivative (alpha-Methoxy-omega-mercapto poly(ethylene glycol)) (component P¹) (dissolved in water) was added and the resulting solution was stirred for another 18h. Subsequent lyophilisation and purification yield the desired polymer. The ratio between PEG component P¹ to peptide component P² typically defines the chain length of the P² polymer.

The condensation reaction in this reaction environment is reversible, therefore the chain length of the polymer was determined by the amount of the monothiol compound which terminates the polymerisation reaction. In summary the length of the polymer chain was determined by the ratio of oligo-peptide and monothiol component. This reaction was supported by the chosen mild oxidation conditions. With more stringent oxidation conditions (30% DMSO) the generation of high molecular (long chain) polymers was induced.

### 1^{st} Step: Exemplary polymerization reaction:

n HS-CHHHRRRHHHC-SH → H-(S-CHHHRRRRHHHC-S)ₙ-H

### 2^{nd} Step: Exemplary stop reaction:

H-(S-CHHHRRRRHHHC-S)ₙ-H + 2 PEG-SH
→ PEG-S-(S-CHHHRRRRHHHC-S)ₙ-S-PEG

### Exemplary synthesis reaction:

1^{st} step:
   5 x HS-CHHHRRRHHHC-SH → H-(S-CHHHRRRRHHHC-S)s-H
2^{nd} step:
   H-(S-CHHHRRRRHHHC-S)₅-H +2x PEG₅₀₀₀-SH
   → PEG₅₀₀₀-S-(S-CHHHRRRRHHHC-S)₅-S-PEG₅₀₀₀

To achieve a polymer length of 5 (n = 5), a molar ratio of peptide:PEG of 5:2 was used.

### 6. Packaging of adjuvant component and antigen:

The adjuvant components in combination with the antigen ("parent" formulation prepared according to Example 4), were complexed for the purposes of the present invention with a carrier, preferably the carrier as defined in Example 5. In some cases an enhancer peptide (KALA) was added before complexation with the carrier. Afterwards the resulting solution was adjusted with water to a final volume of 50 µl and incubated for 30 minutes at room temperature.

| Component | mRNA | Carrier | Complexation reagent | Enhancer Peptide (e.g. KALA) |
|---|---|---|---|---|
| Ratio (mol) | 1 | 5-500 (50) | 0-250 (25) | 0-50 (5) |

The inventive vaccines are termed:
- R1265/PB83
- R1283/PB83
- R1435/PB83

### 7. Size measurement:

The hydrodynamic diameters of polyplexes as prepared above were measured by dynamic light scattering using a Zetasizer Nano (Malvern Instruments, Malvern, UK) according to the SOPs (standard operation procedure) distributed by Malvern. The measurements were performed at 25°C in Ringer Lactate solution and analyzed by a cumulant method to obtain the hydrodynamic diameters and polydispersity indices of the polyplexes.

### 8. Gel Shift Assay

Furthermore, different amounts of inventive vaccine complexes (R1435/PB83) were prepared as indicated in Example 6 above and aliquots were incubated with heparin (100 µg) and dithiothreitol (DTT) for 30 minutes at 37°C. In one group the enhancer peptide KALA was added to the inventive vaccine complexes (R1435/PB83/KALA). Subsequently electrophoresis was done on agarose gel and the nucleic acid sequences were visualized by ethidium bromide staining. For comparison the inventive vaccine formulation without packaging with the carrier (parent formulation) was used (R1435).

### 9. Transfection of B16F10 cells:

The inventive vaccine complexes were prepared according to Example 6. After serum incubation for the indicated time (1, 4, 24 h) the complexes containing 5 µg RNA were transfected in B16F10 cells. Then, B16F10 cells (150x10³/well) were seeded 1 day prior to transfection on 24-well microtiter plates leading to a 70% confluence when transfection was carried out. For transfection 50 µl of the vaccine complex solution were mixed with 250 µl serum free or FCS containing DEMEM and added to the cells (final RNA concentration: 13 µg/ml). Prior to addition of the serum free transfection solution the HeLa-cells were washed gently and carefully 2 times with 1 ml Optimen (Invitrogen) per well. Then, the transfection solution (300 µl per well) was added to the cells and the cells were incubated for 4 h at 37°C. Subsequently 300 µl RPMI-medium (Camprex) containing 10% FCS was added per well and the cells were incubated for additional 20 h at 37°C. The transfection solution was sucked off 24 h after transfection and the cells were lysed in 300 µl lysis buffer (25 mM Tris-PO₄, 2 mM EDTA, 10% glycerol, 1% Triton-X 100, 2 mM DTT). The supernatants were then mixed with luciferin buffer (25 mM Glycylglycin, 15 mM MgSO₄, 5 mM ATP, 62,5 µM luciferin) and luminiscence was detected using a luminometer (Lumat LB 9507 (Berthold Technologies, Bad Wildbad, Germany)).

The results of experiments with "parent" formulations of R1283 consisting of naked mRNA and protamine complexed RNA and of experiments with inventive vaccine formulations based on a R1283 parent formulation, which have been further formulated with the carrier PB83 are shown in Figures 3a and 3b. As can be seen in Figure 3a serum incubation leads to loss of luciferase activity in all parent formulations (R1283). Naked RNA (naked R1283) is even degraded after several seconds of incubation with serum. Thus, RNA comprised in the parent formulation is not protected against nucleases. In contrast thereto, as can be seen in Figure 3b, the carrier PB83 (R1283/PB83) confers protection on the RNA against RNAses compared to the (unprotected) parent formulation (R1283).

### 10. Expression of luciferase in vivo:

"Parent" formulations or inventive vaccine complexes were prepared according to Example 4 or 6 above. For determining luciferase expression *in vivo,* the indicated amount of mRNA containing complexes were injected intradermally (ear pinna or back) to 7 week old BALB/c mice (see Figure 5: 0.1 µg, 1 µg, 10 µg). After 24h the mice were sacrificed and the samples (ear, skin from the back or muscle) were collected, frozen at -78°C and lysed for 3 Minutes at full speed in a tissue lyser (Qiagen, Hilden, Germany). Afterwards 600 µl of lysis buffer were added and the resulting solutions were subjected another 6 minutes at full speed in the tissue lyser. After 10 minutes centrifugation at 13,500 rpm at 4°C the supernatants were mixed with luciferin buffer (25 mM Glycylglycin, 15 mM MgSO₄, 5 mM ATP, 62,5 µM luciferin) and luminiscence was detected using a luminometer (Lumat LB 9507 (Berthold Technologies, Bad Wildbad, Germany)).

The results can be seen in Figure 5. Figure 5 shows the effect of the complexation of a "parent" formulation (R1265) with a carrier on the efficiency of reporter mRNA expression after ID injection into BALB/c mice. As can be seen complexation of the "parent" formulation (R1265) with a carrier (R1265/PB83) significantly increases the expression of the encoded reporter protein luciferase. The achieved levels are even higher than levels achieved with naked mRNA (R1265 naked).

### 11. Immunization experiments:

For immunization the inventive vaccine complexes R1435/PB83, wherein the mRNA codes for *Gallus gallus* ovalbumin, were prepared according to Example 6 and transfected into C57 BL/6 mice. For comparison one group was transfected with a "parent" formulation consisting of the adjuvant component and antigen without complexation with the carrier (R1435). 4 mice per group were immunized intradermally 2 times with the indicated amount of mRNA containing complexes.

The results from an IFN-γ ELISPOT assay are shown in Figure 4a. As can be seen, the inventive vaccine formulations (R1435/PB83) show significantly higher induction of ovalbumine specific cytotoxic T cells (CTLs) at low doses (1 µg) compared to the parent formulations (R1435) which is inactive at this dose level. Thus, the inventive vaccine formulation improves the CTL specific immune response against the antigen ovalbumine compared to the parent formulation. Advantageously, addition of the enhancer peptide KALA further improves the antigen-specific immune response.

### 12. Detection of an antigen-specific immune response (B-cell immune response):

Detection of an antigen specific immune response (B-cell immune response) was carried out by detecting antigen specific antibodies. Therefore, blood samples were taken from vaccinated mice one week after the last vaccination and sera were prepared. MaxiSorb plates (Nalgene Nunc International) were coated with *Gallus gallus* ovalbumine protein. After blocking with 1×PBS containing 0.05% Tween-20 and 1% BSA the plates were incubated with diluted mouse serum (1:30, 1.90, 1:270, 1:810). Subsequently a biotin-coupled secondary antibody (Anti-mouse-IgG2a Pharmingen) was added. After washing, the plate was incubated with Horseradish peroxidase-streptavidin and subsequently the conversion of the ABTS substrate (2,2'-azino-bis(3-ethyl-benzthiazoline-6-sulfonic acid) was measured.

The results from vaccination of mice with different formulations of R1435 are shown in Figures 4b and 4c. Figure 4b shows the induction of OVA specific IgG1 antibodies (endpoint titer). As can be seen, complexation of the parent formulation with carrier PB83 improves the antibody response against the antigen ovalbumine. It can be seen that at the dose of 1.19 µg mRNA there is no response in the parent formulation group (R1435). In contrast, complexation with the carrier leads to an at least partially immune response (R1435/PB83). Figure 4c shows the induction of OVA specific IgG2 antibodies (endpoint titer). As can be seen, complexation of the parent formulation with carrier PB83 improves the antibody response against the antigen ovalbumine. It can be seen that at the dose of 1.19 µg mRNA there is no response in the parent formulation group (R1435). In contrast, complexation with the carrier leads to an at least partially immune response (R1435/PB83).

### 13. Detection of an antigen specific cellular immune response by ELISPOT:

One week after the last vaccination mice were sacrificed, the spleens were removed and the splenocytes were isolated. For detection of INFgamma a coat multiscreen plate (Millipore) was incubated overnight with coating buffer 0.1 M Carbonat-Bicarbonat Buffer pH 9.6, 10.59 g/l Na₂CO₃, 8.4g/l NaHCO₃) comprising antibody against INFγ (BD Pharmingen, Heidelberg, Germany). The next day 2x 10⁵ cells/well were added and re-stimulated with 0.25 µg/well of relevant (SIINFEKL of ovalbumine); irrelevant peptide (Cennexin) or buffer without peptide. Afterwards the cells are incubated for 24h at 37°C. The next day the plates were washed twice with PBS, once with water and once with PBS/0.05% Tween-20 and afterwards incubated with a biotin-coupled secondary antibody for 11-24h at 4°C. Then the plates were washed with PBS/0.05% Tween-20 and incubated for 2h with alkaline phosphatase coupled to streptavidin in blocking buffer. After washing with PBS/0.05% Tween-20 the substrate (5-Bromo-4-Cloro-3-Indolyl Phosphate/Nitro Blue Tetrazolium Liquid Substrate System from Sigma Aldrich, Taufkirchen, Germany) was added to the plate and the conversion of the substrate could be detected visually. The reaction was then stopped by washing the plates with water. The dried plates were then read out by an ELISPOT plate reader. For visualization of the spot levels the numbers were corrected by background subtraction.

### 14. Tumour challenge:

The samples used in this experiment were:
- R1435: Vaccine consisting of protamine complexed mRNA and free mRNA coding for *Gallusgallus* ovalbumine prepared according to example 4.
- R1435/PB83: Inventive vaccine prepared according to example 6 containing protamine complexed mRNA and free mRNA coding for *Gallus gallus* ovalbumine complexed with the carrier PB83.
- RiLa control: 80% Ringer lactate was used as control

At day 0 1x10⁶ E.G7-OVA cells (tumour cells which stably express ovalbumine) were implanted subcutaneously in 7 week old C57BL/6 mice. Beginning with a tumour volume of 60 mm³ mice were vaccinated intradermally with 5 cycles of 80 µl formulations containing 10 or 32 µg mRNA coding for *Gallus gallus* ovalbumine. As a negative control 80 µl 80% Ringer lactate without any RNA were injected. Tumour growth was monitored by measuring the tumour size in 3 dimensions using a calliper.

The effect of the inventive vaccine formulation of 32 µg mRNA coding for the antigen ovalbumine on tumour growth is shown in Figures 6 and 7. As can be seen in Figure 6, vaccination of mice in a therapeutic setting using the inventive vaccine formulation decelerates tumour growth significantly. This was absolutely surprising as the vaccination schedule used herein usually does not lead to an influence on E.G7-OVA tumour growth. In Figure 7 the effect of the inventive vaccine formulation of 10 µg mRNA coding for the antigen ovalbumine on tumour growth is shown. As can be seen, vaccination of mice in a therapeutic setting using the inventive vaccine formulation decelerates the tumour growth significantly. This was absolutely surprising as the vaccination schedule used herein normally does not lead to an influence on E.G7-OVA tumour growth. Furthermore it was absolutely surprising that already 10 µg RNA are sufficient to significantly decelerate tumour growth.

## Claims

1. Vaccine composition, comprising or consisting of:
a) an adjuvant component comprising or consisting of at least one immunostimulatory nucleic acid sequence, complexed with a cationic or polycationic peptide or protein as a complexing agent;
b) a nucleic acid sequence encoding an antigen, wherein the nucleic acid is an RNA; and
c) a polymeric carrier molecule for combined packaging the adjuvant component and the RNA encoding the antigen,
wherein the carrier molecule for combined packaging of the adjuvant component and the RNA encoding the antigen is a polymeric carrier molecule according to generic formula (VI):
L-P¹-S-[S-P²-S]ₙ-S-P³-L
wherein
P¹ and P³ are different or identical to each other and represent a linear or branched hydrophilic polymer chain, each P¹ and P³ exhibiting at least one -SH-moiety, capable to form a disulfide linkage upon condensation with component P², the linear or branched hydrophilic polymer chain being selected from polyethylene glycol (PEG), wherein the hydrophilic polymer chain exhibits a molecular weight of 1 kDa to 100 kDa,
P² is a cationic or polycationic peptide or protein, having a length of 3 to 100 amino acids, or
is a cationic or polycationic polymer, having a molecular weight of 0.5 kDa to 30 kDa, each P² exhibiting at least two -SH-moieties, capable to form a disulfide linkage upon condensation with further components P² or component(s) P¹ and/or P³;
-S-S- is a (reversible) disulfide bond;
L is an optional ligand, which may be present or not, and may be selected independently from the other from RGD, Transferrin, Folate, a signal peptide or signal sequence, a localization signal or sequence, a nuclear localization signal or sequence (NLS), an antibody, a cell penetrating peptide (CPP), TAT, KALA, a ligand of a receptor, cytokines, hormones, growth factors, small molecules, carbohydrates, mannose, galactose, synthetic ligands, small molecule agonists, inhibitors or antagonists of receptors, or RGD peptidomimetic analogues; and
n is an integer, selected from a range of 1 to 50.

2. Vaccine composition according to claim 1, wherein n is an integer in a range of 1, 2, 3, 4, or 5 to 10, preferably in a range of 1, 2, 3, or 4 to 9.

3. Vaccine composition according to claim 1 or 2, wherein the immunostimulatory nucleic acid of the adjuvant component is selected from immunostimulatory CpG nucleic acid sequences, immunostimulatory RNA (is)RNA, ribosomal RNA (rRNA), transfer RNA (tRNA), messenger RNA (mRNA), viral RNA (vRNA) or an RNA or mRNA encoding an antigen.

4. Vaccine composition according to any of claims 1 to 3, wherein the immunostimulatory RNA (is)RNA is selected from
a nucleic acid sequence of formula (I), (II), (III) or (IV):
GₗXₘGₙ (formula (I))
wherein:
G is guanosine, uridine or an analogue of guanosine or uridine ;
X is guanosine, uridine , adenosine, thymidine, cytidine or an analogue of the above-mentioned nucleotides;
l is an integer from 1 to 40,
wherein
when I = 1, G is guanosine or an analogue thereof,
when I > 1, at least 50% of the nucleotides are guanosine or an analogue thereof;
m is an integer and is at least 3;
wherein
when m = 3, X is uridine or an analogue thereof,
when m > 3, at least 3 successive uridines or analogues of uridine occur;
n is an integer from 1 to 40,
wherein
when n = 1, G is guanosine or an analogue thereof,
when n > 1, at least 50% of the nucleotides are guanosine or an analogue thereof;
CₗXₘCₙ (formula (II))
wherein:
C is cytidine, uridine or an analogue of cytidine or uridine;
X is guanosine, uridine, adenosine, thymidine, cytidine or an analogue of the above-mentioned nucleotides;
l is an integer from 1 to 40,
wherein
when l = 1, C is cytidine or an analogue thereof,
when l > 1, at least 50% of the nucleotides are cytidine or an analogue thereof;
m is an integer and is at least 3;
wherein
when m = 3, X is uridine or an analogue thereof,
when m > 3, at least 3 successive uridines or analogues of uridine occur;
n is an integer from 1 to 40,
wherein
when n = 1, C is cytidine or an analogue thereof,
when n > 1, at least 50% of the nucleotides are cytidine or an analogue thereof;
(NᵤGₗXₘGₙNᵥ)ₐ, (formula (III))
wherein:
G is guanosine (guanine), uridine (uracil) or an analogue of guanosine (guanine) or uridine (uracil), preferably guanosine (guanine) or an analogue thereof;
X is guanosine (guanine), uridine (uracil), adenosine (adenine), thymidine (thymine), cytidine (cytosine), or an analogue of these nucleotides (nucleosides), preferably uridine (uracil) or an analogue thereof;
N is a nucleic acid sequence having a length of 4 to 50, preferably of 4 to 40, more preferably of 4 to 30 or 4 to 20 nucleotides (nucleosides),
each N independently being selected from guanosine (guanine), uridine (uracil), adenosine (adenine), thymidine (thymine), cytidine (cytosine) or an analogue of these nucleotides (nucleosides);
a is an integer from 1 to 20, preferably from 1 to 15, most preferably from 1 to 10;
l is an integer from 1 to 40,
wherein when l = 1, G is guanosine (guanine) or an analogue thereof,
when l > 1, at least 50% of these nucleotides (nucleosides) are guanosine (guanine) or an analogue thereof;
m is an integer and is at least 3;
wherein when m = 3, X is uridine (uracil) or an analogue thereof, and
when m > 3, at least 3 successive uridines (uracils) or analogues of uridine (uracil) occur;
n is an integer from 1 to 40,
wherein when n = 1, G is guanosine (guanine) or an analogue thereof,
when n > 1, at least 50% of these nucleotides (nucleosides) are guanosine (guanine) or an analogue
thereof;
u,v may be independently from each other an integer from 0 to 50, preferably wherein when u = 0, v ≥ 1, or
when v = 0, u ≥ 1;
wherein the immunostimulatory nucleic acid sequence of formula (III) has a length of at least 50 nucleotides, preferably of at least 100 nucleotides, more preferably of at least 150 nucleotides, even more preferably of at least 200 nucleotides and most preferably of at least 250 nucleotides; or
(NᵤCₗXₘCₙNᵥ)ₐ (formula (IV))
wherein:
C is cytidine (cytosine), uridine (uracil) or an analogue of cytidine (cytosine) or uridine (uracil), preferably cytidine (cytosine) or an analogue thereof;
X is guanosine (guanine), uridine (uracil), adenosine (adenine), thymidine (thymine), cytidine (cytosine) or an analogue of the above-mentioned nucleotides (nucleosides), preferably uridine (uracil) or an analogue thereof;
N is each a nucleic acid sequence having independent from each other a length of 4 to 50, preferably of 4 to 40, more preferably of 4 to 30 or 4 to 20 nucleotides (nucleosides), each N independently being selected from guanosine (guanine), uridine (uracil), adenosine (adenine), thymidine (thymine), cytidine (cytosine) or an analogue of these nucleotides (nucleosides);
a is an integer from 1 to 20, preferably from 1 to 15, most preferably from 1 to 10;
l is an integer from 1 to 40,
wherein when l = 1, C is cytidine (cytosine) or an analogue thereof,
when l > 1, at least 50% of these nucleotides (nucleosides) are cytidine (cytosine) or an analogue thereof;
m is an integer and is at least 3;
wherein when m = 3, X is uridine (uracil) or an analogue thereof,
when m > 3, at least 3 successive uridines (uracils) or analogues of uridine (uracil) occur;
n is an integer from 1 to 40,
wherein when n = 1, C is cytidine (cytosine) or an analogue thereof,
when n > 1, at least 50% of these nucleotides (nucleosides) are cytidine (cytosine) or an analogue thereof.
u, v may be independently from each other an integer from 0 to 50, preferably wherein when u = 0, v ≥ 1, or
when v = 0, u ≥ 1;
wherein the immunostimulatory nucleic acid sequence of formula (IV) according to the invention has a length of at least 50 nucleotides, preferably of at least 100 nucleotides, more preferably of at least 150 nucleotides, even more preferably of at least 200 nucleotides and most preferably of at least 250 nucleotides.

5. Vaccine composition according to any of claims 1 to 4, wherein the cationic or polycationic protein or peptide as the complexing agent of the adjuvant component is disulfide-crosslinked.

6. Vaccine composition according to claim 5, wherein the complexing agent of the adjuvant component is selected from
an oligopeptide having following sum formula (V):
{(Arg)ₗ;(Lys)ₘ;(His)ₙ;(Orn)ₒ;(Xaa)ₓ}; (formula (V)
wherein l + m + n + o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide of formula (V); and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide of formula (V),
or selected
from a disulfide-crosslinked cationic compound comprising as a repeat unit an oligopeptide having following subformula (Va):
{(Arg)ₗ;(Lys)ₘ;(His)ₙ;(Orn)ₒ;(Xaa')ₓ(Cys)_{y}}; formula (Va)
wherein (Arg)ₗ;(Lys)ₘ;(His)ₙ;(Orn)ₒ; and x is preferably are as defined above for formula (V), Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide,
or selected
from a disulfide-crosslinked cationic compound comprising as a repeat unit an oligopeptide having following subformula (Vb):
Cys¹{(Arg)ₗ;(Lys)ₘ;(His)ₙ;(Orn)ₒ;(Xaa)ₓ}Cys²; (formula (Vb))
wherein component {(Arg)ₗ;(Lys)ₘ;(His)ₙ;(Orn)ₒ;(Xaa)ₓ} (formula (V)) within formula (Vb) is as defined herein and forms a core of subformula (Vb), and wherein Cys¹ and Cys² are Cysteines proximal to, or terminal to (Arg)ₗ;(Lys)ₘ;(His)ₙ;(Orn)ₒ;(Xaa)ₓ.

7. Vaccine composition according to any of claims 1 to 6, wherein the N/P ratio of the at least one immunostimulatory nucleic acid sequence to the complexing agent is 0.01-4.

8. Vaccine composition according to any of claims 1 to 7, wherein the antigen is selected from tumour antigens, pathogenic antigens, animal antigens, viral antigens, protozoan antigens, bacterial antigens, allergic antigens, autoimmune antigens, allergens or antibodies.

9. Vaccine composition according to claims 1 to 8, wherein the carrier molecule for combined packaging the adjuvant component and the RNA encoding the antigen is a polymeric carrier molecule according to generic formula the polymeric carrier molecule according to formula (VIa)
L-P¹-S-{[S-P²-S]ₐ[S-(AA)ₓ-S]_{b}}-S-P³-L,
wherein
S, L, P¹, P² and P³ are preferably as defined above for formula (VI),
a and b are integers, wherein a + b = n and n is an integer as defined above for formula (VI),
x is an integer selected from a range of 1 to 100, and
(AA) is selected from an aromatic, a hydrophilic, a lipophilic, or a weak basic amino acid or peptide, or is a signal peptide or signal sequence, a localization signal or sequence, a nuclear localization signal or sequence (NLS), an antibody, a cell penetrating peptide (CPP), or is selected from therapeutically active proteins or peptides, from antigens, tumour antigens, pathogenic antigens, animal antigens, viral antigens, protozoan antigens, bacterial antigens, allergic antigens, autoimmune antigens, from allergens, from antibodies, from immunostimulatory proteins or peptides, or from antigen-specific T-cell receptors.

10. Vaccine composition according to any of claims 1 to 9, wherein the vaccine composition further comprises cell penetrating peptides (CPPs) or cationic peptides for transportation, selected from protamine, nucleoline, spermine or spermidine, poly-L-lysine (PLL), basic polypeptides, poly-arginine, cell penetrating peptides (CPPs), chimeric CPPs, Transportan, or MPG peptides, HIV-binding peptides, Tat, HIV-1 Tat (HIV), Tat-derived peptides, oligoarginines, members of the penetratin family, Penetratin, Antennapedia-derived peptides, Antennapedia-derived peptides from *Drosophila antennapedia,* pAntp, plsl, antimicrobial-derived CPPs, Buforin-2, Bac715-24, SynB, SynB(1), pVEC, hCT-derived peptides, SAP, MAP, KALA, PpTG20, Proline-rich peptides, Loligomers, Arginine-rich peptides, Calcitonin-peptides, FGF, Lactoferrin, poly-L-Lysine, poly-Arginine, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, or Calcitonin peptide(s).

11. Vaccine composition according to any of claims 1 to 10, wherein the N/P ratio of all components of the vaccine composition is 0.01-2.

12. Vaccine composition according to any of claims 1 to 11 for use as a medicament.

13. Vaccine composition according to any of claims 1 to 11 for use in the treatment of cancer or tumour diseases or infectious diseases.

14. Kit, comprising a vaccine composition according to any of claims 1 to 11 in one or more parts of the kit, and optionally comprising instructions for use of the kit.

## Patentansprüche

1. Vakzinzusammensetzung, umfassend oder bestehend aus:
a) eine(r) Adjuvanskomponente, welche mindestens eine immunstimulatorische Nukleinsäuresequenz umfasst oder aus dieser besteht, die mit einem kationischen oder polykationischen Peptid oder Protein als Komplexierungsagens komplexiert ist;
b) eine(r) Nukleinsäuresequenz, welche ein Antigen kodiert, wobei die Nukleinsäure eine RNA ist; und
c) ein(em) polymeres/polymeren Trägermolekül zur gemeinsamen Verpackung der Adjuvanskomponente und der das Antigen kodierenden RNA,
wobei das Trägermolekül zur gemeinsamen Verpackung der Adjuvanskomponente und der das Antigen kodierenden RNA ein polymeres Trägermolekül gemäß der generischen Formel (VI) ist:
L-P¹-S-[S-P²-S]ₙ-S-P³-L
wobei
P¹ und P³ unterschiedlich oder miteinander identisch sind und eine lineare oder verzweigte hydrophile Polymerkette darstellen, wobei P¹ und P³ jeweils mindestens eine -SH-Gruppe aufweisen, die nach Kondensation mit Komponente P² eine Disulfidverknüpfung ausbilden kann, wobei die lineare oder verzweigte hydrophile Polymerkette aus Polyethylenglycol (PEG) ausgewählt ist, wobei die hydrophile Polymerkette ein Molekulargewicht von 1 kDa bis 100 kDa aufweist;
P² ein kationisches oder polykationisches Peptid oder Protein mit einer Länge von 3 bis 100 Aminosäuren ist oder ein kationisches oder polykationisches Polymer mit einem Molekulargewicht 0,5 kDa bis 30 kDa ist, wobei jede P² mindestens zwei -SH-Gruppen aufweist, die nach Kondensation mit weiteren Komponenten P² oder Komponente(n) P¹ und/oder P³ eine Disulfidverknüpfung ausbilden können;
-S-S- eine (reversible) Disulfidbindung ist;
L ein optionaler Ligand ist, der vorliegen kann oder nicht und unabhängig von den anderen aus RGD, Transferrin, Folat, einem Signalpeptid oder einer Signalsequenz, einem Lokalisationssignal oder einer Lokalisationssequenz, einem Kernlokalisationssignal oder einer Kernlokalisationssequenz (NLS), einem Antikörper, einem zellpenetrierenden Peptid (CPP), TAT, KALA, einem Liganden eines Rezeptors, Zytokinen, Hormonen, Wachstumsfaktoren, kleinen Molekülen, Kohlehydraten, Mannose, Galaktose, synthetischen Liganden, kleinmolekularen Agonisten, Inhibitoren oder Antagonisten von Rezeptoren oder RGD-peptidomimetischen Analoga ausgewählt sein kann; und
n eine ganze Zahl, ausgewählt aus einem Bereich von 1 bis 50, ist.

2. Vakzinzusammensetzung gemäß Anspruch 1, wobei n eine ganze Zahl aus einem Bereich von 1, 2, 3, 4, oder 5 bis 10, bevorzugt einem Bereich von 1, 2, 3, oder 4 bis 9, ist.

3. Vakzinzusammensetzung gemäß Anspruch 1 oder 2, wobei die immunstimulatorische Nukleinsäure der Adjuvanskomponente aus immunstimulatorischen CpG-Nukleinsäuresequenzen, immunstimulatorischer RNA (isRNA), ribosomaler RNA (rRNA), Transfer-RNA (tRNA), Messenger-RNA (mRNA), viraler RNA (vRNA) oder einer ein Antigen kodierenden RNA oder mRNA ausgewählt ist.

4. Vakzinzusammensetzung gemäß irgendeinem der Ansprüche 1 bis 3, wobei die immunstimulatorische RNA (isRNA) aus einer Nukleinsäuresequenz der Formel (I), (II), (III) oder (IV) ausgewählt ist:
GₗXₘGₙ (Formel (I))
wobei:
G Guanosin, Uridin oder ein Analogon von Guanosin oder Uridin ist;
X Guanosin, Uridin, Adenosin, Thymidin, Cytidin oder ein Analogon der oben genannten Nukleotide ist;
l eine ganze Zahl von 1 bis 40 ist,
wobei,
wenn I = 1, G Guanosin oder ein Analogon davon ist,
wenn I > 1, mindestens 50% der Nukleotide Guanosin oder ein Analogon davon sind;
m eine ganze Zahl ist und mindestens 3 ist;
wobei,
wenn m = 3, X Uridin oder ein Analogon davon ist,
wenn m > 3, mindestens 3 aufeinanderfolgende Uridine oder Analoga von Uridin auftreten;
n eine ganze Zahl von 1 bis 40 ist,
wobei,
wenn n = 1, G Guanosin oder ein Analogon davon ist,
wenn n > 1, mindestens 50% der Nukleotide Guanosin oder ein Analogon davon sind;
GₗXₘCₙ (Formel (II))
wobei:
C Cytidin, Uridin oder ein Analogon von Cytidin oder Uridin ist;
X Guanosin, Uridin, Adenosin, Thymidin, Cytidin oder ein Analogon der oben genannten Nukleotide ist;
l eine ganze Zahl von 1 bis 40 ist,
wobei,
wenn I = 1, C Cytidin oder ein Analogon davon ist,
wenn I > 1, mindestens 50% der Nukleotide Cytidin oder ein Analogon davon sind;
m eine ganze Zahl ist und mindestens 3 ist;
wobei,
wenn m = 3, X Uridin oder ein Analogon davon ist,
wenn m > 3, mindestens 3 aufeinanderfolgende Uridine oder Analoga von Uridin auftreten;
n eine ganze Zahl von 1 bis 40 ist,
wobei,
wenn n = 1, C Cytidin oder ein Analogon davon ist,
wenn n > 1, mindestens 50% der Nukleotide Cytidin oder ein Analogon davon sind;
(NᵤGₗXₘGₙNᵥ)ₐ, (Formel (III))
wobei:
G Guanosin (Guanin), Uridin (Uracil) oder ein Analogon von Guanosin (Guanin) oder Uridin (Uracil), vorzugsweise Guanosin (Guanin) oder ein Analogon davon, ist;
X Guanosin (Guanin), Uridin (Uracil), Adenosin (Adenin), Thymidin (Thymin), Cytidin (Cytosin) oder ein Analogon dieser Nukleotide (Nukleoside), vorzugsweise Uridin (Uracil) oder ein Analogon davon, ist;
N eine Nukleinsäuresequenz mit einer Länge von 4 bis 50, bevorzugt von 4 bis 40, bevorzugter von 4 bis 30 oder 4 bis 20 Nukleotiden (Nukleosiden), ist, wobei jedes N unabhängig aus Guanosin (Guanin), Uridin (Uracil), Adenosin (Adenin), Thymidin (Thymin), Cytidin (Cytosin) oder einem Analogon dieser Nukleotide (Nukleoside) ausgewählt ist;
a eine ganze Zahl von 1 bis 20, bevorzugt von 1 bis 15, am meisten bevorzugt von 1 bis 10, ist:
l eine ganze Zahl von 1 bis 40 ist,
wobei, wenn l = 1, G Guanosin (Guanin) oder ein Analogon davon ist, wenn l > 1, mindestens 50% dieser Nukleotide (Nukleoside) Guanosin (Guanin) oder ein Analogon davon sind;
m eine ganze Zahl ist und mindestens 3 ist;
wobei, wenn m = 3, X Uridin (Uracil) oder ein Analogon davon ist, und,
wenn m > 3, mindestens 3 aufeinanderfolgende Uridine (Uracile) oder Analoga von Uridin (Uracil) auftreten;
n eine ganze Zahl von 1 bis 40 ist,
wobei, wenn n = 1, G Guanosin (Guanin) oder ein Analogon davon ist,
wenn n > 1, mindestens 50% dieser Nukleotide (Nukleoside) Guanosin (Guanin) oder ein Analogon davon sind;
u,v unabhängig voneinander eine ganze Zahl von 0 bis 50 sein können,
wobei vorzugsweise, wenn u = 0, v ≥ 1, oder,
wenn v = 0, u ≥ 1;
wobei die immunstimulatorische Nukleinsäuresequenz von Formel (III) eine Länge von mindestens 50 Nukleotiden, bevorzugt von mindestens 100 Nukleotiden, bevorzugter von mindestens 150 Nukleotiden, noch bevorzugter von mindestens 200 Nukleotiden und am meisten bevorzugt von mindestens 250 Nukleotiden aufweist; oder
(NᵤGₗXₘCₙNᵥ)ₐ (Formel (IV))
wobei:
C Cytidin (Cytosin), Uridin (Uracil) oder ein Analogon von Cytidin (Cytosin) oder Uridin (Uracil), vorzugsweise Cytidin (Cytosin) oder ein Analogon davon, ist;
X Guanosin (Guanin), Uridin (Uracil), Adenosin (Adenin), Thymidin (Thymin), Cytidin (Cytosin) oder ein Analogon der oben genannten Nukleotide (Nukleoside), vorzugsweise Uridin (Uracil) oder ein Analogon davon, ist;
N jeweils eine Nukleinsäuresequenz ist, die unabhängig von den anderen eine Länge von 4 bis 50, bevorzugt von 4 bis 40, bevorzugter von 4 bis 30 oder 4 bis 20 Nukleotiden (Nukleosiden) aufweist, wobei jedes N unabhängig aus Guanosin (Guanin), Uridin (Uracil), Adenosin (Adenin), Thymidin (Thymin), Cytidin (Cytosin) oder einem Analogon dieser Nukleotide (Nukleoside) ausgewählt ist;
a eine ganze Zahl von 1 bis 20, bevorzugt von 1 bis 15, am meisten bevorzugt von 1 bis 10, ist;
l eine ganze Zahl von 1 bis 40 ist,
wobei, wenn l = 1, C Cytidin (Cytosin) oder ein Analogon davon ist,
wenn l > 1, mindestens 50% dieser Nukleotide (Nukleoside) Cytidin (Cytosin) oder ein Analogon davon sind;
m eine ganze Zahl ist und mindestens 3 ist;
wobei, wenn m = 3, X Uridin (Uracil) oder ein Analogon davon ist,
wenn m > 3, mindestens 3 aufeinanderfolgende Uridine (Uracile) oder Analoga von Uridin (Uracil) auftreten;
n eine ganze Zahl von 1 bis 40 ist,
wobei, wenn n = 1, C Cytidin (Cytosin) oder ein Analogon davon ist,
wenn n > 1, mindestens 50% dieser Nukleotide (Nukleoside) Cytidin (Cytosin) oder ein Analogon davon sind,
u, v unabhängig voneinander eine ganze Zahl von 0 bis 50 sein können,
wobei vorzugsweise, wenn u = 0, v ≥ 1, oder,
wenn v = 0, u ≥ 1;
wobei die immunstimulatorische Nukleinsäuresequenz der Formel (IV) gemäß der Erfindung eine Länge von mindestens 50 Nukleotiden, bevorzugt von mindestens 100 Nukleotiden, bevorzugter von mindestens 150 Nukleotiden, noch bevorzugter von mindestens 200 Nukleotiden und am meisten bevorzugt von mindestens 250 Nukleotiden aufweist.

5. Vakzinzusammensetzung gemäß irgendeinem der Ansprüche 1 bis 4, wobei das kationische oder polykationische Protein oder Peptid als Komplexierungsagens der Adjuvanskomponente Disulfid-quervernetzt ist.

6. Vakzinzusammensetzung gemäß Anspruch 5, wobei das Komplexierungsagens der Adjuvanskomponente ausgewählt ist aus
einem Oligopeptid mit der folgenden Summenformel (V):
{(Arg)ₗ;(Lys)ₘ;(His)ₙ;(Orn)ₒ;(Xaa)ₓ}; (Formel (V))
wobei l + m + n + o + x = 3-100, und l, m, n oder o unabhängig voneinander irgendeine Zahl, ausgewählt aus 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 und 91-100, sind, unter der Maßgabe, dass der Gesamtgehalt an Arg (Arginin), Lys (Lysin), His (Histidin) und Orn (Ornithin) mindestens 10% aller Aminosäuren des Oligopeptids der Formel (V) ausmacht; und Xaa irgendeine Aminosäure, ausgewählt aus nativen (= natürlich vorkommenden) oder nicht-nativen Aminosäuren, ausgenommen Arg, Lys, His oder Orn, ist; und x irgendeine Zahl, ausgewählt aus 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, ist, mit der Maßgabe, dass der Gesamtgehalt an Xaa 90% aller Aminosäuren des Oligopeptids der Formel (V) nicht übersteigt,
oder ausgewählt ist
aus einer Disulfid-quervernetzten kationischen Verbindung, welche als eine Wiederholungseinheit ein Oligopeptid mit der folgenden Unterformel (Va) umfasst:
{(Arg)ₗ;(Lys)ₘ;(His)ₙ;(Orn)ₒ;(Xaa')ₓ(Cys)_{y}}; (Formel (Va))
wobei (Arg)l;(Lys)m;(His)ₙ;(Orn)ₒ und x vorzugsweise wie oben für Formel (V) definiert sind, Xaa' irgendeine Aminosäure, ausgewählt aus nativen (= natürlich vorkommenden) oder nicht-nativen Aminosäuren, ausgenommen Arg, Lys, His, Orn oder Cys, ist, und y irgendeine Zahl, ausgewählt aus, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 und 81-90, ist, mit der Maßgabe, dass der Gesamtgehalt an Arg (Arginin), Lys (Lysin), His (Histidin) und Orn (Ornithin) mindestens 10% aller Aminosäuren des Oligopeptids ausmacht,
oder ausgewählt ist
aus einer Disulfid-quervernetzten kationischen Verbindung, welche als eine Wiederholungseinheit ein Oligopeptid mit der folgenden Unterformel (Vb) umfasst:
Cys¹{(Arg)ₗ;(Lys)ₘ;(His)ₙ;(Orn)ₒ;(Xaa)ₓ}Cys²; (Formel (Vb))
wobei die Komponente {(Arg)ₗ;(Lys)ₘ;(His)ₙ;(Orn)ₒ;(Xaa)ₓ} (Formel (V)) innerhalb der Formel (Vb) wie hierin definiert ist und einen Kern der Unterformel (Vb) bildet, und wobei Cys¹ und Cys² Cysteine proximal zu oder terminal zu (Arg)ₗ;(Lys)ₘ;(His)ₙ;(Orn)ₒ;(Xaa)ₓ sind.

7. Vakzinzusammensetzung gemäß irgendeinem der Ansprüche 1 bis 6, wobei das N/P-Verhältnis der mindestens einen immunstimulatorischen Nukleinsäuresequenz zu dem Komplexierungsagens 0,01-4 beträgt.

8. Vakzinzusammensetzung gemäß irgendeinem der Ansprüche 1 bis 7, wobei das Antigen aus Tumorantigenen, pathogenen Antigenen, tierischen Antigenen, viralen Antigenen, Protozoen-Antigenen, bakteriellen Antigenen, Allergieantigenen, Autoimmunantigenen, Allergenen oder Antikörpern ausgewählt ist.

9. Vakzinzusammensetzung gemäß irgendeinem der Ansprüche 1 bis 8, wobei das Trägermolekül zur gemeinsamen Verpackung der Adjuvanskomponente und der das Antigen kodierenden RNA ein polymeres Trägermolekül gemäß der generischen Formel (VIa) ist:
L-P¹⁻S-{[S-P²-S]ₐ[S-(AA)ₓ-S]_{b}}-S-P³-L,
wobei
S, L, P¹, P² und P³ vorzugsweise wie oben für Formel (VI) definiert sind,
a und b ganze Zahlen sind, wobei a + b = n, und n eine ganze Zahl, wie oben für Formel (VI) definiert, ist,
x eine ganze Zahl, ausgewählt aus einem Bereich von 1 bis 100, ist, und
(AA) aus einer aromatischen, einer hydrophilen, einer lipophilen oder einer schwach basischen Aminosäure oder einem Peptid ausgewählt ist, oder ein Signalpeptid oder eine Signalsequenz, ein Lokalisationssignal oder eine Lokalisationssequenz, ein Kernlokalisationssignal oder eine Kernlokalisationssequenz (NLS), ein Antikörper, ein zellpenetrierendes Peptid (CPP) ist, oder aus therapeutisch wirksamen Proteinen oder Peptiden, aus Antigenen, Tumorantigenen, pathogenen Antigenen, tierischen Antigenen, viralen Antigenen, Protozoen-Antigenen, bakteriellen Antigenen, Allergieantigenen, Autoimmunantigenen, aus Allergenen, aus Antikörpern, aus immunstimulatorischen Proteinen oder Peptiden oder aus Antigen-spezifischen T-Zell-Rezeptoren ausgewählt ist.

10. Vakzinzusammensetzung gemäß irgendeinem der Ansprüche 1 bis 9, wobei die Vakzinzusammensetzung außerdem zellpenetrierende Peptide (CPPs) oder kationische Peptide für den Transport, ausgewählt aus Protamin, Nucleolin, Spermin oder Spermidin,
Poly-L-Lysin (PLL), basischen Polypeptiden, Polyarginin, zellpenetrierenden Peptiden (CPPs), chimären CPPs, Transportan oder MPG-Peptiden, HIV-bindenden Peptiden, Tat, HIV-1 Tat (HIV), von Tat abgeleiteten Peptiden, Oligoargininen, Mitgliedern der Penetratin-Familie, Penetratin, von Antennapedia abgeleiteten Peptiden, von *Drosophila antennapedia* abgeleiteten Peptiden, pAntp, plsl, antimikrobiell-abgeleiteten CPPs, Buforin-2,
Bac715-24, SynB, SynB(1), pVEC, hCT-abgeleiteten Peptiden, SAP, MAP, KALA, PpTG20, Prolin-reichen Peptiden, Loligomeren, Arginin-reichen Peptiden, Calcitonin-Peptiden, FGF, Lactoferrin, poly-L-Lysin, poly-Arginin, Histonen, VP22-abgeleiteten oder analogen Peptiden, HSV, VP22 (Herpes simplex), MAP, KALA oder Proteintransduktionsdomänen (PTDs, PpT620), Prolin-reichen Peptiden, Arginin-reichen Peptiden, Lysin-reichen Peptiden, Pep-1, L-Oligomeren oder Calcitonin-Peptid(en), umfasst.

11. Vakzinzusammensetzung gemäß irgendeinem der Ansprüche 1 bis 10, wobei das N/P-Verhältnis aller Komponenten der Vakzinzusammensetzung 0,01-2 beträgt.

12. Vakzinzusammensetzung gemäß irgendeinem der Ansprüche 1 bis 11 zur Verwendung als Medikament.

13. Vakzinzusammensetzung gemäß irgendeinem der Ansprüche 1 bis 11 zur Verwendung bei der Behandlung von Krebs- oder Tumorerkrankungen oder Infektionskrankheiten.

14. Kit, umfassend eine Vakzinzusammensetzung gemäß irgendeinem der Ansprüche 1 bis 11 in einem mehreren Teilen des Kits und optional umfassend Anweisungen zum Gebrauch des Kits.

## Revendications

1. Composition de vaccin, comprenant ou constituée par :
a) un composant faisant office d'adjuvant comprenant ou constitué par au moins une séquence immunostimulante d'acides nucléiques, complexée avec un peptide cationique ou polycationique ou protéine à titre d'agent de complexation ;
b) une séquence d'acides nucléiques encodant un antigène ; dans laquelle l'acide nucléique est un ARN ; et
c) une molécule de support polymère pour le conditionnement combiné du composant faisant office d'adjuvant et de l'ARN encodant l'antigène ; dans laquelle la molécule de support pour le conditionnement combiné du composant faisant office d'adjuvant et de l'ARN encodant l'antigène représente une molécule de support polymère répondant à la formule générique (VI) :
L-P¹-S-[S-P²-S]ₙ-S-P³-L
dans laquelle
P¹ et P³ sont identiques ou différents l'un de l'autre et représentent une chaîne polymère hydrophile linéaire ou ramifiée, chaque P¹ et P³ présentant au moins une fraction -SH- capable de former une liaison disulfure après condensation avec le composant P², la chaîne polymère hydrophile linéaire ou ramifiée étant choisie parmi le polyéthylène glycol (PEG) ; dans laquelle la chaîne polymère hydrophile manifeste un poids moléculaire de 1 kDa à 100 kDa ;
P² représente un peptide ou une protéine cationique ou polycationique, dont la longueur s'élève de 3 à 100 acides aminés ; ou
représente un polymère cationique ou polycationique, dont le poids moléculaire s'élève de 0,5 kDa à 30 kDa ; chaque P² présentant au moins deux fractions -SH- capables de former une liaison disulfure après condensation avec d'autres composants P² ou bien un ou des composants P¹ et/ou P³ ;
-S-S- représente une liaison disulfure (réversible) ;
L représente un ligand facultatif, qui peut être présent ou non, et qui peut être choisi de manière respectivement indépendante parmi le motif RGD, la transferrine, un folate, un peptide signal ou une séquence signal, un signal ou une séquence de localisation, un signal ou une séquence de localisation nucléaire (NLS), un anticorps, un peptide du type à pénétration cellulaire (CPP), le gène TAT, le peptide KALA, un ligand d'un récepteur, des cytokines, des hormones, des facteurs de croissance, des petites molécules, des hydrates de carbone, le mannose, le galactose, des ligands synthétiques, des agonistes à petites cellules, des inhibiteurs ou des antagonistes de récepteurs, ou des analogues peptidomimétiques de RGD ; et
n représente un entier choisi parmi une plage de 1 à 50.

2. Composition de vaccin selon la revendication 1, dans laquelle n est un entier compris dans l'intervalle de 1, 2, 3, 4 ou 5 à 10, de préférence dans l'intervalle de 1, 2, 3 ou 4 à 9.

3. Composition de vaccin selon la revendication 1 ou 2, dans laquelle l'acide nucléique immunostimulant du composant faisant office d'adjuvant est choisi parmi des séquences immunostimulantes d'acides nucléiques CpG, de l'ARN immunostimulant (ARNis), de l'ARN ribosomique (ARNr), de l'ARN de transfert (ARNt), de l'ARN messager (ARNm), de l'ARN viral (ARNv) ou bien un ARN ou un ARNm encodant un antigène.

4. Composition de vaccin selon l'une quelconque des revendications 1 à 3, dans laquelle l'ARN immunostimulant (ARNis) est choisi parmi une séquence d'acides nucléiques répondant à la formule (I), (II), (III) ou (IV)
GₗXₘGₙ (formule (I))
dans laquelle :
G représente la guanosine, l'uridine ou un analogue de la guanosine ou de l'uridine ;
X représente la guanosine, l'uridine, l'adénosine, la thymidine, la cytidine ou un analogue des nucléotides mentionnés ci-dessus ;
l représente un entier de 1 à 40 ;
dans lequel :
lorsque l = 1, G représente la guanosine ou un de ses analogues ;
lorsque l > 1, au moins 50 % des nucléotides représentent la guanosine ou un de ses analogues ;
m représente un entier et est égal à au moins 3 ;
dans lequel :
lorsque m = 3, X représente l'uridine ou un de ses analogues ;
lorsque m > 3, on obtient au moins 3 uridine successifs ou trois analogues successifs de l'uridine ;
n représente un entier de 1 à 40 ;
dans lequel :
lorsque n = 1, G représente la guanosine ou un de ses analogues ;
lorsque n > 1, au moins 50 % des nucléotides représentent la guanosine ou un de ses analogues ;
CₗXₘCₙ (formule (II))
dans laquelle :
C représente la cytidine, l'uridine ou un analogue de la cytidine ou de l'uridine ;
X représente la guanosine, l'uridine, l'adénosine, la thymidine, la cytidine ou un analogue des nucléotides mentionnés ci-dessus ;
l représente un entier de 1 à 40 ;
dans lequel :
lorsque l = 1, C représente la cytidine ou un de ses analogues ;
lorsque l > 1, au moins 50 % des nucléotides représentent la cytidine ou un de ses analogues ;
m représente un entier et est égal à au moins 3 ;
dans lequel :
lorsque m = 3, X représente l'uridine ou un de ses analogues ;
lorsque m > 3, on obtient au moins l'uridine successifs ou trois analogues successifs de l'uridine ;
n représente un entier de 1 à 40 ;
dans lequel :
lorsque n = 1, C représente la cytidine ou un de ses analogues ;
lorsque n > 1, au moins 50 % des nucléotides représentent la cytidine ou un de ses analogues ;
(NᵤGₗXₘGₙNᵥ)ₐ, (formule (III))
dans laquelle :
G représente la guanosine (la guanine), l'uridine (l'uracile) ou un analogue de la guanosine (de la guanine) ou de l'uridine (de l'uracile), de préférence la guanosine (la guanine) ou un de ses analogues ;
X représente la guanosine (la guanine), l'uridine (l'uracile), l'adénosine (l'adénine), la thymidine (la thymine), la cytidine (la cytosine) ou un analogue de ces nucléotides (de ces nucléosides), de préférence l'uridine (l'uracile) ou un de ces analogues;
N représente une séquence d'acides nucléiques possédant une longueur de 4 à 50, de préférence 4 à 40, de manière plus préférée de 4 à 30 ou de 4 à 20 nucléotides (nucléosides), chaque N étant choisi de manière indépendante parmi la guanosine (la guanine), l'uridine (l'uracile), l'adénosine (l'adénine), la thymidine (la thymine), la cytidine (la cytosine) ou un analogue de ces nucléotides (de ces nucléosides) ;
a représente un entier de 1 à 20, de préférence de 1 à 15, de manière de loin préférée de 1 à 10 ;
l représente un entier de 1 à 40 ;
dans lequel :
lorsque l = 1, G représente la guanosine (la guanine) ou un de ses analogues ;
lorsque l > 1, au moins 50 % de ces nucléotides (nucléosides) représentent la guanosine (la guanine) ou un de ses analogues ;
m représente un entier et est égal à au moins 3 ;
dans lequel :
lorsque m = 3, X représente l'uridine (l'uracile) ou un de ses analogues ; et
lorsque m > 3, on obtient au moins 3 uridine (uracile) successifs ou trois analogues successifs de l'uridine (l'uracile) ;
n représente un entier de 1 à 40 ;
dans lequel :
lorsque n = 1, G représente la guanosine (la guanine) ou un de ses analogues ;
lorsque n > 1, au moins 50 % de ces nucléotides (nucléosides) représentent la guanosine (la guanine) ou un de ses analogues ;
u, v peuvent représenter, indépendamment l'un de l'autre, un entier de 0 à 50 ;
de préférence, dans lequel, lorsque u = 0, v ≥ 1 ; ou lorsque v = 0, u ≥ 1 ;
dans laquelle la séquence immunostimulante d'acides nucléiques répondant à la formule (III) possède une longueur d'au moins 50 nucléotides, de préférence d'au moins 100 nucléotides, de manière plus préférée d'au moins 150 nucléotides, de manière encore plus préférée d'au moins 200 nucléotides et de manière de loin préférée d'au moins 250 nucléotides ; ou
(NᵤCₗXₘCₙNᵥ)ₐ, (formule (IV))
dans laquelle
C représente la cytidine (la cytosine), l'uridine (l'uracile) ou un analogue de la cytidine (de la cytosine) ou de l'uridine (de l'uracile), de préférence la cytidine (la cytosine) ou un de ses analogues ;
X représente la guanosine (la guanine), l'uridine (l'uracile), l'adénosine (l'adénine), la thymidine (la thymine), la cytidine (la cytosine) ou un analogue des nucléotides (des nucléosides) mentionnés ci-dessus, de préférence l'uridine (l'uracile) ou un de ses analogues ;
N représente à chaque fois une séquence d'acides nucléiques possédant, de manière respectivement indépendante, une longueur de 4 à 50, de préférence 4 à 40, de manière plus préférée de 4 à 30 ou de 4 à 20 nucléotides (nucléosides), N étant choisi à chaque fois parmi la guanosine (la guanine), l'uridine (l'uracile), l'adénosine (l'adénine), la thymidine (la thymine), la cytidine (la cytosine) ou un analogue de ces nucléotides (de ces nucléosides) ;
a représente un entier de 1 à 20, de préférence de 1 à 15, de manière de loin préférée de 1 à 10 ;
l représente un entier de 1 à 40 ;
dans lequel :
lorsque l = 1, C représente la cytidine (la cytosine) ou un de ses analogues ;
lorsque l > 1, au moins 50 % de ces nucléotides (nucléosides) représentent la cytidine (la cytosine) ou un de ses analogues ;
m représente un entier et est égal à au moins 3 ;
dans lequel :
lorsque m = 3, X représente l'uridine (l'uracile) ou un de ses analogues ;
lorsque m > 3, on obtient au moins 3 uridine (uracile) successifs ou trois analogues successifs de l'uridine (l'uracile) ;
n représente un entier de 1 à 40 ;
dans lequel :
lorsque n = 1, C représente la cytidine (la cytosine) ou un de ses analogues ;
lorsque n > 1, au moins 50 % de ces nucléotides (nucléosides) représentent la cytidine (la cytosine) ou un de ses analogues ;
u, v peuvent représenter, indépendamment l'un de l'autre, un entier de 0 à 50 ;
de préférence, dans lequel, lorsque u = 0, v ≥ 1 ; ou lorsque v = 0, u ≥ 1 ;
dans laquelle la séquence immunostimulante d'acides nucléiques répondant à la formule (IV) selon l'invention possède une longueur d'au moins 50 nucléotides, de préférence d'au moins 100 nucléotides, de manière plus préférée d'au moins 150 nucléotides, de manière encore plus préférée d'au moins 200 nucléotides et de manière de loin préférée d'au moins 250 nucléotides.

5. Composition de vaccin selon l'une quelconque des revendications 1 à 4, dans laquelle la protéine ou le peptide cationique ou polycationique à titre d'agent de complexation du composant faisant office d'adjuvant est réticulé à l'aide d'un disulfure.

6. Composition de vaccin selon la revendication 5, dans laquelle l'agent de complexation du composant faisant office d'adjuvant est choisi parmi :
un oligopeptide répondant à la formule développée suivante (V) :
{(Arg}ₗ;{Lys)ₘ;(His)ₙ;(Orn)ₒ;{Xaa)ₓ}; (formule (V))
dans laquelle l + m + n + o = 3 - 100, et l, m, n ou o représente, indépendamment l'un de l'autre, n'importe quel nombre choisi parmi 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 et 91-100, avec cette réserve que la teneur globale en Arg (arginine), Lys (lysine), His (histidine) et Orn (ornithine) représente au moins 10 % de la totalité des acides aminés de l'oligopeptide répondant à la formule (V) ; et Xaa représente n'importe quel acide aminé choisi parmi des acides aminés natifs (= d'origine naturelle) ou non natifs, à l'exception de Arg, Lys, His ou Orn ; et x représente n'importe quel nombre choisi parmi 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, avec cette réserve que la teneur globale en Xaa n'excède pas 90 % de la totalité des acides aminés de l'oligopeptide répondant à la formule (V) ;
ou choisi parmi un composé cationique réticulé avec un disulfure comprenant, à titre de motif récurrent, un oligopeptide répondant à la sous-formule suivante (Va) :
{(Arg)ₗ;(Lys)ₘ;(His)ₙ;(Orn)ₒ;(Xaa')ₓ(Cys)_{y}}; (formule (Va))
dans laquelle (Arg)ₗ ; (Lys)ₘ ; (His)ₙ ; (Orn)ₒ ; et x sont de préférence tels que définis ci-dessus pour la formule (V) ; Xaa' représente n'importe quel acide aminé choisi parmi des acides aminés natifs (= d'origine naturelle) ou non natifs, à l'exception de Arg, Lys, His ou Orn ; et y représente n'importe quel nombre choisi parmi 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 et 81-90, avec cette réserve que la teneur globale en Arg (arginine), Lys (lysine), His (histidine) et Orn (ornithine) représente au moins 10 % de la totalité des acides aminés de l'oligopeptide ;
ou choisi parmi un composé cationique réticulé avec un disulfure comprenant, à titre de motif récurrent, un oligopeptide répondant à la sous-formule suivante (Vb) :
Cys¹{(Arg)ₗ;((Lys)ₘ;(His)ₙ(Orn)ₒ;(Xaa)ₓ}Cys²; (formule (Vb))
dans laquelle le composant {(Arg)ₗ ; (Lys)ₘ ; (His)ₙ ; (Orn)ₒ ; (Xaa)ₓ} (formule (V)) dans la formule (Vb) est tel que défini en l'occurrence et forme un noyau de la sous-formule (Vb), et dans laquelle Cys¹ et Cys² représentent des cystéines en position proximale ou en position terminale par rapport à (Arg)ₗ ; (Lys)ₘ ; (His)ₙ ; (Orn)ₒ ; (Xaa)ₓ.

7. Composition de vaccin selon l'une quelconque des revendications 1 à 6, dans laquelle le rapport N/P de ladite au moins une séquence immunostimulante d'acides nucléiques à l'agent de complexation s'élève de 0,01 à 4.

8. Composition de vaccin selon l'une quelconque des revendications 1 à 7, dans laquelle l'antigène est choisi parmi des antigènes tumoraux, des antigènes pathogènes, des antigènes d'origine animale, des antigènes viraux, des antigènes protozoaires, des antigènes bactériens, des antigènes allergiques, des antigènes auto-immuns, des allergènes ou des anticorps.

9. Composition de vaccin selon l'une quelconque des revendications 1 à 8, dans laquelle la molécule de support pour le conditionnement combiné du composant faisant office d'adjuvant et de l'ARN encodant l'antigène est une molécule de support polymère répondant à la formule générique (VIa) :
L-P¹-S-{[S-P²-S]ₐ[S-(AA)ₓ-S]_{b}}-S-P³-L,
dans laquelle
S, L, P¹, P² et P³ sont de préférence tels que définis ci-dessus pour la formule (VI) ;
a et b représentent des entiers dans lesquels a + b = n et n représente un entier tel que défini ci-dessus pour la formule (VI) ;
x représente un entier choisi parmi une plage de 1 à 100 ; et
(AA) est choisi parmi un peptide ou un acide aminé aromatique, hydrophile, lipophile ou faiblement basique, ou bien un peptide signal ou une séquence signal, une séquence ou un signal de localisation, une séquence ou un signal de localisation nucléaire (NLS), un anticorps, un peptide de pénétration cellulaire (CPP), ou bien est choisi parmi des peptides ou des protéines jouissant d'une activité thérapeutique, ou est choisi parmi des antigènes, des antigènes tumoraux, des antigènes pathogènes, des antigènes d'origine animale, des antigènes viraux, des antigènes protozoaires, des antigènes bactériens, des antigènes allergiques, des antigènes auto-immuns, parmi des allergènes, parmi des anticorps, parmi des peptides ou des protéines immunostimulants, ou parmi des récepteurs des cellules T du type à spécificité antigénique.

10. Composition de vaccin selon l'une quelconque des revendications 1 à 9, dans laquelle la composition de vaccin comprend en outre des peptides du type à pénétration cellulaire (CPP) ou des peptides cationiques à des fins de transport, choisis parmi la protamine, la nucléoline, la spermine ou la spermidine, la poly-L-lysine (PLL), des polypeptides basiques, la polyarginine, des peptides du type à pénétration cellulaire (CPP), des CPP chimères, le Transportan, ou des peptides MPG, des peptides se liant au VIH, le gène Tat, la protéine HIV-1 Tat (HIV), des peptides dérivés du gène Tat, des oligoarginines, des membres de la famille des Pénétratines, la Pénétratine, des peptides dérivés du gène Antennapedia, des peptides dérivés du gène Antennapedia issu de *Drosophila antennapedia,* le peptide pAntp, le peptide plsl, des CPP à dérivation antimicrobienne, la Buforine-2, le peptide Bac715-24, le gène SynB, le gène SynB(1), le peptide pVEC, des peptides dérivés de l'hormone hCT, le gène SAP, le gène MAP, le peptide KALA, le peptide PpTG20, des peptides riches en proline, des L-oligomères, des peptides riches en arginine, des peptides à base de calcitonine, le facteur de croissance FGF, la lactoferrine, la poly-L-lysine, la polyarginine, des histones, des peptides analogues au virus VP22 ou dérivés de celui-ci, le virus HSV, le virus VP22 (herpes simplex), le gène MAP, le peptide KALA ou des domaines de transduction de protéines (PTD), le peptide PpT620, des peptides riches en proline, des peptides riches en arginine, des peptides riches en lysine, le peptide Pep-1, des L-oligomères ou un ou plusieurs peptides à base de calcitonine.

11. Composition de vaccin selon l'une quelconque des revendications 1 à 10, dans laquelle le rapport N/P de la totalité des composants de la composition de vaccin s'élève de 0,01 à 2.

12. Composition de vaccin selon l'une quelconque des revendications 1 à 11, pour son utilisation comme médicament.

13. Composition de vaccin selon l'une quelconque des revendications 1 à 11, pour son utilisation dans le traitement du cancer ou de maladies tumorales ou de maladies infectieuses.

14. Kit comprenant une composition de vaccin selon l'une quelconque des revendications 1 à 11 dans une ou plusieurs parties du kit, et comprenant de manière facultative un mode d'emploi pour le kit.
